(19) Europäisches Patentamt
European Patent Office
Office européen des brevets

(11) Veröffentlichungsnummer : **0 072 352**
**B1**

(12) # EUROPÄISCHE PATENTSCHRIFT

(45) Veröffentlichungstag der Patentschrift : **05.03.86**

(51) Int. Cl.[4] : **C 07 D223/16**, A 61 K 31/55

(21) Anmeldenummer : **82810329.1**

(22) Anmeldetag : **05.08.82**

(54) **Benzazepin-2-one, Verfahren zu ihrer Herstellung, pharmazeutische Präparate die diese Verbindungen enthalten, sowie die Verbindungen zur therapeutischen Verwendung.**

(30) Priorität : **11.08.81 US 291907**
**09.11.81 US 319863**

(43) Veröffentlichungstag der Anmeldung : **16.02.83 Patentblatt 83/07**

(45) Bekanntmachung des Hinweises auf die Patenterteilung : **05.03.86 Patentblatt 86/10**

(84) Benannte Vertragsstaaten :
**AT BE CH DE FR IT LI LU NL SE**

(56) Entgegenhaltungen :
**GB-A- 1 305 278**
**US-A- 3 748 321**
**Chemical Abstracts Band 82, Nr. 21, 26. Mai 1975, Columbus, Ohio, USA H. UNO et al. " Dihydrobenzazepine derivatives", Seite 614, linke Spalte, Abstract Nr. 139981g**

(73) Patentinhaber : **CIBA-GEIGY AG**
**Klybeckstrasse 141**
**CH-4002 Basel (CH)**

(72) Erfinder : **Watthey, Jeffrey W.H., Dr.**
**61 Deepwood Drive**
**Chappaqua New York 10514 (US)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 072 352 B1**

Jouve, 18, rue St-Denis, 75001 Paris, France

**Beschreibung**

In der britischen Patentschrift 1,305,278 werden Chinolin-, Benzazepin- und Benzazocinverbindungen offenbart, die entweder in 3- oder in 4-Stellung des heterocyclischen Ringes obligatorisch sowohl einen gegebenenfalls substituierten Phenylsubstituenten als auch eine Aminoalkylengruppe aufweisen. Sie sind geeignet als Stimulantien des zentralen Nervensystems und können zur Behandlung von Depression verwendet werden. Im US-Patent 3,748,321 sind N-(Diniederalkylaminoniederalkyl)-N-niederalkyl-1-benzalkylenimin-1-carboxamide beschrieben, die als Tranquillizer Verwendung finden können. In der japanischen Auslegeschrift 74 28,754 sind Benzazepin-2,5-dione offenbart, die in 3,4-Position eine Doppelbindung aufweisen. Zur Verwendung dieser Verbindungen wird ausschliesslich mitgeteilt, sie seien nützlich als Zwischenprodukte für die Synthese vom Pharmazeutika.

Die vorliegende Erfindung beruht auf dem Auffinden des Sachverhalts, dass bestimmte substituierte 3-Amino-[1]-benzazepin-2-on-1-alkansäuren und deren Derivate eine neue Klasse stark wirksamer Inhibitoren im Hinblick auf das Angiotensin-umwandelnde Enzym (ACE) darstellen.

Die vorstehenden Eigenschaften machen die erfindungsgemässen 3-Amino-[1] benzazepin-2-one besonders wertvoll bei alleiniger oder kombinierter Verabreichung an Säuger, beispielsweise zur Behandlung von oder Vorbeugung vor Erkrankungen, die auf die Hemmung des Angiotensin-umwandelnden Enzyms ansprechen, z. B. kardiovaskuläre Störungen, wie Bluthochdruck, und kardialen Zuständen, wie kongestive Herzinsuffizienz.

Die Erfindung betrifft neue 3-Amino-[1] benzazepin-2-on-1-alkansäuren und deren Derivate, die wertvoll sind als Inhibitoren für das Angiotensin-umwandelnde Enzym, Verfahren zu deren Herstellung, diese Verbindungen enthaltende pharmazeutische Zusammensetzungen und diese Verbindungen zur Verwendung bei der Behandlung von Erkrankungen, die auf die Inhibierung des Angiotensin-umwandelnden Enzyms ansprechen.

Die erfindungsgemässen Verbindungen sind durch die allgemeine Formel I gekennzeichnet,

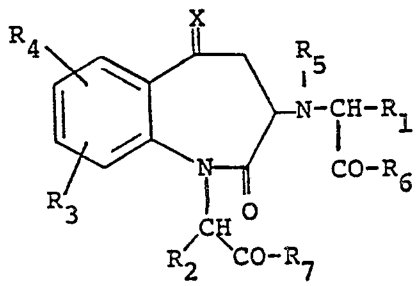

(I)

worin

$R_1$ Wasserstoff, $C_{1-7}$-Alkyl, Amino-$C_{1-7}$-alkyl, Aryl, durch $C_{1-7}$-Alkyl, $C_{1-7}$-Alkoxy, $C_{1-7}$-Alkylendioxy, $C_{1-7}$-Alkanoyloxy, Hydroxy, Halogen oder Trifluormethyl mono- oder disubstituiertes Phenyl, Aryl-$C_{1-7}$-alkyl, durch $C_{1-7}$-Alkyl, $C_{1-7}$-Alkoxy, $C_{1-7}$-Alkylendioxy, $C_{1-7}$-Alkanoyloxy, Hydroxy, Halogen oder Trifluormethyl mono- oder disubstituiertes Phenyl-$C_{1-7}$-alkyl, Cycloalkyl oder Cycloalkyl-$C_{1-7}$-alkyl bedeutet ;

$R_2$ Wasserstoff oder $C_{1-7}$-Alkyl ist ;

$R_3$ und $R_4$ unabhängig Wasserstoff, $C_{1-7}$-Alkyl, $C_{1-7}$-Alkoxy, $C_{1-7}$-Alkanoyloxy, Hydroxy, Halogen oder Trifluormethyl darstellen oder $R_3$ und $R_4$ gemeinsam $C_{1-7}$-Alkylendioxy bedeuten ;

$R_5$ Wasserstoff oder $C_{1-7}$-Alkyl ist,

$R_6$ und $R_7$ unabhängig voneinander Hydroxy, $C_{1-7}$-Alkoxy ; ω-(Amino, Mono- oder Di-$C_{1-7}$-alkylamino)-substituiertes $C_{2-4}$-Alkoxy ; Carboxy-substituiertes $C_{1-7}$-Alkoxy ; $C_{1-7}$-Alkoxycarbonyl-substituiertes $C_{1-7}$-Alkoxy ; Aryl-substituiertes $C_{1-7}$-Alkoxy ; durch $C_{1-7}$-Alkyl, $C_{1-7}$-Alkoxy, $C_{1-7}$-Alkylendioxy, $C_{1-7}$-Alkanoyloxy, Hydroxy, Halogen oder Trifluormethyl mono- oder disubstituiertes Phenyl-$C_{1-7}$-alkoxy ; (Hydroxy, $C_{1-7}$-Alkanoyloxy oder $C_{1-7}$-Alkoxy)-substituiertes $C_{1-7}$-Alkoxy, (Hydroxy, $C_{1-7}$-Alkanoyloxy oder $C_{1-7}$-Alkoxy)-substituiertes $C_{1-7}$-Alkoxymethoxy ; Bicycloalkoxycarbonyl-substituiertes $C_{1-7}$-Alkoxy ; 3-Phthalidoxy ; ($C_{1-7}$-Alkyl, $C_{1-7}$-Alkoxy, Halo)-substituiertes 3-Phthalidoxy ; Amino, $C_{1-7}$-Alkylamino ; Di-$C_{1-7}$-alkylamino ; di-$C_{1-7}$-Alkylamino, worin beide Alkylgruppen durch eine Kohlenstoff-Kohlenstoff-Bindung verbunden sind und gemeinsam mit dem Aminostickstoff einen 5-, 6- oder 7-gliedrigen heterocyclischen Ring bilden ; α-(Carboxy oder $C_{1-7}$-Alkoxycarbonyl)-substituiertes $C_{1-7}$-Alkylamino ; Aryl-substituiertes $C_{1-7}$-Alkylamino oder durch $C_{1-7}$-Alkyl, $C_{1-7}$-Alkoxy, $C_{1-7}$-Alkylendioxy, $C_{1-7}$-Alkanoyloxy-Hydroxy, Halogen oder Trifluormethyl mono- oder disubstituiertes Phenyl-$C_{1-7}$-alkylamino welches jeweils an dem α-Kohlenstoffatom durch Carboxy oder $C_{1-7}$-Alkoxycarbonyl substituiert sein kann, bedeuten ;

und X Oxo, zwei Wasserstoffatome oder ein Wasserstoffatom und eine Hydroxygruppe bedeutet ; und worin der carbocyclische Ring auch Hexahydro oder 6,7,8,9-Tetrahydro sein kann ; und deren Stereoisomere und Salze.

Die Salze der Verbindungen der Formel I leiten sich von solchen Verbindungen ab, die salzbildende Eigenschaften besitzen, und sind vorzugsweise pharmazeutisch verträgliche Salze.

$COR_6$, worin $R_6$ Hydroxy ist, oder $COR_7$, worin $R_7$ Hydroxy ist, stellen eine Carboxylgruppe dar.

$COR_6$ oder $COR_7$, worin einer der Reste $R_6$ und $R_7$ oder beide Reste $C_{1-7}$-Alkoxy ; ω-(Amino, Mono- oder Di-$C_{1-7}$-alkylamino)-substituiertes $C_{2-4}$-Alkoxy ; Carboxy-substituiertes $C_{1-7}$-Alkoxy, z. B. α-Carboxy-substituiertes $C_{1-7}$-Alkoxy ; $C_{1-7}$-Alkoxycarbonyl-substituiertes $C_{1-7}$-Alkoxy, z. B. α-$C_{1-7}$-Alkoxycarbonyl-substituiertes $C_{1-7}$-Alkoxy ; Aryl-substituiertes $C_{1-7}$-Alkoxy, z. B. Benzyloxy ; entsprechend substituiertes Phenyl-$C_{1-7}$ Alkoxy z. B. substituiertes Benzyloxy ; (Hydroxy, $C_{1-7}$-Alkanoyloxy oder $C_{1-7}$-Alkoxy)-substituiertes $C_{1-7}$-Alkoxy, z. B. Pivaloyloxymethoxy ; (Hydroxy, $C_{1-7}$-Alkanoyloxy oder $C_{1-7}$-Alkoxy)-substituiertes $C_{1-7}$-Alkoxymethoxy ; Bicycloalkoxycarbonyl-substituiertes $C_{1-7}$-Alkoxy, z. B. Bicyclo-[2.2.1] heptyloxycarbonyl-substituiertes $C_{1-7}$-Alkoxy, insbesondere Bicyclo [2.2.1] heptyloxycarbonyl-substituiertes Methoxy ; 3-Phthalidoxy ; ($C_{1-7}$-Alkyl, $C_{1-7}$-Alkoxy, Halo)-substituiertes 3-Phthalidoxy bedeuten, stellen eine veresterte Carboxylgruppe dar.

$COR_6$ oder $COR_7$, worin einer der Reste $R_6$ und $R_7$ oder beide Amino ; $C_{1-7}$-Alkylamino ; Di-$C_{1-7}$-alkylamino ; Di-$C_{1-7}$-Alkylamino, worin beide Alkylgruppen durch eine Kohlenstoff-Kohlenstoff-Bindung verbunden sind und gemeinsam mit dem Aminostickstoff einen 5-, 6- oder 7-gliedrigen heterocyclischen Ring, z. B. Pyrrolidino, Piperidino oder Perhydroazepino, bilden ; α-(Carboxy oder $C_{1-7}$-Alkoxycarbonyl)-substituiertes $C_{1-7}$-Alkylamino ; Aryl-substituiertes $C_{1-7}$-Alkylamino, worin Aryl vorzugsweise Phenyl bedeutet und das jeweils am α-Kohlenstoffatom durch Carboxy oder $C_{1-7}$-Alkoxycarbonyl substituiert sein kann, bedeuten, stellen eine gegebenenfalls substituierte Carbamoylgruppe dar.

Die vorstehend genannten Ester und Amide der erfindungsgemässen Mono- oder Di-carbonsäuren stellen Wirkstoff-Vorstufen dar, die durch Solvolyse oder unter physiologischen Bedingungen in die vorliegenden Carbonsäuren übergeführt werden können. Diese Derivate stellen einen speziellen Gegenstand der Erfindung dar.

Diese Ester sind vorzugsweise z. B. geradkettige oder verzweigte unsubstituierte oder in geeigneter Weise substituierte $C_{1-7}$-Alkylester, wie die Pivaloyloxymethyl-, Bornyloxycarbonylmethyl-, Benzyl-, α-Carboxyäthyl- oder in geeigneter Weise veresterte α-Carboxyäthylester.

Diese Amide sind vorzugsweise z. B. einfache primäre und sekundäre Amide und Amide, abgeleitet von den Aminosäuren oder deren Derivaten, wie die von Alanin, Phenylalanin abgeleiteten Amide.

Insbesondere betrifft die Erfindung die Verbindungen der Formel I, worin

$R_2$, $R_3$, $R_4$, $R_5$ und X die vorstehend angegebenen Bedeutungen haben,

$R_1$ Wasserstoff, $C_{1-7}$-Alkyl, Amino-$C_{1-7}$-alkyl, Aryl, Aryl-$C_{1-7}$-alkyl, Cycloalkyl-$C_{1-7}$-alkyl oder durch $C_{1-7}$-Alkyl, $C_{1-7}$-Alkoxy, $C_{1-7}$-Alkylendioxy, $C_{1-7}$-Alkanoyloxy, Hydroxy, Halogen oder Trifluormethyl mono- oder disubstituiertes Phenyl oder Phenyl-$C_{1-7}$-alkyl bedeutet,

und $R_6$ und $R_7$ unabhängig voneinander Hydroxy, Amino, Mono- oder Di-$C_{1-7}$-alkylamino, $C_{1-7}$-Alkoxy, Aryl-$C_{1-7}$-alkoxy durch $C_{1-7}$-Alkyl, $C_{1-7}$-Alkoxy, $C_{1-7}$-Alkylendioxy, $C_{1-7}$-Alkanoyloxy, Hydroxy, Halogen oder Trifluormethyl mono- oder disubstituiertes Phenyl-$C_{1-7}$-alkoxy, $C_{1-7}$-Alkanoyloxymethoxy, ω-(Amino, Mono- oder Di-$C_{1-7}$-alkylamino)-$C_{2-4}$-alkoxy, (Carboxy oder $C_{1-7}$-Alkoxycarbonyl)-$C_{1-7}$-alkoxy bedeuten, oder deren Salze.

Bevorzugte Ausführungsformen der Erfindung betreffen Verbindungen der Formel I, worin $R_2$, $R_5$ und X die vorstehend angegebenen Bedeutungen haben, $R_1$ Wasserstoff, $C_{1-7}$-Alkyl, Amino-$C_{1-7}$-alkyl, Phenyl-$C_{1-7}$-alkyl, oder im Phenylteil durch $C_{1-7}$-Alkyl, Hydroxy, $C_{1-7}$-Alkoxy, $C_{1-7}$-Alkylendioxy, $C_{1-7}$-Alkanoyloxy, Halogen oder Trifluormethyl mono- oder disubstituiertes Phenyl $C_{1-7}$-Alkyl bedeutet, ist, $R_3$ und $R_4$ Wasserstoff, $C_{1-7}$-Alkoxy, $C_{1-7}$-Alkyl, Halogen oder Trifluormethyl bedeuten, oder $R_3$ und $R_4$ gemeinsam $C_1$-$C_7$-Alkylendioxy bedeuten, $R_6$ und $R_7$ unabhängig voneinander Hydroxy, Amino, $C_{1-7}$-Alkoxy, Phenyl-$C_{1-7}$-alkoxy, $C_{1-7}$-Alkoxycarbonyl-$C_{1-7}$-alkoxy bedeuten, oder deren Salze.

Sehr wertvoll sind Verbindungen der Formel I, worin $R_1$ die Bedeutung hat von Wasserstoff, $C_{1-7}$-Alkyl, ω-Amino-$C_{1-7}$-alkyl, Phenyl-$C_{1-7}$-alkyl, oder im Phenylteil durch $C_{1-7}$-Alkyl, Hydroxy, $C_{1-7}$-Alkoxy, $C_{1-7}$-Alkanoyloxy, Halogen oder Trifluormethyl monosubstituiertes Phenyl-$C_{1-7}$-alkyl bedeutet, $R_2$ und $R_5$ Wasserstoff oder $C_{1-7}$-Alkyl bedeuten, $R_3$ und $R_4$ Wasserstoff, $C_{1-7}$-Alkoxy, $C_{1-7}$-Alkyl, Halogen oder Trifluormethyl bedeuten oder $R_3$ und $R_4$ gemeinsam $C_{1-7}$-Alkylendioxy bedeuten, X Oxo, eine Hydroxygruppe und ein Wasserstoffatom, oder zwei Wasserstoffatome bedeutet, $R_6$ und $R_7$ unabhängig voneinander Hydroxy, Amino, $C_{1-7}$-Alkoxy, Phenyl-$C_{1-7}$-alkoxy, $C_{1-7}$-Alkoxycarbonyl-$C_{1-7}$-alkoxy bedeuten, oder deren Salze.

Besonders wertvoll sind Verbindungen der Formel I, worin $R_1$ Wasserstoff, $C_{1-7}$-Alkyl, ω-Amino-$C_{1-7}$-alkyl, Aryl-$C_{1-7}$-alkyl oder durch $C_{1-7}$-Alkyl, $C_{1-7}$-Alkoxy, $C_{1-7}$-Alkylendioxy, $C_{1-7}$-Alkanoyloxy, Hydroxy, Halogen oder Trifluormethyl mono- oder disubstituiertes Phenyl-$C_{1-7}$-alkyl bedeutet, $R_2$ und $R_5$ Wasserstoff oder $C_{1-7}$-Alkyl bedeuten, $R_3$ Wasserstoff bedeutet, $R_4$ Wasserstoff, $C_{1-7}$-Alkoxy, $C_{1-7}$-Alkyl, Halogen oder Trifluormethyl bedeutet, X Oxo, eine Hydroxygruppe und ein Wasserstoffatom, oder zwei Wasserstoffatome bedeutet, $R_6$ und $R_7$ unabhängig voneinander Hydroxy, Amino, $C_{1-7}$-Alkoxy, Phenyl-$C_{1-7}$-alkoxy, $C_{1-7}$-Alkoxycarbonyl-$C_{1-7}$-alkoxy bedeuten, oder deren Salze.

Vor allem wertvoll sind Verbindungen der Formel I, worin $R_1$ die Bedeutung hat von Wasserstoff, Methyl, Aethyl, Isopropyl, ω-Aminopropyl, ω-Aminobutyl, Aryl-(methyl, äthyl, propyl), worin Aryl unsubstituiertes Phenyl oder ein im Phenylteil substituiertes Phenyl-(methyl, äthyl, propyl) darstellt wobei als Substituenten eine Methyl-, Hydroxy-, Methoxy-, Methylendioxy-, Acetoxygruppe, ein Chloratom oder eine Trifluormethylgruppe in Frage kommen, $R_2$ und $R_5$ Wasserstoff oder Methyl sind, $R_3$ und $R_4$ Wasserstoff, Methoxy, Methyl, Chlor oder Trifluormethyl sind, X Oxo, eine Hydroxygruppe und ein

Wasserstoffatom, oder zwei Wasserstoffatome darstellt, $R_6$ und $R_7$ unabhängig voneinander Hydroxy, Amino, Aethoxy, Methoxy, Benzyloxy, Aethoxycarbonylmethoxy oder Pivaloyloxymethoxy bedeuten, oder deren Salze.

Ueberragend wertvoll sind Verbindungen der Formel IA

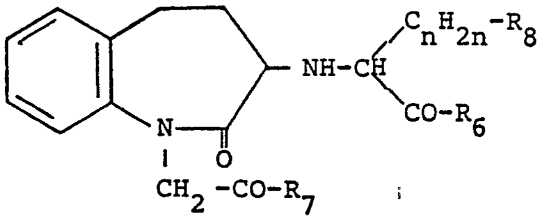

(IA)

;

worin n eine ganze Zahl von 1 bis 4 bedeutet, $R_8$ Wasserstoff, unsubstituiertes Phenyl oder durch $C_{1-7}$-Alkyl, $C_{1-7}$-Alkoxy, $C_{1-7}$-Alkanoyloxy, Halogen, Hydroxy oder Trifluormethyl monosubstituiertes Phenyl, bedeutet, $R_6$ und $R_7$ unabhängig voneinander Hydroxy, $C_{1-4}$-Alkoxy, Benzyloxy oder Amino bedeuten, oder deren pharmazeutisch verträgliche Salze.

Besonders wertvoll sind Verbindungen der Formel IA, worin $C_nH_{2n}$ Aethylen darstellt, $R_8$ Phenyl oder, durch $C_{1-4}$-Alkoxy, $C_{1-4}$-Alkyl, Halogen oder Trifluormethyl monosubstituiertes Phenyl, darstellt, $R_6$ und $R_7$ unabhängig voneinander Hydroxy oder $C_{1-4}$-Alkoxy bedeuten, oder deren pharmazeutisch verträgliche Salze.

Die vorliegende Erfindung betrifft auch die Stereoisomere der Verbindungen der Formel I. Racemate sind erhältlich, wenn z. B. in der Formel I zumindest einer der Reste $R_1$ und $R_2$ nicht Wasserstoff und/oder X H(OH) bedeutet.

Die individuellen Enantiomere dieser Racemate können ihrerseits erhalten werden. bestimmte spezifische Vertreter dieser Isomere sind als Inhibitoren für das Angiotensin-umwandelnde Enzym bevorzugt.

Herausragend sind Verbindungen der Formel IB

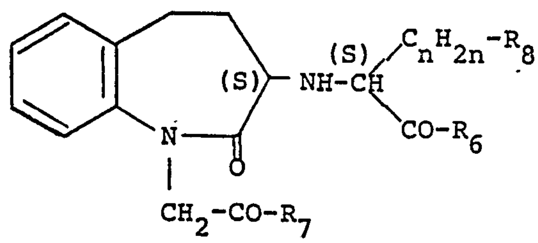

(IB)

worin S die Chiralität bedeutet, n eine ganze Zahl von 1 bis 4 bedeutet, $R_8$ Wasserstoff, unsubstituiertes Phenyl oder durch $C_{1-7}$-Alkyl, $C_{1-7}$-Alkoxy, $C_{1-7}$-Alkanoyloxy, Halogen, Hydroxy oder Trifluormethyl monosubstituiertes Phenyl bedeutet, $R_6$ und $R_7$ unabhängig voneinander Hydroxy, $C_{1-4}$-Alkoxy, Benzyloxy oder Amino bedeuten, oder deren pharmazeutisch verträgliche Salze.

Die vorliegend verwendeten allgemeinen Definitionen besitzen im Bereich der Erfindung die folgenden Bedeutungen :

Aryl bedeutet einen carbocyclischen aromatischen Rest, vorzugsweise unsubstituiertes Phenyl.

Die Bezeichnung Cycloalkyl bedeutet einen cyclischen Kohlenwasserstoffrest, der vorzugsweise 3 bis 8 Kohlenstoffatome enthält und beispielsweise Cyclopentyl oder Cyclohexyl ist.

Die Bezeichnung Aryl-$C_{1-7}$-alkyl bedeutet vorzugsweise Benzyl, 1- oder 2-Phenäthyl, 1-, 2- oder 3-Phenylpropyl, 1-, 2-, 3- oder 4-Phenylbutyl.

Die Bezeichnung Cycloalkyl-$C_{1-7}$-alkyl bedeutet vorzugsweise 1- oder 2-(Cyclopentyl oder Cyclohexyl)äthyl, 1-, 2- oder 3-(Cyclopentyl oder Cyclohexyl)propyl oder 1-, 2-, 3- oder 4-(Cyclopentyl oder Cyclohexyl)butyl.

Die Bezeichnung « $C_{1-7}$ », die vorstehend und nachfolgend in Verbindung mit organischen Resten bzw. Verbindungen angewandt wird, definiert Reste mit bis zu und einschliesslich 7 Kohlenstoffatomen. Solche Reste haben vorzugsweise bis zu und einschliesslich 4 und vorteilhafterweise 1 oder 2 Kohlenstoffatome.

Eine $C_{1-7}$-Alkylgruppe enthält vorzugsweise 1 bis 4 Kohlenstoffatome und bedeutet z. B. Aethyl, Propyl, Butyl oder vorteilhafterweise Methyl.

Eine $C_{1-7}$-Alkoxygruppe enthält vorzugsweise 1 bis 4 Kohlenstoffatome und ist beispielsweise Methoxy, Propoxy, Isopropoxy oder vorteilhafterweise Aethoxy. Eine Mono-$C_{1-7}$-alkylaminogruppe

enthält vorzugsweise 1 bis 4 Kohlenstoffatome in dem Alkylteil und ist beispielsweise N-Methylamino, N-Propylamino oder vorteilhafterweise N-Aethylamino. Eine Di-$C_{1-7}$-alkylaminogruppe enthält vorzugsweise 1 bis 4 Kohlenstoffatome in jedem $C_{1-7}$-Alkyl-Teil und bedeutet z. B. N,N-Dimethylamino, N-Methyl-N-äthylamino und vorteilhafterweise, N,N-Diäthylamino.

$C_{1-7}$-Alkanoyloxy bedeutet vorzugsweise Acetoxy, Propionoxy oder Pivaloyloxy.

$C_{1-7}$-Alkylendioxy bedeutet vorzugsweise Aethylendioxy und vorteilhafterweise Methylendioxy.

Aryl-$C_{1-7}$-alkoxy bedeutet vorteilhafterweise z. B. Benzyloxy. Von den substituierten Phenyl-$C_{1-7}$-alkoxyresten ist im Phenylteil durch Methyl, Methoxy oder Chlor substituiertes Benzyloxy bevorzugt.

Carboxy-$C_{1-7}$-alkoxy bedeutet vorteilhafterweise z. B. 1-Carboxyäthoxy.

$C_{1-7}$-Alkoxycarbonyl-$C_{1-7}$-alkoxy bedeutet vorteilhafterweise z. B. 1-(Aethoxycarbonyl)-äthoxy.

Amino-$C_{1-7}$-alkoxy, Mono-$C_{1-7}$-alkylamino-$C_{1-7}$-alkoxy, Di-$C_{1-7}$-alkylamino-$C_{1-7}$-alkoxy bedeuten vorteilhafterweise z. B. Aminoäthoxy, Aethylaminoäthoxy bzw. Diäthylaminoäthoxy. $C_{1-7}$-Alkanoyloxymethoxy bedeutet vorteilhafterweise z. B. Pivaloyloxymethoxy.

Bicycloalkyloxycarbonyl-$C_{1-7}$-alkoxy bedeutet vorzugsweise Bicyclo [2.2.1] heptyloxycarbonyl-$C_{1-7}$-alkoxy, unsubstituiert oder substituiert durch $C_{1-7}$-Alkyl, vorteilhafterweise Bornyloxycarbonylmethoxy.

Amino-$C_{1-7}$-alkyl und ω-Amino-$C_{1-7}$-alkyl bedeuten vorzugsweise Amino (äthyl, propyl oder butyl) bzw. ω-Amino (äthyl, propyl oder butyl).

Halogen bedeutet vorzugsweise Chlor, kann jedoch auch Brom, Fluor oder Jod sein.

Erfindungsgemäss können eine oder beide der Carboxylgruppen der Dicarbonsäuren, d. h. der Verbindungen der Formel I, worin $R_6$ und $R_7$ Hydroxy sind, in Form der funktionellen Derivate als Ester oder Amide vorliegen. Diese funktionellen Derivate sind vorzugsweise die Mono- oder Bis-$C_{1-7}$-alkylester, z. B. Methyl-, Aethyl-, n- oder i-Propyl-, Butyl- oder Benzylester ; die Mono- oder Bis-amide, die mono- oder di-N-alkylierten Amide, z. B. Mono- oder Diäthylamide ; die mono- oder di-substituierten $C_{1-7}$-Alkylester, z. B. die ω-(Amino, Mono, oder Dimethylamino, Carboxy oder Carbäthoxy)-(äthyl, propyl oder butyl)-ester. Stark bevorzugte funktionelle Derivate sind die Monoester der Formel I, z. B. solche, worin einer der Reste $R_6$ und $R_7$ Hydroxy und der andere $C_{1-7}$-Alkoxy bedeutet.

Pharmazeutisch verträgliche Salze sind vorzugsweise Metall- oder Ammoniumsalze der Verbindungen der Formel I, worin $COR_6$ und/oder $COR_7$ Carboxy bedeuten, insbesondere Alkali- oder Erdalkalimetallsalze, z. B. das Natrium-, Kalium- Magnesium- oder Calciumsalz ; oder vorteilhafterweise leicht kristallisierende Ammoniumsalze, abgeleitet von Ammoniak oder organischen Aminen, wie Mono-, Di- oder Tri-$C_{1-7}$ (alkyl, cycloalkyl oder hydroxyalkyl)aminen, $C_{1-7}$-Alkylendiaminen oder $C_{1-7}$-(Hydroxyalkyl oder Aralkyl) alkylammonium-Basen, z. B. Methylamin, Diäthylamin, Triäthylamin, Dicyclohexylamin, Triäthanolamin, Aethylendiamin, Tris-(hydroxymethyl) aminomethan oder Benzyltrimethylammoniumhydroxid. Diese Verbindungen der Formel I bilden Säureadditionsalze, bei denen es sich vorzugsweise um solche von therapeutisch verträglichen anorganischen oder organischen Säuren handelt, wie starken Mineralsäuren, z. B. Halogenwasserstoffsäuren, z. B. Chlorwasserstoff- oder Bromwasserstoffsäure ; Schwefelsäure, Phosphorsäure, Salpetersäure oder Perchlorsäure ; aliphatischen oder aromatischen Carbon- oder Sulfonsäuren, z. B. Ameisensäure, Essigsäure, Propionsäure, Bernsteinsäure, Glykolsäure, Milchsäure, Apfelsäure, Weinsäure, Gluconsäure, Citronensäure, Ascorbinsäure, Maleinsäure, Fumarsäure, Hydroxymaleinsäure, Brenztraubensäure, Phenylessigsäure, Benzoesäure, 4-Aminobenzoesäure, Anthranilsäure, 4-Hydroxybenzoesäure, Salicylsäure, 4-Aminosalicylsäure, Pamoasäure, Nicotinsäure ; Methansulfonsäure, Aethansulfonsäure, Hydroxyäthansulfonsäure, Benzolsulfonsäure, p-Toluolsulfonsäure, Naphthalinsulfonsäure, Sulfanilsäure oder Cyclohexylsulfaminsäure.

Die Verbindungen der Formel I zeigen wertvolle pharmakologische Eigenschaften, welche sie unter anderem aufgrund ihrer Hemmwirkung auf die Absonderung von Angiotensin II hervorrufen. Diese Wirkung entsteht durch selektive Hemmung des Enzyms, welches das Angiotensin in Säugern umwandelt. Die Verbindungen sind daher nützlich für die Behandlung von Krankheiten, welche auf die Hemmung des Enzyms, welches das Angiotensin in Säugern, einschließlich Menschen, umwandelt, ansprechen.

Die Verbindungen der Erfindung zeigen in erster Linie hypotensive/antihypertensive Wirkungen und beeinflussen die Herztätigkeit. Diese Eigenschaften können durch in-vivo- oder in-vitro-Tierversuche, vorzugsweise an Säugetieren, z. B. Ratten, Katzen, Hunden oder ihren isolierten Organen, als Testobjekte nachgewiesen werden. Die Tiere können entweder normotensiv oder hypertensiv, z. B. genetisch spontan hypertensive Ratten, oder renal hypertensive Ratten oder Hunde, und Hunde, denen Natrium entzogen ist, sein. Die Verbindungen können den Versuchstieren enteral oder parenteral, vorzugsweise oral oder intravenös, z. B. durch Gelatine-Kapseln oder in Form von Stärke enthaltenden Suspensionen bzw. wäßrigen Lösungen, verabreicht werden. Die verwendete Dosis kann in einem Bereich von zwischen 0,01 und 100 mg/kg/Tag, vorzugsweise 0,05 und 50 mg/kg/Tag, insbesondere 0,1 und 25 mg/kg/Tag liegen.

Die in vivo blutdrucksenkende Wirkung wird entweder direkt mit einem Katheter, der in die femorale Arterie des Versuchstieres eingeführt ist, oder indirekt durch Sphygmomanometrie am Rattenschwanz, und einem Übertragungsinstrument registriert. Der Blutdruck wird vor und nach der Verabreichung des Wirkstoffes in mm Hg bestimmt.

So sind die antihypertensiven Wirkungen in spontan hypertensiven Ratten durch indirekte Messung des systolischen Blutdrucks nachweisbar. Nicht narkotisierte Ratten werden einzeln in Käfigen, welche ihre Bewegung einschränken, in eine leicht geheizte Kammer gebracht. Ein Puls-Sensor wird innerhalb

einer aufblasbaren Manschette am Schwanz jeder Ratte angebracht. Die Manschette wird aufgeblasen, um die Schwanz-Arterie zu schließen. Der Druck in der Manschette wird kontinuierlich reduziert, und der systolische Druck entspricht demjenigen Druck in der Manschette, bei welchem die Pulswellen wieder auftreten. Nach der Registrierung von Kontrollwerten des Blutdrucks und der Herzfrequenz werden die Testverbindungen peroral einmal täglich an vier aufeinanderfolgenden Tagen verabreicht. Zusätzliche Blutdruckmessungen werden üblicherweise nach 2,0, 4,0 und 23,5 Stunden nach jeder täglichen Dosis vorgenommen. Die Werte werden mit denjenigen von Ratten, welche lediglich mit der Trägersubstanz behandelt worden sind, verglichen.

Als Illustration der Erfindung sei der antihypertensive Effekt des « höher schmelzenden » 1-Carboxymethyl-3-(1-äthoxycarbonyl-3-phenylpropylamino)-2,3,4,5-tetrahydro-1H-[1]-benzazepin-2-ons des Beispiels 1 angeführt : bei einer Dosis von 3 mg/kg p. o. wird der Blutdruck durchschnittlich um 40 mm Hg gesenkt. Die Messungen sind nach 2 und 4 Stunden nach der Verabreichung an den letzten zwei Tagen durchgeführt worden. Die entsprechende S,S-enantiomere Verbindung des Beispiels 12 senkt den Blutdruck bei einer peroralen Dosis von 1 mg/kg um 30 mm Hg.

Die Verbindungen der Erfindung zeigen nach intravenöser oder oraler Verabreichung eine hemmende Wirkung gegen die durch Angiotensin I hervorgerufene Blutdrucksteigerung auch an normotensiven Ratten. Das Angiotensin I wird durch das genannte Umwandlungsenzym zu der stark blutdrucksteigernden Substanz Angiotensin II hydrolysiert. Die Hemmung des genannten Enzyms blockiert die Bildung von Angiotensin II aus Angiotensin I. In dieser Weise wird die durch Angiotensin I provozierte Blutdrucksteigerung abgeschwächt.

Der entsprechende in-vivo-Test mit den intravenös verabreichten Verbindungen wird mit männlichen normotensiven Ratten, welche mit dem Natriumsalz der 5-Äthyl-5-(1-methylpropyl)-2-thiobarbitursäure narkotisiert sind, durchgeführt. Eine femorale Arterie und eine Wadenvene werden mit je einer Hohlnadel für die direkte Messung des Blutdrucks und für die intravenöse Verabreichung des Angiotensins I und einer erfindungsgemäßen Verbindung versehen. Nach der Stabilisierung des basalen Blutdrucks wird die Druckänderung nach drei, in 5-minütigen Intervallen, i. v. durchgeführten Reizungen mit 333 ng/kg Angiotensin I bestimmt. Solche Druckänderungen werden wieder nach 5, 10, 15, 30 und 60 Minuten nach intravenöser Verabreichung der zu prüfenden Verbindung bestimmt. Die letztgenannten Druckänderungen werden mit den Anfangswerten verglichen. Jede festgestellte Abnahme vom Ansprechen des Blutdrucks ist ein Hinweis auf die Hemmung des Enzyms, welches das Angiotensin I umwandelt. Als Illustration der Erfindung sei auf das « höher schmelzende » 1-Carboxymethyl-3-(1-äthoxycarbonyl-3-phenylpropylamino)-2,3,4,5-tetrahydro-1H-[1] benzazepin-2-on des Beispiels 1 und auf das entsprechende S,S-Enantiomere des Beispiels 12 hingewiesen. Diese hemmen vollständig die Blutdrucksteigerung nach Angiotensin I-Reizung während 30 Minuten nach intravenöser Verabreichung einer Dosis von 1 mg/kg pro Substanz.

Die in-vitro-Hemmung des Enzyms, welches das Angiotensin umwandelt, kann bei den Verbindungen dieser Erfindung analog zu Biochim. Biophys. Acta *293,* 451 (1973) nachgewiesen werden. Gemäß dieser Methode werden die genannten Verbindungen in ungefähr 1 mMol-Konzentrationen in Phosphatpuffer aufgelöst. Zu 100 Mikroliter von Lösungen der Testverbindung in Phosphatpuffer, welche man auf die gewünschte Konzentration verdünnt, gibt man zuerst 100 Mikroliter von 5 millimolarem Hippuryl-histidylleucin in Phosphatpuffer und dann 50 μl des Angiotensin-umwandelnden Enzyms. Dieses Enzym wird aus Lungen von erwachsenen männlichen Kaninchen in Tris-Puffer, der Kalium- und Magnesiumchlorid und auch Rohrzucker enthält, hergestellt. Die genannten Lösungen werden 30 Minuten bei 37 °C inkubiert, und die weitere Reaktion wird durch Hinzufügen von 0,75 ml einer 0,6-normalen wäßrigen Natriumhydroxidlösung gestoppt. Dann gibt man bei Zimmertemperatur 100 Mikroliter einer 0,2 %-igen Lösung von o-Phthalaldehyd in Methanol und nach 10 Minuten 100 Mikroliter 6n-Chlorwasserstoffsäure dazu. Diese Proben werden in einem Spektrophotometer bei 360 nm mit Wasser verglichen, und ihre optische Dichte wird bestimmt. Diese Werte werden durch einen Konversionsfaktor einer Standard-Kurve angepaßt, wobei man die während der genannten Inkubation von 30 Minuten gebildete Histidyl-leucin-Menge in Nanomolen erhält. Die Ergebnisse werden, um den $IC_{50}$-Wert zu bestimmen, gegenüber den Wirkstoff-Konzentrationen graphisch als Kurve dargestellt. Der $IC_{50}$-Wert bedeutet diejenige Wirkstoff-Konzentration, welche die Hälfte der Aktivität einer Kontrollprobe, die keinen Wirkstoff enthält, ergibt. Als Illustration der Erfindung sei auf das « höher schmelzende » 1-Carboxymethyl-3-(1-carboxy-3-phenylpropylamino)-2,3,4,5-tetrahydro-1H-[1]-benzazepin-2-on des Beispiels 9 und auf das entsprechende S,S-Enantiomere des Beispiels 19 hingewiesen, welche einen $IC_{50}$-Wert von $5{,}2 \times 10^{-9}$ M bzw. $1{,}7 \times 10^{-9}$ M aufweisen. Das entsprechende « niedriger schmelzende » 1-Carboxymethyl-3-(1-carboxy-3-phenylpropylamino)-2,3,4,5-tetrahydro-1H-[1] benzazepin-2-on des Beispiels 8 zeigt einen $IC_{50}$-Wert von $5{,}8 \times 10^{-8}$ M.

Das Enzym, welches das Angiotensin umwandelt, beteiligt sich nicht nur an der Umwandlung von Angiotensin I in Angiotensin II, sondern es spielt auch bei der Kontrolle von Bradykinin- und Aldosteron-Spiegeln eine Rolle. Die Wirkung von Verbindungen der Erfindung auf diese Faktoren kann auch zu ihren antihypertensiven und herzwirksamen Eigenschaften beitragen.

Wegen der vorher genannten vorteilhaften Eigenschaften sind die Verbindungen der Erfindung sehr wertvoll als spezifische therapeutische Mittel für Säuger inklusive Menschen.

Dementsprechend sind die Verbindungen der Erfindung wertwolle antihypertensive Mittel, insbesondere für die Herabsetzung des hohen Blutdrucks (ohne Rücksicht auf die Ätiologie) und/oder bei

Herzerkrankungen, wie Herzinsuffizienz, und/oder anderen Zuständen, die mit Ödemen oder Ascites einhergehen. Sie können auch als Zwischenprodukte zur Herstellung von anderen wertvollen Produkten, insbesondere von pharmakologisch wirksamen Präparaten eingesetzt werden.

Die Verbindungen der Formel I gemäß der Erfindung können in an sich bekannter Weise hergestellt werden, indem man z. B.

a) in eine Verbindung der Formel II

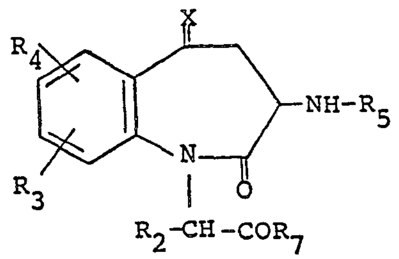

(II)

worin der carbocyclische Ring auch Hexahydro oder 6,7,8,9-Tetrahydro bedeuten kann und worin X, $R_2$, $R_3$, $R_4$, $R_5$ und $R_7$ die vorstehend angegebenen Bedeutungen besitzen, den Rest $—CH(R_1)COR_6$ durch Alkylierung mit einer Verbindung der Formel $R_1—CH(Z)—COR_6$ (IIIA), worin Z eine reaktive veresterte Hydroxygruppe bedeutet und $R_1$ und $R_6$ die vorstehend angegebenen Bedeutungen besitzen, oder mit einer Verbindung der Formel $R_1—CO—COR_6$ (IV), worin $R_1$ und $R_6$ die oben angegebenen Bedeutungen haben, in Gegenwart eines Reduktionsmittels, unter vorübergehendem Schutz etwaiger primärer und sekundärer Aminogruppen und/oder gegebenenfalls Hydroxy- und/oder Oxogruppen, die in irgendeinem der Reste X, $R_1$, $R_3$, $R_4$, $R_6$ und $R_7$ anwesend sein können, einführt oder

b) eine Verbindung der Formel V

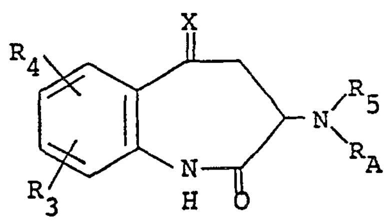

(V)

worin der carbocyclische Ring auch Hexahydro oder 6,7,8,9-Tetrahydro sein kann und worin X, $R_3$, $R_4$ und $R_5$ die vorstehend angegebenen Bedeutungen besitzen und $R_A$ Wasserstoff oder $—CH(R_1)COR_6$ wie vorstehend definiert ist, mit einer Verbindung der Formel $R_2—CH(Z)—COR_7$ (IIIB), worin Z eine reaktive veresterte Hydroxygruppe bedeutet und $R_2$ und $R_7$ die vorstehend angegebenen Bedeutungen besitzen, alkyliert, wobei man vorübergehend etwaige primäre und sekundäre Aminogruppen und/oder gegebenenfalls Hydroxy- und/oder Oxogruppen, die in irgendeinem der Reste X, $R_1$, $R_3$, $R_4$, $R_6$ und $R_7$ anwesend sein können, schützt oder

c) eine Verbindung der Formel VI

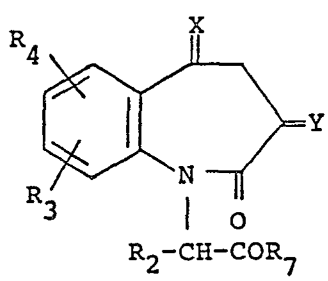

(VI)

worin der carbocyclische Ring auch Hexahydro oder 6,7,8,9-Tetrahydro sein kann und worin Y Oxo oder eine reaktive veresterte Hydroxygruppe Z gemeinsam mit Wasserstoff bedeutet und X, $R_2$, $R_3$, $R_4$ und $R_7$ die vorstehend angegebenen Bedeutungen besitzen, mit einem Amin der Formel VII

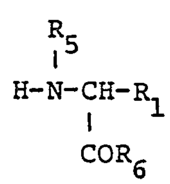

(VII)

worin $R_1$, $R_5$ und $R_6$ die vorstehend angegebenen Bedeutungen besitzen, kondensiert, wobei, wenn Y Oxo bedeutet, die Kondensation in Gegenwart eines Reduktionsmittels und unter vorübergehendem Schutz der Oxogruppe, die als Substituent X anwesend sein kann, durchgeführt wird, oder

d) in einer Verbindung der Formel VIII

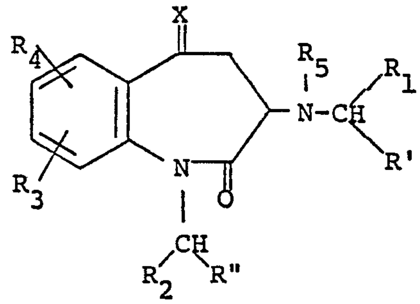

(VIII)

worin der carbocyclische Ring auch Hexahydro oder 6,7,8,9-Tetrahydro sein kann und worin X und $R_1$ bis $R_5$ die vorstehend angegebenen Bedeutungen besitzen, eines der Symbole R′ und R″ Cyan ist und das andere ebenfalls Cyan oder $COR_6$ bzw. $COR_7$, wie vorstehend definiert, ist, die Cyangruppe(n) einer Solvolyse unterzieht oder

e) eine Verbindung der Formel IX

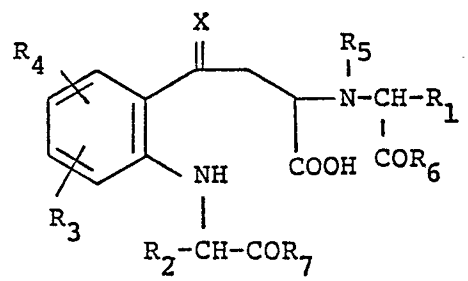

(IX)

worin der carbocyclische Ring auch Hexahydro oder 3,4,5,6-Tetrahydro sein kann und worin X und $R_1$ bis $R_7$ die vorstehend angegebenen Bedeutungen besitzen, oder einen Ester hiervon cyclisiert oder

f) eine Verbindung, die strukturell mit einer Verbindung der vorstehend angegebenen Formel I identisch ist, mit Ausnahme dessen, dass sie eine zusätzliche Doppelbindung in C-3-Stellung oder zwischen dem exocyclischen Stickstoffatom und dem benachbarten Kohlenstoffatom in der Seitenkette aufweist, mit einem Reduktionsmittel behandelt, um diese Doppelbindung zu sättigen, oder

g) um eine Verbindung der Formel I wie vorstehend angegeben, worin X Oxo bedeutet, herzustellen, eine Verbindung der Formel X

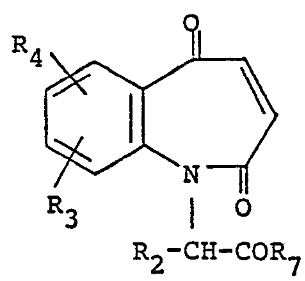

(X)

worin der carbocyclische Ring auch Hexahydro oder 6,7,8,9-Tetrahydro sein kann und worin $R_2$ bis $R_4$ und $R_7$ die vorstehend angegebenen Bedeutungen besitzen, mit einem Amin der Formel VII

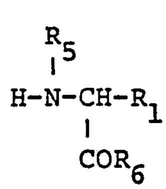

(VII)

worin $R_1$, $R_5$ und $R_6$ die vorstehend angegebenen Bedeutungen besitzen, kondensiert und gewünschtenfalls eine erhaltene Verbindung der Formel I, wie vorstehend definiert, in eine andere Verbindung der Formel I innerhalb des vorstehend angegebenen Bereichs umwandelt und/oder gewünschtenfalls eine erhaltene Verbindung der Formel I wie vorstehend definiert, die salzbildende Eigenschaften aufweist, in ihr Salz überführt oder eine freie Verbindung aus einem derartigen Salz freisetzt und/oder gewünschtenfalls ein optisches Isomeres, das eine spezifische Konfiguration im Hinblick auf zumindest ein Chiralitätszentrum besitzt, aus einer Mischung der stereoisomeren Formen einer erhaltenen Verbindung der Formel I anreichert.

Die Alkylierung gemäss den Verfahren a) und b), wird in herkömmlicher Weise durchgeführt, vorteilhaft, indem man ein entsprechendes Ausgangsmaterial der Formeln II bzw. V mit einem Alkylierungsmittel der Formel (IIIA) bzw. (IIIB) behandelt, worin Z eine reaktive veresterte Hydroxygruppe, wie eine mit einer starken organischen Säure, z. B. einer aliphatischen oder aromatischen Sulfonsäure (wie einer $C_{1-7}$-Alkansulfonsäure, insbesondere Methansulfonsäure, Trifluormethansulfonsäure, insbesondere Benzolsulfonsäure, p-Toluolsulfonsäure, p-Brombenzolsulfonsäure und p-Nitrobenzolsulfonsäure) oder mit einer starken anorganischen Säure, wie insbesondere Schwefelsäure, oder einer Halogenwasserstoffsäure, wie Chlorwasserstoffsäure, oder am meisten bevorzugt Jodwasserstoffsäure oder Bromwasserstoffsäure, veresterte Hydroxygruppe ist. Die Alkylierung wird unter herkömmlichen allgemeinen Bedingungen bei Temperaturen im Bereich von 0 °C bis zur Siedetemperatur der Reaktionsmischung, vorzugsweise bei Temperaturen zwischen Raumtemperatur und 100 °C, durchgeführt. Die Umsetzung findet vorteilhaft in Gegenwart eines Lösungsmittels, das im Hinblick auf die Reaktanten inert ist, statt, wie in Gegenwart eines chlorierten $C_{1-7}$-Alkans (z. B. Chloroform oder Methylenchlorid), eines acyclischen oder cyclischen Äthers (z. B. Diäthyläther, 1,2-Dimethoxyäthan, Dioxan oder Tetrahydrofuran) und insbesondere eines tertiären Amids mit niedrigem Molekulargewicht (z. B. N,N-Dimethylformamid, N,N-Dimethylacetamid, N-Methylpyrrolidon, N-Äthylpiperidon und Hexamethylphosphorsäure-trisamid). Vorteilhafterweise wird die während der Reaktion freigesetzte starke Säure HZ durch Zugabe eines säurebindenden Mittels gebunden, wie vorzugsweise eines anorganischen Säurefängers, wie eines Alkalimetallbicarbonats, -carbonats oder -hydroxids, eines organischen quaternären Ammoniumsalzes (z. B. eines Tetrabutylammoniumsalzes) oder einer organischen tertiären Base, wie Triäthylamin, N-Äthylpiperidin, Pyridin oder Chinolin.

Bei dem Verfahren a) kann die Alkylierung auch unter an sich bekannten und im Stand der Technik verwendeten Bedingungen einer reduktiven Alkylierung durchgeführt werden. Bei Durchführung der Alkylierung wird eine Verbindung der allgemeinen Formel IV

$$R_1—CO—COR_6 \qquad (IV)$$

worin $R_1$ und $R_6$ die vorstehend angegebenen Bedeutungen besitzen, mit der bicyclischen Ausgangsverbindung II und gleichzeitig oder in einer nachfolgenden Stufe mit einem Reduktionsmittel umgesetzt. Unter Reduktionsmitteln, die gleichzeitig mit dem Alkylierungsmittel verwendet werden, sollten die Ameisensäure und komplexe Metallhydride, wie Natriumcyanoborhydrid, erwähnt werden ; unter den Reduktionsmitteln, die überwiegend in einer getrennten nachfolgenden Arbeitsstufe, d. h. Reduktion eines vorher gebildeten Imins (Schiff'scher Base) verwendet werden, sollten Diboran und komplexe Metallhydride, wie Natriumborhydrid und Natriumcyanborhydrid, erwähnt werden, die vorteilhaft zu der primären Reaktionsmischung ohne Isolierung eines Zwischenprodukts, z. B. Imins, zugegeben werden. In diesem Fall wird die Alkylierung vorteilhaft in einem gegenüber dem Reduktionsmittel inerten organischen Lösungsmittel durchgeführt, wie in einem aliphatischen oder cyclischen Äther (wie Diäthyläther, Diisopropyläther, 1,2-Dimethoxyäthan, Dioxan oder Tetrahydrofuran) oder in einem aliphatischen Alkohol (wie Methanol, Äthanol, Isopropylalkohol, Glykol, Glykolmonomethyläther oder Diäthylenglykol), vorzugsweise bei 0 bis 80 °C. Ein wesentliches Reduktionsmittel jedoch, das sowohl gleichzeitig als auch anschließend verwendet werden kann, ist Wasserstoif, insbesondere katalytisch aktivierter Wasserstoff. Die Katalysatoren sind diejenigen, die herkömmlich als Hydrierungskatalysatoren eingesetzt werden, nämlich vorzugsweise diejenigen der Klasse der Edelmetalle (wie Palladium, Platin und Rhodium) auf einem Träger (wie Calciumcarbonat, Aluminiumoxid oder Bariumsulfat), in feindisperser Suspension ohne Träger oder in Form von Komplexen in homogener Phase. Auch feindispergierte Übergangsmetalle, z. B. Raney-Metalle, insbesondere Raney-Nickel, sind sehr geeignete Katalysatoren für die reduktive Alkylierung. Die speziellen Reaktionsbedingungen hängen in großem Ausmaß von dem jeweiligen Hydrierungskatalysator und dessen exakter Aktivität ab und weichen von den allgemein für die Hydrierung bekannten nicht ab. Temperaturen im Bereich von Raumtemperatur bis zu 150 °C und Wasserstoffdrücke im Bereich von Atmosphärendruck bis zu 300 Atmosphären sind gemäß den Standardverfahren des Stands der Technik anwendbar. Zusätzlich zu den vorstehend im Zusammenhang mit der Hydridreduktion erwähnten inerten Lösungsmitteln können auch niedrigmolekulare Amide, insbesondere tertiäre Amide (wie N,N-Dimethylformamid, N,N-Dimethylacetamid, N-Methylpyrrolidon, N-Äthylpiperidon, Hexamethylphosphorsäure-trisamid), jedoch auch Formamid und Acetamid als geeignete Lösungsmittel verwendet werden. Spezielle Maßnahmen müssen bei Ausgangsmaterialien der Formel II angewandt werden, die eine leicht reduzierbare funktionelle Gruppe, wie die 5-Oxogruppe, aufweisen ; um diese Gruppen aufrechtzuerhalten, müssen selektive Reduktionsbedingungen, wie sie aus dem Stand

der Technik bekannt sind, angewandt werden, oder wenn eine gleichzeitige Reduktion dieser Gruppen erwünscht oder erforderlich ist, werden dementsprechend energische Reagentien und/oder Bedingungen angewandt.

Die oben genannten im voraus gebildeten Imine werden vorzugsweise durch Kondensation eines Amins der Formel II mit einer Verbindung der Formel IV, in einem inerten Lösungsmittel, z. B. Toluol oder Methylenchlorid, in erster Linie in Gegenwart von Dehydratisierungskatalysatoren, z. B. Bortrifluoridätherat, p-Toluolsulfonsäure oder Molekularsieben, hergestellt.

Das Verfahren b) wird vorzugsweise in Gegenwart von sehr starken Basen, wie Alkalimetallhydriden (z. B. Natrium- oder Kaliumhydrid), -alkoholaten (z. B. Natrium-methylat oder -äthylat, Kalium-tert.-butylat) oder -amiden (z. B. Lithiumdiisopropylamid) durchgeführt, wobei die vorstehend erwähnten Äther und Amide als Lösungsmittel bevorzugt sind. Bei einer speziellen Ausführungsform des Verfahrens b) werden Ausgangsmaterialien verwendet, worin $R_A$ Wasserstoff bedeutet, und es werden zumindest zwei Aequivalente der Reaktante IIIB verwendet. In dem erhaltenen Produkt sind die beiden eingeführten Reste identisch und liegen im Bereich der Bedeutungen von $-CH(R_2)COR_7$.

In jedem der Alkylierungsverfahren müssen primäre und sekundäre Aminogruppen in den Ausgangsmaterialien, mit Ausnahme der zu alkylierenden sekundären Aminogruppe, in einer vorübergehend geschützten Form während der Alkylierung vorliegen. Geeignete Schutzgruppen sowie Verfahren zu deren Einführung und Abspaltung sind aus dem Stand der Technik, der bis in die Einzelheiten ausgearbeitet ist, insbesondere als allgemeine Methoden für die Synthese von Peptiden, bekannt, vergl. Houben-Weyl: Methoden der organischen Chemie, 4. Ausgabe, Band 15/I und II, E. Wünsch (Herausgeber): Synthese von Peptiden (Georg Thieme-Verlag, Stuttgart, 1974). Die nähere Auswahl der Schutzgruppen hängt von dem speziellen Zweck ab, wobei es erforderlich ist, insbesondere die speziellen Eigenschaften der jeweiligen Ausgangsmaterialien und die Reaktionsbedingungen des jeweiligen Verfahrens mit zu berücksichtigen. Im Fall von verschiedenen zu schützenden funktionellen Gruppen können vorteilhafte Kombinationen ausgewählt werden. Vorzugsweise werden z. B. ähnliche oder besser identische Aminoschutzgruppen sowohl in den Resten $COR_6$ und/oder $COR_7$ als auch im Rest $R_1$ verwendet und gleichzeitig im Anschluss an die Alkylierung abgespalten.

Geeignet als Aminoschutzgruppen sind insbesondere Aminoschutzgruppen, die durch Reduktion entfernt werden können, wie z. B. insbesondere diejenigen vom Benzyloxycarbonyl-Typ, bei denen die Benzyloxycarbonylgruppe im aromatischen Teil durch Halogenatome, $C_{1-7}$-Alkoxygruppen und/oder $C_{1-7}$-Alkylreste und insbesondere durch Nitrogruppen, substituiert sein kann, wie die p-Chlor- und p-Brombenzyloxycarbonyl-, p-Methoxybenzyloxycarbonyl-, p-Methylbenzyloxycarbonyl- und insbesondere p-Nitrobenzyloxycarbonylgruppe, oder alternativ die Isonicotinyloxycarbonylgruppe. Eine vorteilhafte Aminoschutzgruppe ist eine Aethoxycarbonylgruppe, die in der β-Stellung eine durch drei Kohlenwasserstoffreste substituierte Silylgruppe enthält, wie Triphenylsilyl, Dimethyltert.-butylsilyl oder insbesondere Trimethylsilyl. Eine β-(Trihydrocarbylsilyl)-äthoxycarbonylgruppe dieses Typs, wie eine β-(Tri-$C_{1-7}$-alkylsilyl)-äthoxycarbonylgruppe, z. B. insbesondere β-(Trimethylsilyl)-äthoxycarbonyl, bildet mit der zu schützenden Aminogruppe eine entsprechende β-Trihydrocarbylsilyläthoxycarbonylaminogruppe (z. B. die β-Trimethylsilyläthoxycarbonylaminogruppe), die lediglich unter sehr speziellen, sehr milden Bedingungen durch Einwirken von Fluorid-Ionen abgespalten werden kann.

Es ist auch möglich, Gruppen zu verwenden, die durch Acidolyse abgespalten werden können, wie die tert.-Butoxycarbonylgruppen und analoge Gruppen, ebenso diejenigen des Aralkyl-Typs, wie Benzhydryl, Di-(4-Methoxy)-benzhydryl und Triphenylmethyl (Trityl), oder bestimmte Aralkoxycarbonylgruppen vom 2-(p-Biphenylyl)-2-propoxycarbonyl-Typ, die in der CH-A 509 266 beschrieben werden. Es sei bemerkt, dass sich von Estern der Kohlensäuren ableitende Schutzgruppen in den meisten Fällen auch durch basische Hydrolyse abspaltbar sind.

Für den fakultativen vorübergehenden Schutz der Hydroxygruppen können mit Vorteil Schutzgruppen verwendet werden, die durch Reduktion abgespalten werden können, vgl. den vorstehend zitierten Text (Houben-Weyl), und auch Gruppen, die durch Acidolyse entfernt werden können, wie 2-Tetrahydropyranyl, tert.-Butoxycarbonyl und tert.-Butyl. Bevorzugte Hydroxyschutzgruppen, die durch Reduktion abgespalten werden können, sind beispielsweise Benzylgruppen, die an dem aromatischen Teil durch Halogen, $C_{1-7}$-Alkyl, $C_{1-7}$-Alkoxy und/oder insbesondere Nitro, insbesondere die 4-Nitrobenzylgruppe substituiert sein können. Es ist auch möglich, Acylgruppen, die unter schwach basischen Bedingungen abgespalten werden können, wie Formyl oder Trifluoracetyl, zu verwenden.

Für den fakultativen Schutz der Oxogruppen werden diese vorzugsweise als Ketale, insbesondere als Ketale, abgeleitet von $C_{1-7}$-Alkanolen, wie Methanol oder Aethanol, oder vorteilhaft von Athylenglykol, oder als entsprechende Thioketale, vorzugsweise als solche von 1,2-Äthandithiol, geschützt. Alle diese Gruppen können unter den nachstehend angegebenen Bedingungen Oxogruppen in Freiheit setzen.

Die anschliessende Abspaltung der Schutzgruppen gemäss der Erfindung hängt von deren Natur ab und wird in jedem Fall in an sich bekannter herkömmlicher Weise durchgeführt, wobei die allgemeinen Eigenschaften des sich ergebenden Produkts in Betracht gezogen werden. Wenn die Schutzgruppen für Amino, Hydroxy und Oxo so ausgewählt worden sind, dass sie unter ähnlichen Bedingungen abgespalten werden können (insbesondere sind vorliegend bevorzugt die durch Acidolyse oder bei Amino und Hydroxy durch Reduktion abspaltbaren Gruppen, die bereits im einzelnen erwähnt worden sind), werden

alle diese Schutzgruppen mit Vorteil in einem einzigen Arbeitsgang abgespalten ; in speziellen Fällen ist es jedoch möglich, verschiedene Typen von Gruppen zu verwenden und jede von ihnen einzeln abzuspalten.

Die Gruppen, die durch Reduktion abgespalten werden können, insbesondere diejenigen, die halogenierte $C_{1-7}$-Alkylreste (z. B. 2,2,2-Trichloräthylreste), Isonicotinylreste (z. B. Isonicotinyloxycarbonyl), und insbesondere substituierte Benzylreste, vor allem 4-Nitrobenzylreste jeglicher Art enthalten, werden vorzugsweise durch Reduktion mit Zink, gewöhnlich in Gegenwart einer Säure, vorzugsweise Essigsäure, und mit oder ohne Zugabe eines inerten organischen Lösungsmittels, gewöhnlich bei Raumtemperatur abgespalten. Die Abspaltung einer Schutzgruppe durch saure Hydrolyse (Acidolyse) wird im Fall von Gruppen des tert.-Butyl-Typs mit Hilfe von Chlorwasserstoff, Fluorwasserstoff oder Trifluoressigsäure und im Fall von säureempfindlichen Schutzgruppen hauptsächlich mit Hilfe einer $C_{1-7}$ aliphatischen Carbonsäure, wie Ameisensäure und/oder Essigsäure, in Gegenwart von Wasser und gegebenenfalls eines polyhalogenierten $C_{1-7}$-Alkanols oder $C_{1-7}$-Alkanons, wie 1,1,1,3,3,3-Hexafluorpropan-2-ol oder Hexafluoraceton, durchgeführt. Auf diese Weise ist es beispielsweise möglich, eine N-Tritylgruppe durch eine organische Säure, wie Ameisensäure, Essigsäure, Chloressigsäure oder Trifluoressigsäure, in wässerigem oder absolutem Trifluoräthanol als Lösungsmittel (vgl. DE-A-2 346 147) oder durch wäßrige Essigsäure zu spalten, die tert.-Butoxycarbonylgruppe durch Trifluoressigsäure oder Chlorwasserstoffsäure abzuspalten und die 2-(p-Biphenylyl)-isopropoxycarbonylgrpe durch wäßrige Essigsäure oder beispielsweise durch eine Mischung von Eisessig, Ameisensäure (Stärke von 82,8 %) und Wasser (7 : 1 : 2) oder gemäß dem Verfahren in der DE-A-2 346 147 zu entfernen. Die β-Silyläthylestergruppen werden vorzugsweise durch Fluorid-Ionen abgebende Reagentien, beispielsweise Fluoride von quaternären organischen Basen, wie Tetraäthylammoniumfluorid, abgespalten.

Ketalisierte und thioketalisierte Oxogruppen werden in freie Oxogruppen durch Acidolyse mit üblichen starken anorganischen Säuren oder mit Oxalsäure in Gegenwart von Wasser abgespalten, die letztgenannten vorteilhaft durch Behandlung mit einem schwefelbindenden Mittel, z. B. einem Quecksilber(II)-Salz und/oder Cadmiumcarbonat. Schutzgruppen, die gegenüber basischen Bedingungen instabil sind, beispielsweise Formyl, Trifluoracetyl und Kohlensäureestergruppen, können vorsichtig durch Einwirken einer wäßrigen Natrium- oder Kaliumbicarbonat- oder -carbonatlösung oder auch durch Einwirken von wäßrigem Ammoniak in einem organischen Lösungsmittel gewöhnlich bei Raumtemperatur entfernt werden. Die Schutzgruppen werden vorzugsweise unter Reaktionsbedingungen der Beispiele oder unter analogen Bedingungen abgespalten.

Diejenigen der erfindungsgemäßen Endprodukte, die basische Gruppen enthalten, werden in Abhängigkeit von der Art der Isolierung in Form von Basen oder Säureadditionssalzen erhalten. Analog können Produkte mit sauren Gruppen auch in Form von Salzen erhalten werden. Jede Form kann in die andere in bekannter Weise übergeführt werden. Die Basen können aus den Säureadditionssalzen in an sich bekannter Weise übergeführt werden. Die Basen können aus den Säureadditionssalzen in an sich bekannter Weise erhalten werden. Aus den Basen wiederum ist es möglich, Säureadditionssalze, insbesondere therapeutisch verwendbare Säureadditionssalze, durch Umsetzung mit Säuren, beispielsweise mit Säuren des Typs, der die vorstehend erwähnten Salze bildet, zu erhalten. Die Säuren und ihre Salze stehen auch in einem ähnlichen Verhältnis zueinander. Verbindungen, die sowohl eine freie Carboxygruppe als auch eine basische Gruppe enthalten, können in Form innerer Salze vorliegen, und diese werden beispielsweise erhalten, indem man den isoelektrischen Punkt einstellt.

Die Ausgangsmaterialien der Formeln IIIA, IIIB und IV, d. h. die Alkylierungsmittel, sind bekannt, oder wenn sie unbekannt sind, können sie auf einfache Weise nach herkömmlichen Syntheseverfahren hergestellt werden.

Die Ausgangsmaterialien der Formeln II und V können nach herkömmlichen Syntheseverfahren erhalten werden und mit Vorteil in der Weise, die nachstehend eingehender für die speziellen Zwischenprodukte beschrieben und veranschaulicht ist.

Das Verfahren c), das auch eine Alkylierungsreaktion darstellt, wird entsprechend den gleichen allgemeinen Gesichtspunkten und unter den gleichen experimentellen Bedingungen wie die vorstehenden Verfahren a) und b) durchgeführt, wie es im einzelnen vorstehend für die Behandlung mit einem Alkylierungsmittel der Formel IIIA, IIIB oder IV beschrieben wird (nämlich die substitutive Alkylierung oder die reduktive Alkylierung). Die Ausgangsmaterialien der Formel VI können mit Hilfe an sich bekannter herkömmlicher Verfahren hergestellt werden, z. B. in der nachstehend eingehender beschriebenen Weise. Die Amine der Formel VII sind bekannt, oder handelt es sich um unbekannte, sind sie auf einfache Weise mit Hilfe herkömmlicher Synthesemethoden zugänglich.

Das Verfahren d) wird auch in herkömmlicher Weise unter den allgemeinen Bedingungen der Solvolyse durchgeführt, die dafür bekannt sind, daß sie Cyanide (Nitrile) in die freien Carbonsäuren oder ihre Salze, Ester oder Amide überführen.

Für die Umwandlung in eine freie Säure wird die Hydrolyse mit Wasser vorteilhaft in einem inerten organischen Lösungsmittel, das zumindest teilweise mit Wasser mischbar ist, wie Äther (z. B. Diäthyl- und Diisopropyläther, 1,2-Dimethoxyäthan oder insbesondere Dioxan oder Tetrahydrofuran), oder $C_{1-7}$-Alkanolen (z. B. Methanol, Aethanol, Isopropylalkohol, Butylalkohole, insbesondere tert.-Butylalkohol) durchgeführt, wobei eine größere Menge Wasser in den letztgenannten Fällen erforderlich ist, um eine Alkoholyse zu verhindern. Die Hydrolyse kann sowohl durch starke Säuren, insbesondere anorganische

Säuren, wie Schwefelsäure, oder vorzugsweise Halogenwasserstoffsäuren (z. B. Bromwasserstoffsäure oder als erste Wahl Chlorwasserstoffsäure), als auch durch Basen, insbesondere anorganische Basen, wie Hydroxide und Carbonate der Alkalimetalle, z. B. Natrium- und Kaliumhydroxid, katalysiert werden. Die Basen werden gewöhnlich in zumindest stöchiometrischen Anteilen verwendet, die Carbonsäuresalze als primäre Produkte entstehen lassen. Die sauren Katalysatoren werden vorteilhaft in Form einer verdünnten wäßrigen Lösung zur Erzielung des besten Resultats angewandt. Die Endprodukte der Formel I, worin $COR_6$ und $COR_7$ eine veresterte Carboxylgruppe bedeutet, können erhalten werden, indem man die Solvolyse des Nitrils mit dem entsprechenden Alkohol (Alkoholyse) in Gegenwart einer katalytischen Menge einer wasserfreien starken Säure, vorteilhaft von gasförmigem Chlorwasserstoff, durchführt. Gewöhnlich wird ein Überschuß an Alkohol als Lösungsmittel verwendet, jedoch können inerte organische Lösungsmittel, wie acyclische und cyclische Äther (insbesondere die vorstehend erwähnten), und/oder halogenierte $C_{1-7}$-Alkane (insbesondere Chloroform und Dichlormethan) zugegeben werden. Wird die Alkoholyse unter streng wasserfreien Bedingungen durchgeführt, muß das primäre Produkt (Imidoester) vorteilhaft durch Zugabe von Wasser zu der Reaktionsmischung hydrolysiert werden. Andererseits wird, wenn man die Alkoholyse in Gegenwart eines annähernd stöchiometrischen Äquivalents von Wasser durchführt, der gewünschte Ester direkt erhalten.

Um ein entsprechendes Amid zu erhalten, kann ein entsprechendes Nitril der Formel VIII vorzugsweise einer alkalischen Hydrolyse in Gegenwart von Wasserstoffperoxid unterzogen werden.

Die Ausgangsmaterialien der Formel VIII können mit Hilfe an sich bekannter herkömmlicher Methoden, z. B. durch eine Kondensation analog derjenigen des Verfahrens c), erhalten werden, wobei ein Ausgangsmaterial der vorstehend definierten Formel VI mit einem Amin der Formel VII'

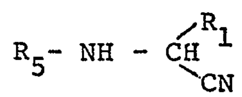

(VII')

worin $R_1$ und $R_5$ die vorstehend angegebenen Bedeutungen besitzen, und das dem vorstehend definierten Amin der Formel VII entspricht, behandelt wird. Auch die Verfahren a) und b) können in analoger Weise zur Herstellung von Nitrilen der Formel VIII verwendet werden.

Die Cyclisierung entsprechend der Verfahrensvariante e) kann auch in an sich bekannter Weise, z. B. durch Dehydratation, durchgeführt werden. Besonders anwendbare allgemeine Methoden für diesen Zweck sind die im Zusammenhang mit der Bildung der Amidbindung in Peptiden entwickelten, wie sie in Sammelwerken, z. B. Houben-Weyl, Band 15/I und Band 15/II, wie vorstehend zitiert, angegeben werden. Gemäß einer bevorzugten Abwandlung wird die zu cyclisierende Aminogruppe durch Protonierung (d. h. in Form eines Säureadditionssalzes) inaktiv gemacht und die Carboxylgruppe in einen aktivierten Ester, wie einen solchen mit 2,4,5-Trichlorphenol, Pentachlorphenol, Pentafluorphenol, 2-Nitrophenol oder insbesondere 4-Nitrophenol, oder mit einer N-Hydroxyverbindung, wie N-Hydroxysuccinimid, 1-Hydroxybenztriazol oder N-Hydroxypiperidin, oder alternativ mit einem N,N'-disubstituierten Isoharnstoff, wie insbesondere N,N'-Dicyclohexylisoharnstoff, oder einem analogen allgemein bekannten aktivierenden Mittel übergeführt. Die Cyclisierung wird durchgeführt, indem man vorzugsweise durch Zugabe einer organischen Base, z. B. eines quaternären Ammoniumsalzes oder insbesondere eines tertiären Amins, wie Triäthylamin, N-Aethylmorpholin oder N-Methylpiperidin, basisch macht, um die zu cyclisierende Aminogruppe durch Umwandlung in die unprotonierte Form zu reaktivieren. Die Reaktionstemperatur beträgt gewöhnlich von − 20 bis + 50 °C, vorzugsweise annähernd Raumtemperatur, und es werden übliche Lösungsmittel verwendet, z. B. Dioxan, Tetrahydrofuran, Acetonitril, Pyridin, Dimethylformamid, Dimethylacetamid, Dimethylsulfoxid, N-Methylpyrrolidon, Hexamethylphosphorsäure-trisamid, sowie Chloroform und Methylenchlorid und bewährte Mischungen derselben. Bei einer speziellen Variante des Verfahrens kann die Carboxygruppe direkt *in situ* durch Einwirken der freien Säure mit einem Carbodiimid, wie N,N'-Dicyclohexylcarbodiimid (gegebenenfalls unter Zusatz von N-Hydroxysuccinimid, eines unsubstituierten oder beispielsweise Halogen-, Methyl- oder Methoxy-substituierten 1-Hydroxybenztriazols oder von 4-Hydroxybenzo-1,2,3-triazin-3-oxyd oder N-Hydroxy-5-norbornen-2,3-dicarboximid) oder mit N,N'-Carbonyldiimidazol aktiviert werden.

Die Ausgangsmaterialien der Formel IX können nach an sich bekannten allgemeinen Methoden, z. B. wie sie nachstehend anhand speziellerer Beispiele erörtert werden, hergestellt werden.

Auch die Reduktion entsprechend dem Verfahren f) kann in einer Weise durchgeführt werden, wie sie an sich allgemein für die Sättigung derartiger Doppelbindungen bekannt ist. Im einzelnen kann die Doppelbindung in den der Formel I entsprechenden ungesättigten Ausgangsmaterialien zwischen C-3 und C-4 oder zwischen C-3 und dem benachbarten Stickstoffatom, oder zwischen diesem exocyclischen Stickstoffatom und dem benachbarten Kohlenstoffatom in der Seitenkette liegen. Die Sättigung der Doppelbindung wird vorteilhaft durch katalytische Hydrierung, beispielsweise unter den im einzelnen vorstehend erörterten bevorzugten Bedingungen sowie auch durch Metallreduktion, wie Zinkreduktion, in neutralem oder saurem Medium oder insbesondere im Fall der C-N-Doppelbindung durch Diboran oder komplexe Hydride, wie Natriumborhydrid, wie vorstehend erwähnt, durchgeführt. Die ungesättigten Ausgangsmaterialien für diese Verfahrensvariante werden nach allgemein bekannten Methoden, z. B. den bei den Verfahren a) und c) und/oder in einer spezielleren Form, wie nachstehend erörtert, erhalten.

Die Kondensation gemäss dem Verfahren g) wird unter herkömmlichen allgemeinen Bedingungen bei Temperaturen im Bereich zwischen 0 °C und 100 °C in einem Lösungsmittel durchgeführt, das gegenüber den Reaktanten inert ist, z. B. Methylenchlorid, 1,2-Dimethoxyäthan, N,N-Dimethylformamid, gegebenenfalls in Gegenwart einer Base, z. B. eines tertiären Amins, wie Triäthylamin, oder eines Alkalimetallhydrids, wie Natriumhydrid.

Bei der Durchführung der fakultativen Umwandlung eines erhaltenen Endprodukts der Formel I in eine andere Verbindung innerhalb des vorstehend angegebenen Bereichs für die Formel I werden Umwandlungen wie die folgenden durchgeführt: eine Aminogruppe wird alkyliert, und/oder eine Oxogruppe, insbesondere diejenige des Symbols X, wird in eine Hydroxygruppe (plus Wasserstoff) oder in zwei Wasserstoffatome durch Reduktion umgewandelt, und/oder Hydroxy wird in Oxo umgewandelt durch Oxidation oder in Wasserstoff durch Reduktion, und/oder eine freie Hydroxy- oder Carboxylgruppe wird aus ihrer veresterten Form durch Hydrolyse oder Hydrogenolyse freigesetzt, und/oder eine Hydroxy- oder Aminogruppe wird acyliert, und/oder ein freies Carboxyl wird verestert, und/oder der aromatische carbocyclische Ring in der Formel I wird hydriert zu Hexahydro oder 6,7,8,9-Tetrahydro, und/oder der Hexahydro-carbocyclische Ring wird dehydriert zu 6,7,8,9-Tetrahydro oder dem aromatischen carbocyclischen Ring.

Alle diese fakultativen Umwandlungen werden nach gut bekannten herkömmlichen Methoden durchgeführt. Durch die Alkylierungsreaktion kann z. B. das durch $R_5$ dargestellte $C_{1-7}$-Alkyl in das Endprodukt der Formel I eingeführt werden, worin $R_5$ Wasserstoff bedeutet, wobei sämtliche der im einzelnen in Verbindung mit der Verfahrensvariante a) erörterten Abwandlungen angewandt werden können. Sowohl die substitutive als auch die reduktive Alkylierung können angewandt werden, die erstgenannte mit Alkylhalogeniden, die letztgenannte mit niedrigen aliphatischen Aldehyden und Ketonen und katalytisch aktiviertem Wasserstoff oder im Fall von Formaldehyd vorteilhaft mit Ameisensäure als Reduktionsmittel. Durch die substitutive Alkylierung können $C_{1-7}$-Alkyle auch in eine Aminogruppe eingeführt werden, die eine Komponente einer durch das Symbol $COR_6$ und/oder $COR_7$ dargestellten Carbamoylgruppe ist. Auch die Reduktion der 5-Oxogruppe in Hydroxy wird in üblicher Weise, z. B. unter Verwendung eines komplexen Metallhydrids, insbesondere eines milden, wie eines Alkalimetallborhydrids (z. B. Natriumborhydrid), oder gemäss der Meerwein-Ponndorf-Methode oder einer Abwandlung derselben unter Verwendung eines Alkanols, insbesondere Isopropylalkohol, sowohl als Lösungsmittel als auch als Reduktionsmittel und eines Metallalkoholats, vorzugsweise eines dem reduzierenden Alkohol entsprechenden, wie Aluminiumisopropylat, als Katalysator durchgeführt. Die Reduktion der Oxogruppe zu zwei Wasserstoffatomen kann vorteilhaft z. B. durch Behandlung mit amalgamiertem Zink und Chlorwasserstoffsäure oder durch Raney-Nickel-Desulfurierung eines entsprechenden Dithioketals bewirkt werden. Die Oxidation von Hydroxy zur Bildung von Oxo kann vorzugsweise mit einem Derivat des sechswertigen Chroms, wie Chromsäure und deren Salze, mit einem Permanganatsalz (insbesondere Kaliumpermanganat) oder unter den Bedingungen der Oppenauer-Oxidation mit Aceton oder Cyclohexanon als Oxidationsmittel und Aluminiumisopropylat als Katalysator durchgeführt werden. Veresterte Hydroxygruppen werden insbesondere mit Hilfe der vorstehend im einzelnen im Zusammenhang mit der Abspaltung der Hydroxyschutzgruppen erörterten Methoden freigesetzt. Die Acylierung von sowohl Hydroxy- als auch Aminogruppen wird auf übliche Weise, vorzugsweise unter Verwendung eines entsprechenden Säureanhydrids oder Halogenids durchgeführt. Zur Veresterung kann eine Carboxylgruppe direkt mit einem Diazoalkan, insbesondere Diazomethan, oder mit einem entsprechenden Alkohol in Gegenwart eines stark sauren Katalysators (z. B. Schwefelsäure oder eine organische Sulfonsäure) und/oder eines Dehydratationsmittels (z. B. Dicyclohexylcarbodiimid) umgesetzt werden. Alternativ kann die Carboxylgruppe in ein reaktives Derivat derselben, wie einen im Zusammenhang mit dem Verfahren e) erwähnten aktiven Ester, oder in ein gemischtes Anhydrid, z. B. mit einem Säurehalogenid (nämlich insbesondere Säurechlorid) oder mit Trifluoressigsäure übergeführt werden und dieses aktivierte Zwischenprodukt mit dem gewünschten Alkohol umgesetzt werden.

Die freie Carboxylgruppe kann aus einem veresterten Carboxyl in allgemein bekannter Weise, insbesondere durch Basen-katalysierte Hydrolyse, freigesetzt werden. Von speziellem Interesse sind jedoch Methoden, die befähigt sind, selektiv eine spezielle durch die Symbole $-COR_6$ und $-COR_7$ wiedergegebene Carboxylgruppe freizusetzen. In einem derartigen Fall kann von einer geeigneten Kombination von Estergruppen Gebrauch gemacht werden, die im Stand der Technik insbesondere als Carboxylschutzgruppen bekannt und in großer Vielfalt insbesondere für die Synthese von Peptiden entwickelt worden sind, vergl. Houben-Weyl, Band 15/I und Band 15/II wie vorstehend zitiert. Für die selektive Abspaltung unter Freisetzung der Carboxylgruppe geeignete Reste sind Ester, die sich z. B. von Alkoholen, die durch Acidolyse entfernbare Reste ergeben, ableiten, wie Cyanmethylalkohol, Benzoylmethylalkohol oder tert.-Butylalkohol, jedoch insbesondere von Alkoholen, die Reste ergeben, die abgespalten werden können durch Reduktion, wie 2,2,2-Trichloräthanol, Benzylalkohol, und insbesondere 4-Nitrobenzylalkohol, oder alternativ Isonicotinylalkohol. Eine besonders bevorzugte Klasse von substituierten Alkanolen sind Aethylalkohole, die in β-Stellung eine trisubstituierte Silylgruppe enthalten, wie Triphenylsilyl, Dimethylbutylsilyl oder insbesondere Trimethylsilyl. Wie beispielsweise in der BE-PS 851 576 beschrieben, sind diese Alkohole besonders zur selektiven Entfernung geeignet, da die entsprechenden β-Silyläthylester, beispielsweise der β-(Trimethylsilyl)-äthylester, die Stabilität herkömmlicher Alkylester besitzen, jedoch selektiv unter milden Bedingungen durch Einwirken von

Fluorid-Ionen unter Beibehaltung anderer veresterter Carboxylgruppen, z. B. von Alkoxycarbonylgruppen, abgespalten werden können.

Die Abspaltung der veresternden Gruppen hängt von ihrer Natur ab und wird in jedem Fall in herkömmlicher, an sich bekannter Weise durchgeführt, wobei man die Eigenschaften der anderen beteiligten Reste mit berücksichtigt. Die Gruppen, die durch Reduktion abgespalten werden können, insbesondere diejenigen, die halogenierte $C_{1-7}$-Alkylreste (beispielsweise 2,2,2-Trichloräthylreste), Isonicotinylreste (z. B. Isonicotinyloxycarbonyl) und gegebenenfalls substituierte Benzylreste, vor allem 4-Nitrobenzylreste jeglicher Art enthalten, werden bevorzugt durch Reduktion mit Zink, gewöhnlich in Gegenwart einer Säure, vorzugsweise Essigsäure, und mit oder ohne Zusatz eines inerten organischen Lösungsmittels, gewöhnlich bei Raumtemperatur abgespalten, diejenigen des Benzyl-Typs, insbesondere unsubstituierte Benzylester, werden auch durch Hydrogenolyse-Techniken, wie sie üblicherweise für Benzylgruppen angewandt werden, entfernt.

Die Spaltung einer Estergruppe durch saure Hydrolyse (Acidolyse) kann insbesondere im Fall von Gruppen des tert.-Butyl-Typs mit Hilfe von Chlorwasserstoff, Fluorwasserstoff oder Trifluoressigsäure durchgeführt werden. Die β-Silyläthylestergruppen werden vorzugsweise durch Fluorid-Ionen erzeugende Reagentien, z. B. Fluoride der quaternären organischen Basen, wie Tetraäthylammoniumfluorid, abgespalten. Estergruppen, die Basen-instabil sind, können vorsichtig durch rasches Einwirken einer wässerigen Natrium- oder Kaliumbicarbonatlösung oder vorzugsweise von wässerigem Ammoniak in einem organischen Lösungsmittel, gewöhnlich bei Raumtemperatur entfernt werden. Die Estergruppen werden vorzugsweise unter den Reaktionsbedingungen der Beispiele oder unter analogen Bedingungen abgespalten.

Eine geeignete Kombination der Estergruppen kann bei den früheren Synthesestufen oder durch eine geeignete Wahl an Ausgangsmaterialien und Reaktanten ausgewählt werden, wobei z. B. beim Verfahren a) eine selektiv abspaltbare Estergruppe mit einem Carboxyl eingeführt wird, das in der letzten Stufe freigesetzt wird.

Die Verbindungen der Formel I werden vorteilhaft entsprechend der Reaktionsfolge 1 hergestellt, die eine zweckmässige Auswahl von Ausgangsmaterialien und Zwischenprodukten beinhaltet und die folgenden Stufen umfasst:

a) die Kondensation unter Bedingungen der basischen Katalyse einer Verbindung der Formel XI

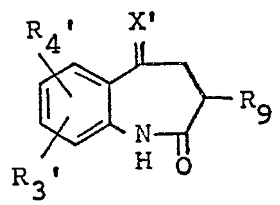

(XI)

worin $R_{3'}$ und $R_{4'}$ Wasserstoff, $C_{1-7}$-Alkyl, $C_{1-7}$-Alkoxy, $C_{1-7}$-Alkanoyloxy, Halogen oder Trifluormethyl bedeuten oder $R_{3'}$ und $R_{4'}$ gemeinsam $C_{1-7}$-Alkylendioxy bedeuten, X' zwei Wasserstoffatome, ein Wasserstoffatom und ein veräthertes oder verestertes Hydroxy, Oxo oder Oxo, geschützt in Form eines Ketals oder Thioketals, bedeutet und $R_9$ Amino, $C_{1-7}$-Alkylamino, Azido oder Acylamino, z. B. $C_{1-7}$-Alkanoylamino oder -Alkoxycarbonylamino, bedeutet, mit einer Verbindung der Formel

$$R_2'-\underset{\displaystyle Z}{\underset{|}{C}H}-COR_7' \qquad \text{(III'B)}$$

worin $R_{2'}$ Wasserstoff oder $C_{1-7}$-Alkyl darstellt, Z reaktiv verestertes Hydroxy bedeutet und $R_{7'}$ Hydroxy, Di-$C_{1-7}$-alkylamino, $C_{1-7}$-Alkoxy, Aryl-$C_{1-7}$-alkoxy, durch $C_{1-7}$-Alkyl, $C_{1-7}$-Alkoxy, $C_{1-7}$-Alkylendioxy, $C_{1-7}$-Alkanoyloxy, Hydroxy, Halogen oder Trifluormethyl mono- oder disubstituiertes Phenyl-$C_{1-7}$-alkoxy, $C_{1-7}$-Alkanoyloxymethoxy oder $C_{1-7}$-Alkoxycarbonyl-$C_{1-7}$-alkoxy bedeutet,

b) gegebenenfalls Reduktion, Hydrogenolyse, Hydrolyse oder Alkylierung des erhaltenen Zwischenprodukts zur Erzielung einer Verbindung der Formel II'

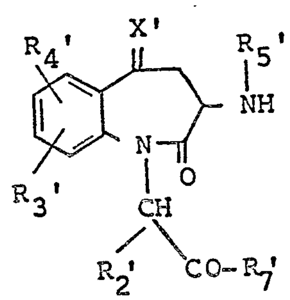

(II')

worin $R_{3'}$, $R_4'$ und X' wie für die Formel XI definiert sind, $R_{2'}$ und $R_{5'}$ Wasserstoff oder $C_{1-7}$-Alkyl bedeuten und $R_{7'}$ hydroxy, Amino, Mono- oder Di-$C_{1-7}$-alkylamino, $C_{1-7}$-Alkoxy, Aryl-$C_{1-7}$-alkoxy, durch $C_{1-7}$-Alkyl, $C_{1-7}$-Alkoxy, $C_{1-7}$-Alkylendioxy, $C_{1-7}$-Alkanoyloxy, Hydroxy, Halogen oder Trifluormethyl mono- oder disubstituiertes Phenyl-$C_{1-7}$-alkoxy, $C_{1-7}$-Alkanoyloxymethoxy, Di-($C_{1-7}$-alkylamino)-$C_{1-7}$-alkoxy oder $C_{1-7}$-Alkoxycarbonyl-$C_{1-7}$-alkoxy bedeutet,

c) Kondensation einer Verbindung der vorstehenden Formel II' unter Bedingungen einer reduktiven Alkylierung mit einer Verbindung der Formel IV'

$$R_1'-\overset{\overset{\displaystyle O}{\|}}{C}-COR_6' \qquad (IV')$$

worin $R_{1'}$ Wasserstoff, $C_{1-7}$-Alkyl, acyliertes Amino-$C_{1-7}$-alkyl, Aryl, durch $C_{1-7}$-Alkyl, $C_{1-7}$-Alkoxy, $C_{1-7}$-Alkylendioxy $C_{1-7}$-Alkanoyloxy, Hydroxy, Halogen oder Trifluormethyl mono- oder disubstituiertes Phenyl, Aryl-$C_{1-7}$-alkyl, durch $C_{1-7}$-Alkyl, $C_{1-7}$-Alkoxy, $C_{1-7}$-Alkylendioxy $C_{1-7}$-Alkanoyloxy, Hydroxy, Halogen oder Trifluormethyl mono- oder disubstituiertes Phenyl-$C_{1-7}$-alkyl, oder Cycloalkyl-$C_{1-7}$-alkyl bedeutet und $R_{6'}$ Hydroxy, Di-$C_{1-7}$-alkylamino, $C_{1-7}$-Alkoxy, Aryl-$C_{1-7}$-alkoxy, durch $C_{1-7}$-Alkyl, $C_{1-7}$-Alkoxy, $C_{1-7}$-Alkylendioxy $C_{1-7}$-Alkanoyloxy, Hydroxy, Halogen oder Trifluormethyl mono- oder disubstituiertes Phenyl-$C_{1-7}$-alkoxy, $C_{1-7}$-Alkanoyloxymethoxy oder $C_{1-7}$-Alkoxycarbonyl-$C_{1-7}$-alkoxy bedeutet oder Kondensation unter Alkylierungsbedingungen einer Verbindung der vorstehenden Formel II' mit einer Verbindung der Formel III'A

$$R_1'-\underset{\underset{\displaystyle Z}{|}}{CH}-COR_6' \qquad (III'A)$$

worin $R_1'$ und $R_6'$ die für die Formel IV' angegebenen Bedeutungen besitzen und Z reaktiv verestertes Hydroxy bedeutet,

d) gegebenenfalls Hydrolyse des erhaltenen produkts oder Ueberführen des erhaltenen Produkts in ein Derivat,

e) Umwandlung irgendeiner erhaltenen Verbindung der Formel I in eine andere erfindungsgemäße Verbindung.

Die Verbindungen der Formel XI werden aus den entsprechenden gegebenenfalls substituierten und/oder in Derivate übergeführten 2,3,4,5-Tetrahydro-1H-[1] benzazepin-2-onen [J. Chem. Soc. *1937,* 456 ; GB-PS 1 359 285 ; Liebig's Annalen der Chemie, *574,* 171 (1951)] erhalten. Neue in geeignete Derivate übergeführte Ausgangs-[1] benzazepin-2-one werden vorteilhaft durch Beckmann-Umlagerung der entsprechenden Derivate von Naphthalin-1-onen unter Verwendung aus dem Stand der Technik bekannter und vorliegend veranschaulichter Verfahren hergestellt.

Die Tetrahydro-[1] benzazepin-2-one werden in die 3-Halo-, z. B. 3-Chlor-2,3,4,5-tetrahydro-1H-[1] benzazepin-2-one, unter vorliegend veranschaulichten Bedingungen, z. B. durch Behandlung mit Phosphorpentachlorid und anschliessende Hydrierung, umgewandelt. Die Substitution dieser Halo-Derivate mit einem Metallazid, z. B. Natriumazid, und etwaige Reduktion oder Substitution mit Ammoniak oder einem $C_{1-7}$-Alkylamin und etwaige Acylierung ergibt Verbindungen der Formel XI.

Alternativ werden die Verbindungen der Formel XI, worin $R_9$ Amino, Alkylamino oder Acylamino bedeutet, durch Reduktion und Cyclisierung der geeignet substituierten und/oder in ein Derivat überführten 4-(o-Nitrophenyl)-2-aminobuttersäure und gegebenenfalls anschliessende N-Alkylierung oder N-Acylierung erhalten.

Eine andere Synthese der erfindungsgemäßen optisch aktiven Verbindungen geht von der natürlichen Aminosäure Tryptophan aus. Im einzelnen wird L-4-(o-Aminophenyl)-4-oxo-2-aminobuttersäure [L-Kynurenin, J. Am. Chem. Soc. *76,* 1708 (1954), abgeleitet von L-Tryptophan] in ein optisch aktives Ausgangsmaterial der Formel XI übergeführt, worin $R_9$ Acylamino, z. B. 3-(S)-tert.-Butyloxycarbonylamino-2,3,4,5-tetrahydro-1H-[1] benzazepin-2,5-dion, bedeutet, wie in Australian Journal of Chemistry *33,* 633-40 (1980) beschrieben. Die Lactam-Alkylierung einer Verbindung der Formel XI mit einer Reaktante der Formel III'B, die aus dem Stand der Technik gut bekannt ist, wird vorzugsweise in Gegenwart von Basen, wie Alkalimetallhydriden, z. B. Natrium- oder Kaliumhydrid, Alkalimetallalkoholaten, z. B. Kalium-tert.-butylat oder Natrium-methylat, organometallischen Reagentien, z. B. Lithium-diisopropylamid, oder unter Bedingungen der Phasen-Transfer-Katalyse, z. B. in Gegenwart eines Tetrabutylammoniumsalzes, vorzugsweise in einem Lösungsmittel, z. B. Tetrahydrofuran, Dimethylformamid, bei einer Temperatur von vorzugsweise zwischen etwa 0 und 75 °C durchgeführt.

Die Kondensation der Zwischenprodukte der Formel II' mit den bekannten α-Ketosäure-Derivaten der Formel IV' (vgl. Chem. Ber. *31*, 551, 3133) durch reduktive N-Alkylierung wird unter aus dem Stand der Technik bekannten Bedingungen, z. B. durch katalytische Hydrierung mit Wasserstoff in Gegenwart von Platin-, Palladium- oder Nickel-Katalysatoren oder mit chemischen Reduktionsmitteln, wie einfachen oder komplexen Leichtmetallhydriden, vorteilhaft einem Alkalimetallcyanborhydrid, wie Natriumcyanborhydrid, durchgeführt. Die reduktive Aminierung mit einem Alkalimetallcyanborhydrid wird vorzugsweise in einem inerten Lösungsmittel, z. B. Methanol oder Acetonitril, vorteilhaft in Gegenwart einer Säure, z. B. Chlorwasserstoffsäure oder Essigsäure, bei einer Temperatur zwischen etwa 0 und 50 °C, vorzugsweise Raumtemperatur, durchgeführt.

Die Alkylierung der Zwischenprodukt-Amine der Formel II' mit einer Reaktante der Formel III'A, die aus dem Stand der Technik gut bekannt ist, wird mit oder ohne basische Katalysatoren, wie Triäthylamin oder Kaliumcarbonat in einem inerten Lösungsmittel durchgeführt.

Die Verbindungen der Formel I können auch durch die Folgen 2 und 3 hergestellt werden.

Die Folge 2 umfaßt die folgenden Stufen :

a) die Kondensation unter Bedingungen einer reduktiven Alkylierung einer Verbindung der Formel

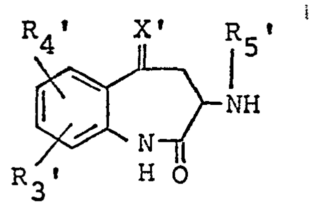

(XII)

worin $R_3'$, $R_4'$ und X' die für die Formel XI angegebenen Bedeutungen besitzen und $R_5'$ Wasserstoff oder $C_{1-7}$-Alkyl ist, mit einer Verbindung der Formel IV'

$$R_1'\text{-}\overset{O}{\overset{\|}{C}}\text{-}CO\text{-}R_6' \qquad (IV')$$

worin $R_1'$ und $R_6'$ wie zuvor definiert sind, oder unter Alkylierungsbedingungen mit einer Verbindung der Formel III'A

$$R_1'\text{-}\underset{Z}{\underset{|}{CH}}\text{-}COR_6' \qquad (III'A)$$

worin $R_1'$, $R_6'$ und Z wie vorstehend definiert sind, zur Erzielung einer Verbindung der Formel V'

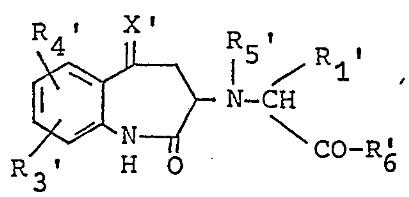

(V')

worin $R_1'$, $R_3'$, $R_4'$, $R_5'$, $R_6'$ und X' wie vorstehend definiert sind,

b) die Kondensation unter Bedingungen einer basischen Katalyse einer erhaltenen Verbindung der Formel V' mit einer Verbindung der Formel III'B

$$R_2'\text{-}\underset{Z}{\underset{|}{CH}}\text{-}COR_7' \qquad (III'B)$$

worin $R_2'$, $R_7'$ und Z wie vorstehend definiert sind,

c) gegebenenfalls Hydrolyse des erhaltenen Produkts oder Ueberführung des erhaltenen Produkts in ein Derivat,

d) gegebenenfalls Umwandlung irgendeiner erhaltenen Verbindung der Formel I in eine andere erfindungsgemässe Verbindung.

Die Folge 3 umfaßt die folgenden Stufen :

a) die Kondensation einer Verbindung der Formel VII′

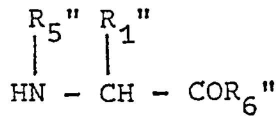

(VII′)

worin $R_1''$ Wasserstoff, $C_{1-7}$-Alkyl, acyliertes Amino-$C_{1-7}$-alkyl, Aryl, Aryl-$C_{1-7}$-alkyl, durch $C_{1-7}$-Alkyl, $C_{1-7}$-Alkoxy, $C_{1-7}$-Alkylidendioxy, $C_{1-7}$-Alkanoyloxy, Hydroxy, Halogen oder Trifluormethyl mono- oder disubstituiertes Phenyl oder Phenyl-$C_{1-7}$ alkyl oder Cycloalkyl-$C_{1-7}$-alkyl bedeutet, $R_5''$ Wasserstoff oder $C_{1-7}$-Alkyl bedeutet und $R_6''$ Hydroxy, Di-$C_{1-7}$-alkylamino, $C_{1-7}$-Alkoxy, Aryl-$C_{1-7}$-alkoxy, durch $C_{1-7}$-Alkyl, $C_{1-7}$-Alkoxy, $C_{1-7}$-Alkylidendioxy, $C_{1-7}$-Alkanoyloxy, Hydroxy, Halogen oder Trifluormethyl mono- oder disubstituiertes Phenyl-$C_{1-7}$-alkoxy, $C_{1-7}$-Alkanoyloxymethoxy oder $C_{1-7}$-Alkoxycarbonyl-$C_{1-7}$-alkoxy bedeutet, mit einer Verbindung der Formel VI′

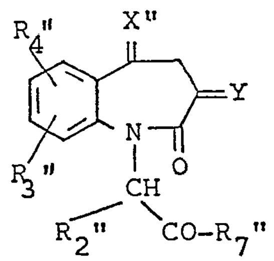

(VI′)

worin $R_2''$ Wasserstoff oder $C_{1-7}$-Alkyl bedeutet, $R_3''$ und $R_4''$ Wasserstoff, $C_{1-7}$-Alkyl, $C_{1-7}$-Alkoxy, $C_{1-7}$-Alkanoyloxy, Halogen oder Trifluormethyl bedeuten oder $R_3''$ und $R_4''$ gemeinsam $C_{1-7}$-Alkylendioxy bedeuten, X″ zwei Wasserstoffatome, ein Wasserstoffatom und ein veräthertes oder verestertes Hydroxy, Oxo oder Oxo, geschützt in Form eines Ketals oder Thioketals, bedeutet, $R_7''$ Hydroxy, Di-$C_{1-7}$-alkylamino, $C_{1-7}$-Alkoxy, Aryl-$C_{1-7}$-alkoxy, durch $C_{1-7}$-Alkyl, $C_{1-7}$-Alkoxy, $C_{1-7}$-Alkylendioxy, $C_{1-7}$-Alkanoyloxy, Hydroxy, Halogen oder Trifluormethyl mono- oder disubstituiertes Phenyl-$C_{1-7}$-alkoxy, $C_{1-7}$-Alkanoyloxymethoxy oder $C_{1-7}$-Alkoxycarbonyl-$C_{1-7}$-alkoxy bedeutet und Y Oxo oder Dichlor bedeutet, unter Bedingungen einer reduktiven N-Alkylierung oder Kondensation einer Verbindung der Formel VII′ mit einer Verbindung der vorstehenden Formel VI′, worin X″ Oxo bedeutet, Y Wasserstoff und ein reaktiv verestertes oder veräthertes Hydroxy bedeutet, oder mit einem 3,4-Dehydro-Eliminierungsprodukt dieser Verbindung oder mit einem 3,4-Dehydro-Derivat dieser Verbindung,

b) gegebenenfalls Reduktion, Hydrolyse oder Derivatisierung des erhaltenen Produkts,

c) gegebenenfalls Umwandlung irgendeiner erhaltenen Verbindung in eine andere erfindungsgemässe Verbindung.

Bei den vorstehenden Folgen 2 und 3 werden die Stufen der Lactam-Alkylierung, reduktiven N-Alkylierung und Alkylierung von Aminen vorteilhaft unter den für das Verfahren 1 beschriebenen Bedingungen durchgeführt.

Bei den vorliegend beschriebenen Reaktionsfolgen 1, 2 und 3 können die Reaktanten beispielsweise der Formeln III′A, III′B und VII′ durch die entsprechenden Nitrile, z. B. $R_2'CH(Z)CN$, $R_1'CH(Z)CN$ bzw. $R_5NHCH(R_1')CN$ ersetzt werden. Die so erhaltenen Nitrile können in die entsprechenden Carbonsäuren, Ester und Amide der Formel I unter Verwendung von aus dem Stand der Technik bekannten Methoden übergeführt werden.

Die Ausgangsmaterialien der Formel VII′ stellen Aminosäuren und Derivate, die aus dem Stand der Technik bekannt sind, dar. Es ist erwähnenswert, daß die optisch aktiven erfindungsgemäßen Verbindungen ausgehend von einer optisch aktiven Verbindung der Formel VII′, z. B. von L-α-Aminophenylbuttersäure, L-Phenylalanin und deren Derivaten, hergestellt werden können.

Im Fall der Reaktanten der Formeln III′A, III′B, IV′ und VII′, worin $R_7'$, $R_6'$ oder $R_6''$ Hydroxy bedeuten, kann ein geeignetes Carboxylatsalz, vorzugsweise *in situ*, vor der Kondensation mit den oben genannten, gewünschten Zwischenprodukten hergestellt werden.

Bestimmte bei den vorstehenden Verfahren verwendete Bezeichnungen besitzen die nachstehend definierten Bedeutungen.

Eine reaktiv veresterte Hydroxygruppe bedeutet eine durch eine starke anorganische oder organische Säure, vor allem Halogenwasserstoffsäure, z. B. Chlorwasserstoffsäure, Bromwasserstoffsäure oder Jodwasserstoffsäure, eine aliphatische oder aromatische Sulfonsäure, z. B. Methansulfonsäure oder p-Toluolsulfonsäure, veresterte Hydroxygruppe.

Veräthertes Hydroxy bedeutet vorzugsweise $C_{1-7}$-Alkoxy, z. B. Methoxy, Aethoxy oder tert.-Butoxy.

Die fakultativen Stufen einer Reduktion, Hydrogenolyse, Hydrolyse oder eines Ueberführens in ein

Derivat der Ausgangsprodukte der vorstehenden Verfahren und die Umwandlung eines erhaltenen Produkts in eine andere erfindungsgemässe Verbindung werden mit Hilfe der aus dem Stand der Technik bekannten und vorliegend veranschaulichten chemischen Arbeitsweisen durchgeführt.

Die Verbindungen der Formel I, worin $R_6$ und/oder $R_7$ $C_{1-7}$-Alkoxy bedeuten, können mit Ammoniak, Mono- oder Di-$C_{1-7}$-alkylaminen amidiert werden, um Verbindungen der Formel I zu ergeben, worin $R_6$ und/oder $R_7$ unsubstituiertes, Mono- oder Di-$C_{1-7}$-alkylamino bedeuten.

Die Umwandlung der Verbindungen der Formel I, worin $R_6$ und/oder $R_7$ $C_{1-7}$-Alkoxy, Aryl-$C_{1-7}$-alkoxy, durch $C_{1-7}$-Alkyl, $C_{1-7}$-Alkoxy, $C_{1-7}$-Alkylendioxy, $C_{1-7}$-Alkanoyloxy, Hydroxy, Halogen oder Trifluormethyl mono- oder disubstituiertes Phenyl-$C_{1-7}$-alkoxy, Amino, Mono- oder Di-($C_{1-7}$-alkyl)-amino bedeuten in die Verbindungen der Formel I, worin $R_6$ und/oder $R_7$ Hyroxy bedeuten, wird vorteilhaft durchgeführt durch Hydrolyse mit anorganischen Säuren, wie Halogenwasserstoffsäuren oder Schwefelsäure, oder mit wässrigen Alkalien, vorzugsweise Alkalimetallhydroxiden, wie Lithium- oder Natriumhydroxid.

Die selektive Umwandlung von Verbindungen der Formel I, worin $R_6$ und/oder $R_7$ $\alpha$-Aryl-$C_{1-7}$-alkoxy, z. B. Benzyloxy, bedeuten, in Verbindungen der Formel I, worin $R_6$ und/oder $R_7$ Hydroxy bedeuten, wird vorteilhaft durch Hydrogenolyse unter Verwendung von Wasserstoff in Gegenwart eines Katalysators, z. B. Palladium, durchgeführt.

Die Verbindungen der Formel I, worin weder $R_6$ noch $R_7$ Hydroxy bedeuten, können in Monocarbonsäuren der Formel I übergeführt werden, worin einer der Reste $R_6$ und $R_7$ Hydroxy bedeutet. Eine derartige Umwandlung wird durch selektive hydrolytische oder hydrogenolytische Verfahren, die aus dem Stand der Technik gut bekannt sind und sich nach dem chemischen Charakter der $R_6$- und $R_7$-Substituenten richten, durchgeführt.

Die freien Carbonsäuren der Formel I, worin $R_6$ und/oder $R_7$ Hydroxy bedeuten oder deren Salze, können mit geeigneten aus dem Stand der Technik gut bekannten Alkoholen oder deren reaktiven Derivaten verestert werden, um die entsprechenden Mono- oder Di-ester, nämlich die Verbindungen der Formel I zu ergeben, worin $R_6$ und/oder $R_7$ $C_{1-7}$-Alkoxy, Aryl-$C_{1-7}$-alkoxy, durch $C_{1-7}$-Alkyl, $C_{1-7}$-Alkoxy, $C_{1-7}$-Alkylendioxy, $C_{1-7}$-Alkanoyloxy, Hydroxy, Halogen oder Trifluormethyl mono- oder disubstituiertes Phenyl-$C_{1-7}$-alkoxy, $C_{1-7}$-Alkanoyloxymethoxy oder $C_{1-7}$-Alkoxycarbonyl-$C_{1-7}$-alkoxy bedeuten. Weiterhin können die freien Carbonsäuren über die reaktiven Zwischenprodukte in die Mono- oder Bis-amide der Formel I übergeführt werden, worin $R_6$ und/oder $R_7$ Amino, Mono- oder Di-$C_{1-7}$-alkylamino bedeuten.

Die Verbindungen der Formel I und die Zwischenprodukte hierfür, z. B. der Formeln X und V', worin X oder X' Oxo bedeutet, können in die entsprechenden Verbindungen, worin X oder X' ein Wasserstoffatom und eine Hydroxygruppe bedeutet, durch Reduktion, z. B. katalytische Hydrierung, z. B. Wasserstoff in Gegenwart eines Platinkatalysators, oder mit einem Metallhydrid-Reduktionsmittel, wie Natriumborhydrid, übergeführt werden. Erhaltene Verbindungen, worin X oder X' ein Wasserstoffatom und eine Hydroxygruppe bedeutet, können in die Verbindungen, worin X oder X' zwei Wasserstoffatome bedeutet, z. B. durch katalytische Hydrierung des Addukts eines Carbodiimids, beispielsweise des Addukts, gebildet durch Kondensation einer Verbindung, worin X oder X' ein Wasserstoffatom und eine Hydroxygruppe bedeutet, mit Dicyclohexylcarbodiimid in Gegenwart von Cuprochlorid gemäß der allgemeinen, in Chem. Ber., *107,* 1353 (1974) beschriebenen Methode übergeführt werden.

Alternativ können die Verbindungen, worin X oder X' ein Wasserstoffatom und eine Hydroxygruppe bedeutet, zuerst in die entsprechenden Verbindungen, worin X oder X' ein Wasserstoffatom und eine Acyloxygruppe (z. B. Acetoxy) bedeutet, übergeführt und anschließend z. B. durch katalytische Hydrierung in Gegenwart eines Palladiumkatalysators, zu Verbindungen reduziert werden, worin X oder X' zwei Wasserstoffatome bedeutet.

Die vorstehenden Reaktionen werden nach Standard-Methoden in Gegenwart oder Abwesenheit von Verdünnungsmitteln, vorzugsweise solchen, die gegenüber den Reagentien inert sind und deren Lösungsmittel darstellen, von Katalysatoren, Kondensationsmitteln bzw. den anderen Mitteln und/oder inerten Atmosphären, bei niedrigen Temperaturen, Raumtemperatur oder erhöhten Temperaturen, vorzugsweise beim Siedepunkt der verwendeten Lösungsmittel, bei atmosphärischem oder überatmosphärischem Druck, durchgeführt.

Die Erfindung umfaßt weiterhin jegliche Variante der vorliegenden Verfahren, bei der ein bei irgendeiner Stufe derselben erhältliches Zwischenprodukt als Ausgangsmaterial verwendet wird und die verbliebenen Stufen ausgeführt werden, oder das Verfahren bei irgendeiner Stufe desselben unterbrochen wird oder bei der die Ausgangsmaterialien unter Reaktionsbedingungen gebildet werden oder bei der die Reaktionskomponenten in Form ihrer Salze oder optisch reinen Antipoden verwendet werden. Hauptsächlich diejenigen Ausgangsmaterialien sollten bei diesen Reaktionen verwendet werden, die zur Bildung der vorstehend als besonders wertvoll angegebenen Verbindungen führen.

Die Erfindung betrifft auch neue Ausgangsmaterialien und Verfahren zu deren Herstellung.

In Abhängigkeit von der Wahl der Ausgangsmaterialien und Methoden können die neuen Verbindungen in Form eines der möglichen Isomeren oder von deren Gemischen vorliegen, beispielsweise in Abhängigkeit von der Anzahl der asymmetrischen Kohlenstoffatome, als reine optische Isomere, wie die Antipoden, oder als Gemische von optischen Isomeren, wie die Racemate, oder als Gemische von Diastereoisomeren.

Erhaltene Gemische der Diastereoisomere und Gemische der Racemate können auf Basis der

physiko-chemischen Unterschiede der Bestandteile in bekannter Weise in die reinen Isomere, Diastereoisomere oder Racemate getrennt werden, z. B. durch Chromatographie und/oder fraktionierte Kristallisation.

Die erhaltenen Racemate können weiterhin in die optischen Antipoden nach an sich bekannten Methoden, beispielsweise durch Umkristallisation aus einem optisch aktiven Lösungsmittel, mit Hilfe von Mikroorganismen oder durch Umsetzung eines sauren Endprodukts mit einer optisch aktiven Base, die Salze mit der racemischen Säure bildet, und Trennung der auf diese Weise erhaltenen Salze, beispielsweise auf Basis ihrer unterschiedlichen Löslichkeiten, in die Diastereoisomere, aus denen die Antipoden durch Einwirken geeigneter Mittel freigesetzt werden können, aufgetrennt werden. Basische racemische Produkte können gleichfalls in die Antipoden, z. B. durch Trennung von deren diastereoisomeren Salzen, z. B. durch fraktionierte Kristallisation von deren d- oder l-Tartraten, getrennt werden. Irgendwelche racemischen Zwischenprodukte oder Ausgangsmaterialien können in ähnlicher Weise getrennt werden.

Zweckmäßigerweise wird der aktivere der beiden Antipoden isoliert.

Schließlich werden die erfindungsgemäßen Verbindungen entweder in freier Form oder in Form von deren Salzen erhalten. Irgendeine erhaltene Base kann in ein entsprechendes Säureadditionssalz, vorzugsweise unter Verwendung einer pharmazeutisch verträglichen Säure oder eines Anionenaustausch-Präparates, übergeführt werden, oder erhaltene Salze können in die entsprechenden freien Basen, beispielsweise unter Verwendung einer stärkeren Base, wie eines Metall- oder Ammoniumhydroxids oder eines basischen Salzes, z. B. eines Alkalimetallhydroxids oder -carbonats, oder einer Kationenaustausch-Präparates, übergeführt werden. Eine Verbindung der Formel I,
worin $COR_6$ und/oder $COR_7$ Carboxy bedeuten, kann somit auch in die entsprechenden Metall- oder Ammoniumsalze übergeführt werden. Diese oder andere Salze, beispielsweise die Pikrate, können auch für die Reinigung der erhaltenen Basen verwendet werden. Die Basen werden in die Salze übergeführt, die Salze werden getrennt, und die Basen werden aus den Salzen freigesetzt. Im Hinblick auf die enge Verwandtschaft zwischen den freien Verbindungen und den Verbindungen in Form ihrer Salze ist, wenn immer in diesem Zusammenhang auf eine Verbindung Bezug genommen wird, auch ein entsprechendes Salz mit beabsichtigt, vorausgesetzt, daß es unter den gegebenen Umständen möglich oder geeignet ist.

Die Verbindungen einschließlich ihrer Salze können auch in Form ihrer Hydrate erhalten werden oder andere für die Kristallisation verwendete Lösungsmittel einschließen.

Die erfindungsgemäßen pharmazeutischen Präparate sind solche, die für die enterale, wie die orale oder rektale, und die parenterale Verabreichung an Säuger, einschließlich des Menschen, zur Behandlung oder Verhinderung von Erkrankungen geeignet sind, die auf die Inhibierung des Angiotensin-umwandelnden Enzyms ansprechen, z. B. von kardiovaskulären Erkrankungen, wie der Hypertension, und von kongestiver Herzinsuffizienz und umfassen eine wirksame Menge einer pharmakologisch aktiven Verbindung der Formel I oder eines pharmazeutisch verträglichen Salzes derselben, allein oder in Kombination mit einem oder mehreren pharmazeutisch verträglichen Trägern.

Die pharmakologisch aktiven Verbindungen der Erfindung sind bei der Herstellung von pharmazeutischen Zusammensetzungen verwendbar, die eine wirksame Menge derselben in Verbindung oder Beimischung mit Excipienten oder Trägern, die für die enterale oder parenterale Anwendung geeignet sind, umfassen. Bevorzugt sind Tabletten und Gelatinekapseln, die den aktiven Bestandteil zusammen mit a) Verdünnungsmitteln, z. B. Lactose, Dextrose, Sucrose, Mannit, Sorbit, Cellulose und/oder Glycin, b) Gleitmitteln, z. B. Siliciumdioxid, Talk, Stearinsäure, deren Magnesium- oder Calciumsalz und/oder Polyäthylenglykol, für Tabletten auch c) Bindemitteln, z. B. Magnesiumaluminiumsilicat, Stärkepaste, Gelatine, Tragant, Methylcellulose, Natriumcarboxymethylcellulose und/oder Polyvinylpyrrolidon, gewünschtenfalls d) Zerteilungs- bzw. Desintegrationsmitteln, z. B. Stärken, Agar, Alginsäure oder deren Natriumsalz, oder schäumenden Mischungen und/oder e) Absorbentien, farbgebenden Mitteln, Geschmacksstofen und süßenden Mitteln umfassen. Injizierbare Präparate sind vorzugsweise wäßrige isotonische Lösungen oder Suspensionen, und Suppositorien werden vorteilhaft aus Fettemulsionen oder -suspensionen hergestellt. Diese Zusammensetzungen können sterilisiert werden und/oder Adjuvantien, wie Konservierungs-, Stabilisierungs-, Netz- oder Emulgiermittel, Lösungspromotoren, Salze für die Regulierung des osmotischen Drucks und/oder Puffer, enthalten. Zusätzlich können sie auch andere therapeutisch wertvolle Substanzen enthalten. Diese Präparate werden nach herkömmlichen Misch-, Granulier- bzw. Überzugsmethoden hergestellt und enthalten 0,1 bis 75 %, vorzugsweise 1 bis 50 %, des aktiven Bestandteils. Eine Einheitsdosis für einen Säuger von 50 bis 70 kg kann zwischen 10 und 200 mg aktiven Bestandteil enthalten.

Die folgenden Beispiele sollen die Erfindung erläutern und keine Einschränkungen darstellen. Die Temperaturen sind in °C angegeben, und sämtliche Teile sind in Form von Gewichtsteilen angegeben. Wenn nicht anders erwähnt, werden sämtliche Verdampfungen unter vermindertem Druck, vorzugsweise zwischen 15 und 100 mm Hg, durchgeführt.

Im Fall der Verbindungen der Formel I, worin mehr als ein Asymmetrie-Zentrum vorliegt, werden die erhaltenen diastereoisomeren Verbindungen als A, B etc. in den betreffenden Beispielen bezeichnet. Die jeweiligen diastereoisomeren Verbindungen werden durch die physikalischen Eigenschaften, z. B. den Schmelzpunkt, die relative Wanderung bei der Chromatographie sowie die spektralen Eigenschaften bei der Infrarot- oder kernmagnetischen Resonanz-Messung charakterisiert.

Im Fall der Verbindungen der Formel I, worin X $H_2$ bedeutet und ein Asymmetrie-Zentrum in der Seitenkette an dem das Stickstoffatom tragenden Kohlenstoffatom vorliegt, wurden die Symbole A und B wie folgt den jeweiligen Isomeren auf Basis ihrer relativen Wanderung bei der Chromatographie zugeordnet. Auf Basis der Wanderung bei der Dünnschichtchromatographie und Normalphasen-Hochdruck-Flüssigkeits-Chromatographie unter Verwendung von Siliciumdioxidgel als stationäre Phase wird das sich schnell bewegende Isomer als Isomer A und das sich langsam bewegende Isomer als Isomer B bezeichnet. Auf Basis der Wanderung bei der Hochdruck-Flüssigkeits-Chromatographie mit reverser Phase wird das sich langsam bewegende Isomer als Isomer A und das sich schnell bewegende Isomer als Isomer B bezeichnet.

Beispiel 1

1-Carboxymethyl-3-(1-äthoxycarbonyl-3-phenylpropylamino)-2,3,4,5-tetrahydro-1H-[1] benzazepin-2-on (Das höher schmelzende Isomer)

Eine Lösung von 3-Amino-1-carboxymethyl-2,3,4,5-tetrahydro-1H-[1]-benzazepin-2-on (10,0 g) und Benzylpyruvinsäure-äthylester (26,4 g) in Essigsäure (75 ml) und Methanol (75 ml) wird unter Stickstoff bei Zimmertemperatur eine Stunde gerührt. Das Reaktionsgemisch wird dann innerhalb 4 Stunden mit Natriumcyanborhydrid (3,4 g) in Methanol (25 ml) tropfenweise versetzt und bei Zimmertemperatur 24 Stunden gerührt. Das Gemisch wird mit konz. Chlorwasserstoffsäure (4 ml) tropfenweise versetzt, bei Zimmertemperatur 1 Stunde gerührt und zur Trockene eingedampft. Der Rückstand wird zwischen 150 ml Wasser und 50 ml Aether verteilt und mit 40 %iger wässeriger Natriumhydroxidlösung auf den pH-Wert 9 eingestellt. Die Schichten werden getrennt und die Aetherschicht wird verworfen. Die wässerige Schicht wird mit konz. Chlorwasserstoffsäure auf den pH-Wert 4,3 eingestellt und mit 3 × 75 ml Essigsäureäthylester extrahiert. Die organischen Extrakte werden über Magnesiumsulfat getrocknet und zur Trockene eingedampft. Chlorwasserstoffgas wird 5 Minuten in die Lösung des Rohproduktes in 310 ml Methylenchlorid eingeleitet. Die Lösung wird eingedampft und der Rückstand in 225 ml Aether gerührt. Das Produkt wird abfiltriert. Man erhält ein 70 : 30 diastereomeres Gemisch, wie dies durch Hochdruck-Flüssigkeitschromatographie festgestellt ist. Das Produkt wird aus Aethanol/Essigsäureäthylester (1 : 3) umkristallisiert. Man erhält das 1-Carboxymethyl-3-(1-äthoxycarbonyl-3-phenylpropylamino)-2,3,4,5-tetrahydro-1H-[1] benzazepin-2-on-hydrochlorid, welches bei 246-248° (unter Zersetzung) schmilzt und dem racemischen Isomer B entspricht.

Eine Lösung des obigen Hydrochloridsalzes (0,9 g) und von Propylenoxid (10 ml) in Aethanol (150 ml) wird 18 Stunden unter Stickstoff gerührt. Die Lösung wird zur Trockene eingedampft und der Rückstand in 3 ml Aethanol aufgelöst. Aether (75 ml) wird zugegeben, welcher eine geringe Menge des obigen Hydrochlorids ausfällt. Das Filtrat wird zur Trockene eingedampft, mit Aether/Petroläther (1 : 9) gerührt und das feste Material abfiltriert. Man erhält das 1-Carboxymethyl-3-(1-äthoxycarbonyl-3-phenylpropylamino)-2,3,4,5-tetrahydro-1H-[1] benzazepin-2-on, welches bei 139-141° schmilzt. Dies ist das höher schmelzende racemische Isomer B der Verbindung der Formel IA, worin $C_nH_{2n}$ Aethylen ist, $R_6$ Aethoxy bedeutet, $R_7$ für Hydroxy steht und $R_8$ Phenyl bedeutet.

Auftrennung mit einem optisch aktiven Amin unter üblichen Bedingungen und Trennung der diastereomeren Salze ergibt das reine Enantiomer, z. B. das 1-Carboxymethyl-3S-(1S-äthoxycarbonyl-3-phenylpropylamino)-2,3,4,5-tetrahydro-1H-[1] benzazepin-2-on des Beispiels 12.

Bei Verwendung der Hochdruck-Flüssigkeitschromatographie (weiterhin als h.p.l.c. — d. h. « high pressure liquid chromatography » bezeichnet) auf einer Säule mit umgekehrten Phasen (Lösungsmittelsystem : Methanol/Wasser (3 : 1), welches 0,025 % Essigsäure enthält), wandert das Isomer B schneller als das niedriger schmelzende Isomer A des Beispiels 5.

Der Ausgangsstoff, nämlich das 3-Amino-1-carboxymethyl-2,3,4,5-tetrahydro-1H-[1] benzazepin-2-on, wird wie folgt hergestellt :

Ein Gemisch von 2,3,4,5-Tetrahydro-1H-[1] benzazepin-2-on (48,3 g ; s. Briggs et al., J. Chem. Soc. 1937, 456), Phosphorpentachlorid (188 g) und Xylol (1 300 ml) wird in einer Stickstoffatmosphäre unter Rühren auf 90° (Oelbadtemperatur) innerhalb 30 Minuten erhitzt. Das Erwärmen wird Pausen bei 30° (man lässt das Phosphorpentachlorid sich lösen) und bei 50°, durchgeführt. Es entsteht eine ausgiebige Chlorwasserstoff-Entwicklung. Die Temperatur wird 30 Minuten bei 90° gehalten. Das Reaktionsgemisch wird heiss filtriert, um die geringe Menge eines suspendierten festen Materials zu entfernen, und das Filtrat wird unter vermindertem Druck zur Trockene eingedampft. Der Rückstand wird unter Rühren zu einer gesättigten wässerigen Natriumcarbonatlösung (100 ml) gegeben. Das Produkt wird nach dem Abschluss seiner Verfestigung abfiltriert, dann in Aethanol (150 ml) aufgeschlämmt, mit Aethanol (50 ml) und Aether (50 ml) gewaschen und getrocknet. Man erhält das 3,3-Dichlor-2,3,4,5-tetrahydro-1H-[1] benzazepin-2-on, welches bei 185-187° schmilzt.

Eine Lösung von 3,3-Dichlor-2,3,4,5-tetrahydro-1H-[1] benzazepin-2-on (20 g, 0,174 mol) und wasserfreiem Natriumacetat (15,4 g, 0,188 mol) in Eisessig (920 ml) wird bei atmosphärischem Druck, mit 5 %igem Palladium-auf-Kohle-Katalysator (1,72 g) bis zur beendigten Wasserstoffaufnahme hydriert. Der Katalysator wird abfiltriert und die Essigsäure unter vermindertem Druck abgedampft. Der Rückstand wird zwischen 10 %iger Natriumhydrogencarbonatlösung (900 ml) und Dichlormethan (300 ml) verteilt. Die wässerige Schicht (pH 8) wird mit Dichlormethan (3 × 300 ml) weiter extrahiert. Die vereinigten

Extrakte werden über wasserfreiem Natriumsulfat getrocknet und eingedampft. Man erhält das 3-Chlor-2,3,4,5-tetrahydro-1H-[1] benzazepin-2-on, welches bei 163-167° schmilzt.

Eine Lösung von 3-Chlor-2,3,4,5-tetrahydro-1H-[1] benzazepin-2-on (15,9 g, 0,08 mol) und Natriumazid (6,36 g, 0,10 mol) in Dimethylsulfoxid (320 ml) wird unter Stickstoff 3 Stunden bei 80° gehalten. In diesem Zeitpunkt zeigt eine Probe im IR-Spektrum ein starkes Maximum bei 2 150 $cm^{-1}$, welches für die Azidgruppe charakteristisch ist. Das Reaktionsgemisch wird in 1 000 ml Eiswasser gegossen und die Suspension 30 Minuten gerührt. Das feste Material wird abfiltriert, mit Wasser (250 ml) gewaschen und getrocknet. Man erhält das 3-Azido-2,3,4,5-tetrahydro-1H-[s] benzazepin-2-on, welches bei 142-145° schmilzt.

Eine Lösung von 3-Azido-2,3,4,5-tetrahydro-1H-[1] benzazepin-2-on (8,7 g, 0,043 mol) in trockenem Dimethylformamid (75 ml) wird innerhalb 30 Minuten zu einer gerührten Suspension von Natriumhydrid [hergestellt aus einer 60 %igen Dispersion in Mineralöl (1,9 g), gewaschen mit Petroläther (3 × 150 ml)] in trockenem Dimethylformamid (250 ml), bei 0°, unter Stickstoff, gegeben. Das Rühren wird weitere 1,5 Stunden fortgesetzt, dann gibt man Bromessigsäure-benzylester (10,8 g ; 0,047 mol) in trockenem Dimethylformamid (75 ml) innerhalb 45 Minuten dazu, wobei man die Temperatur bei 0° hält. Dann rührt man das Reaktionsgemisch weitere 18 Stunden, wobei es sich auf Zimmertemperatur erwärmt. Das Dimethylformamid wird unter vermindertem Druck abgedampft und der Rückstand zwischen Wasser (500 ml) und Dichlormethan (500 ml) verteilt. Die wässerige Phase wird mit Dichlormethan (3 × 500 ml) extrahiert. Die vereinigten Extrakte werden über Natriumsulfat getrocknet und das Lösungsmittel wird unter vermindertem Druck abgedampft. Man erhält das rohe Ester-azid als ein Oel. Dieses wird in Toluol (500 ml) gelöst und mit Silicagel (48 g) versetzt. Filtrierung und Abdampfen des Lösungsmittels unter vermindertem Druck ergibt das 3-Azido-1-benzyloxycarbonylmethyl-2,3,4,5-tetrahydro-1H-[1] benzazepin-2-on, als ein Oel, welches ohne weitere Reinigung im nächsten Syntheseschritt verwendet wird.

Eine Suspension von aktivem Raney-Nickel Katalysator in Wasser (15 ml) wird mit Aethanol (5 × 100 ml) gewaschen und zu einer mechanisch gerührten Lösung von 3-Azido-1-benzyloxycarbonylmethyl-2,3,4,5-tetrahydro-1H-[1] benzazepin-2-on (5,0 g) in Aethanol (300 ml) gegeben. Die Suspension wird 18 Stunden unter Stickstoff bei Zimmertemperatur gerührt. Der Katalysator wird abfiltriert und das Lösungsmittel unter vermindertem Druck abgedampft. Der Rückstand wird in 2-normaler Chlorwasserstoffsäure (200 ml) gelöst und die Lösung mit Aether (2 × 250 ml) extrahiert. Die wässerige Lösung wird mit konzentriertem wässerigem Ammoniak basisch gemacht (pH 9) und die Lösung mit Aether extrahiert (3 × 200 ml). Die vereinigten Aetherextrakte werden über Natriumsulfat getrocknet und unter vermindertem Druck eingedampft. Man erhält das 3-Amino-1-benzyloxycarbonylmethyl-2,3,4,5-tetrahydro-1H-[1] benzazepin-2-on als ein Oel, welches ohne weitere Reinigung im nächsten Syntheseschritt verwendet wird.

Das 3-Amino-1-benzyloxycarbonylmethyl-2,3,4,5-tetrahydro-1H-[1] benzazepin-2-on wird auch wie folgt hergestellt :

Eine Lösung von 3-Amino-2,3,4,5-tetrahydro-1H-[1] benzazepin-2-on (5,0 g ; 0,028 mol) in Dimethylformamid (100 ml) wird in einer Stickstoffatmosphäre zu einer gerührten Suspension von Natriumhydrid [hergestellt aus 60 %iger Dispersion in Mineralöl (1,2 g) durch Waschen mit Petroläther (3 × 150 ml)] in Dimethylformamid (400 ml), zu welchem Tetrabutylammoniumbromid (10,0 g ; 0,031 mol) gegeben worden ist. Das Reaktionsgemisch wird 15 Minuten bei 50° gehalten und dann mit einer Lösung von Bromessigsäure-benzylester (7,2 g ; 0,031 mol) ein Dimethylformamid (25 ml) versetzt. Das Reaktionsgemisch wird weitere 18 Stunden bei 50° gerührt, dann auf Zimmertemperatur gekühlt und das Dimethylformamid im Hochvakuum abgedampft. Der Rückstand wird mit Toluol/Dichlormethan (1 : 1, 500 ml) gerührt, um die anorganischen Salze auszufällen. Nach Filtrierung wird die Lösung unter vermindertem Druck eingedampft und der Rückstand auf Silicagel (200 g) chromatographiert. Man eluiert mit 0-15 % Essigsäureäthylester in Toluol. Als Hauptprodukt wird das 3-Amino-1-benzyloxycarbonylmethyl-2,3,4,5-tetrahydro-1H-[1]-benzazepin-2-on erhalten.

Eine Lösung von 3-Amino-1-benzyloxycarbonylmethyl-2,3,4,5-tetrahydro-1H-[1] benzazepin-2-on [1,3 g] in Aethanol (250 ml) wird mit 10 %igem Palladium-auf-Kohle-Katalysator (0,20 g), bis zur beendigten Wasserstoffaufnahme bei Zimmertemperatur und atmosphärischem Druck hydriert. Der Katalysator wird abfiltriert und das Lösungsmittel unter vermindertem Druck abgedampft. Man erhält einen weissen Schaum (0,90 g).

Dieser wird mit Aether trituriert, wobei man das 3-Amino-1-carboxymethyl-2,3,4,5-tetrahydro-1H-[1] benzazepin-2-on erhält. F. 147-150°.

Eine Lösung von 3-Azido-1-benzyloxycarbonylmethyl-2,3,4,5-tetrahydro-1H-[1] benzazepin-2-on (14,0 g ; 0,04 mol) in Aethanol (300 ml) wird 25 Stunden bei 3,1 Atmosphären und Zimmertemperatur unter Verwendung von 5 %igem Palladium-auf-Kohle-Katalysator (2,0 g) hydriert. Der Katalysator wird abfiltriert und das Lösungsmittel unter vermindertem Druck abgedampft. Der Rückstand wird in Wasser (500 ml) gelöst und die Lösung mit Dichlormethan (2 × 400 ml) extrahiert. Die wässerige Lösung wird filtriert und unter vermindertem Druck eingedampft. Der Rückstand wird in Aethanol (50 ml) gelöst und die Lösung unter vermindertem Druck eingedampft. Der Rückstand wird wieder in Aethanol (50 ml) aufgenommen und das Eindampfen wiederholt. Der Rückstand wird aus Aethanol/Essigsäureäthylester umkristallisiert. Man erhält das 3-Amino-1-carboxymethyl-2,3,4,5-tetrahydro-1H-[1] benzazepin-2-on, welches bei 147-150° schmilzt.

Beispiel 2

1-Benzyloxycarbonylmethyl-3-(1-carboxy-3-phenylpropylamino)-2,3,4,5-tetrahydro-1H-[1] benzazepin-2-on

Man gibt Natriumcyanborhydrid (0,152 g ; 0,001 4 mol) zu einer Lösung von 1-Benzyloxycarbonylmethyl-3-amino-2,3,4,5-tetrahydro-1H-[1] benzazepin-2-on (0,45 g, 0,001 4 mol) und Pyruvinsäurebenzylester (0,48 g ; 0,002 8 mol) in Methanol (35 ml). Das Reaktionsgemisch wird bei Zimmertemperatur in einer Stickstoffatmosphäre 2 Stunden gerührt. Das Reaktionsgemisch wird wieder mit Pyruvinsäurebenzylester (0,48 g ; 0,002 8 mol) versetzt und weitere 18 Stunden gerührt. Man gibt konz. Chlorwasserstoffsäure (0,5 ml) dazu und rührt die erhaltene Lösung 1 Stunde. Die Lösungsmittel werden unter vermindertem Druck abgedampft und der Rückstand wird mit Dichlormethan (100 ml) behandelt, um das Natriumchlorid auszufällen. Das Gemisch wird filtriert, das Lösungsmittel unter vermindertem Druck abgedampft und der Rückstand auf Silicagel (30 g) chromatographiert. Es wird mit Essigsäureäthylester/Methanol/Essigsäure (90 : 10 : 0,2) eluiert. Man erhält das 1-Benzyloxycarbonylmethyl-3-(1-carboxy-3-phenylpropylamino)-2,3,4,5-tetrahydro-1H-[1] benzazepin-2-on als ein Oel. NMR ($CDCl_3$) δ 7,35 (m, 14H), 5,10 (s, 2H), 4,60 (m, 2H), 3,00 (m, 12H).

Beispiel 3

1-Benzyloxycarbonylmethyl-3-(1-äthoxycarbonyl-3-phenylpropylamino)-2,3,4,5-tetrahydro-1H-[1] benzazepin-2-on

Eine Lösung von 1-Benzyloxycarbonylmethyl-3-(1-carboxy-3-phenylpropylamino)-2,3,4,5-tetrahydro-1H-[1] benzazepin-2-on (0,364 g ; 0,000 75 mol), Natriumhydrogencarbonat (0,190 g ; 0,002 2 mol) und Aethyljodid (0,315 g ; 0,002 mol) in Dimethylacetamid (15 ml) wird unter Stickstoff, bei Zimmertemperatur, 72 Stunden gerührt. Das Reaktionsgemisch wird filtriert und unter vermindertem Druck eingedampft. Man gibt Wasser (100 ml) dazu und extrahiert die erhaltene Lösung mit Dichlormethan (4 × 50 ml). Die vereinigten Extrakte werden über Natriumsulfat getrocknet und das Lösungsmittel wird unter vermindertem Druck eingedampft. Man erhält den Diester als ein Oel. Dieses Material wird durch h.p.l.c. in drei Fraktionen getrennt, wobei man als Lösungsmittel Essigsäureäthylester/Toluol (30 : 70) verwendet.

Die erste Fraktion ergibt das Isomer A der Titelverbindung als ein Oel. Die zweite Fraktion enthält ein Gemisch von den Isomeren A und B und die dritte Fraktion ergibt das Isomer B der Titelverbindung. Verwendet man h.p.l.c. auf einer Säule mit umgekehrten Phasen [Lösungsmittelsystem : Methanol/Wasser (3 : 1), enthaltend 0,025 % Essigsäure], wandert das Isomer A langsamer als das Isomer B.

Beispiel 4

1-Carboxymethyl-3-(1-äthoxy-carbonyl-3-phenylpropylamino)-2,3,4,5-tetrahydro-1H-[1]-benzazepin-2-on (Das höher schmelzende Isomer)

Eine Lösung von 1-Benzyloxycarbonylmethyl-3-(1-äthoxycarbonyl-3-phenylpropylamino)-2,3,4,5-tetrahydro-1H-[1] benzazepin-2-on (das Isomer B des Beispiels 3 ; 0,9 g) in Aethanol (150 ml) wird unter Verwendung von 10 %-igem Palladium-auf-Kohle-Katalysator (0,5 g), bei Zimmertemperatur und atmosphärischem Druck hydriert. Nach beendigter Wasserstoffaufnahme wird der Katalysator abfiltriert und das Lösungsmittel unter vermindertem Druck abgedampft. Man erhält ein festes Material, welches man mit Aether (8 ml) trituriert. Man erhält die Titelverbindung, welche bei 138-140° schmilzt und mit dem Produkt des Beispiels 1 identisch ist.

Beispiel 5

1-Carboxymethyl-3-(1-äthoxycarbonyl-3-phenylpropylamino)-2,3,4,5,-tetrahydro-1H-[1] benzazepin-2-on (Das niedriger schmelzende Isomer)

Eine Lösung von 1-Benzyloxycarbonylmethyl-3-(1-äthoxycarbonyl-3-phenyl-propylamino)-2,3,4,5-tetrahydro-1H-[1] benzazepin-2-on (das Isomer A des Beispiels 3 ; 1,2 g) in Aethanol (125 ml) wird mit 10 %igem Palladium-auf-Kohle-Katalysator (0,5 g), bei Zimmertemperatur und atmosphärischem Druck hydriert. Nach beendeter Wasserstoffaufnahme wird der Katalysator abfiltriert und das Lösungsmittel unter vermindertem Druck abgedampft. Man erhält ein festes Material, welches man mit Aether (8 ml) trituriert. Man erhält das 1-Carboxymethyl-3-(1-äthoxycarbonyl-3-phenylpropylamino)-2,3,4,5-tetrahydro-1H-[1] benzazepin-2-on, welches bei 126-129° schmilzt und das niedriger schmelzende racemische Isomer A ist.

Verwendet man h.p.l.c. auf einer Säule mit umgekehrten Phasen [Lösungsmittelsystem : Methanol/Wasser (3 : 1), welches 0,025 % Essigsäure enthält], wandert das Isomer A langsamer als das höher schmelzende racemische Isomer B des Beispiels 1.

Beispiel 6

1-Benzyloxycarbonylmethyl-3-(1-äthoxycarbonyl-3-phenylpropylamino)-2,3,4,5-tetrahydro-1H-[1] benzazepin-2-on

Eine Lösung von 3-(1-Aethoxycarbonyl-3-phenylpropylamino)-2,3,4,5-tetrahydro-1H-[1] benzazepin-2-on (5,0 g) in trockenem Dimethylformamid (20 ml) wird in einer Stickstoffatmosphäre zu einer gerührten Suspension von Natriumhydrid [hergestellt aus einer 60 %igen Dispersion in Mineralöl (0,6 g), welches mit Petroläther gewaschen wird (3 × 75 ml)] in trockenem Dimethylformamid (85 ml), welches Tetrabutylammoniumbromid (4,4 g) enthält, gegeben. Das Reaktionsgemisch wird 30 Minuten bei Zimmertemperatur gerührt und dann mit einer Lösung von Bromessigsäure-benzylester (3,2 g) in trockenem Dimethylformamid (10 ml) versetzt. Das Gemisch wird weitere 30 Minuten bei Zimmertemperatur gerührt, auf 60° erhitzt und bei dieser Temperatur 18 Stunden gehalten. Das Reaktionsgemisch wird auf Zimmertemperatur gekühlt und das Lösungsmittel im Hochvakuum abgedampft. Man gibt Wasser dazu (150 ml) und extrahiert die erhaltene Lösung mit Essigsäureäthylester (2 × 250 ml). Die vereinigten Essigsäureäthylester-Extrakte werden mit Wasser gewaschen (100 ml), über Magnesiumsulfat getrocknet und unter vermindertem Druck abgedampft. Man erhält ein braunes Oel, welches auf Silicagel (150 g) chromatographiert und mit Toluol/Essigsäureäthylester (3 : 1) eluiert wird. Man erhält zuerst das Isomer A des 1-Benzyloxy-carbonylmethyl-3-(1-äthoxycarbonyl-3-phenylpropylamino)-2,3,4,5-tetrahydro-1H-[1] benzazepin-2-ons. Es folgt dann das Isomer B. Die Isomere A und B sind identisch mit den Verbindungen des Beispiels 3, wie man dies durch h.p.l.c. auf einer Säule mit umgekehrten Phasen [Lösungsmittelsystem : Methanol/Wasser (3 : 1), enthaltend 0,025 % Essigsäure] bestimmt hat.

Der Ausgangsstoff wird wie folgt hergestellt. Eine Lösung von Acetamido-malonsäure-diäthylester (33,2 g) in Aethanol (150 ml) wird zu einer Lösung von Natrium-äthoxid in Aethanol [hergestellt aus Natrium (3,8 g) und Aethanol (200 ml)] gegeben. Das Reaktionsgemisch wird bei Zimmertemperatur 30 Minuten gerührt und innerhalb 20 Minuten mit einer Lösung von 2-Nitro-phenyläthyl-bromid [J. Med. Chem. *20,* 1020 (1977) ; 40,0 g] in Aethanol (100 ml) tropfenweise versetzt. Nach der beendeten Zugabe wird das Reaktionsgemisch 18 Stunden unter Rückfluss gekocht, dann auf Zimmertemperatur gekühlt und unter vermindertem Druck eingedampft. Der Rückstand wird in Wasser (350 ml) gelöst und die Lösung mit Essigsäureäthylester (2 × 350 ml) extrahiert. Die vereinigten Essigsäureäthylester-Extrakte werden mit Wasser (200 ml) gewaschen und über Magnesiumsulfat getrocknet. Das Lösungsmittel wird unter vermindertem Druck abgedampft. Man erhält den 2-Acetamido-2-(o-nitro-phenyl-äthyl)-malonsäure-diäthylester als ein niedrig schmelzendes festes Material, welches ohne weitere Reinigung im nächsten Syntheseschritt verwendet wird.

Eine Lösung von 2-Acetamido-2-(o-nitro-phenyläthyl)-malonsäure-diäthyl-ester (80 g) in 3-normaler Chlorwasserstoffsäure (900 ml) wird 12 Stunden unter Rückfluss gekocht. Die Lösung wird abgekühlt und mit Essigsäureäthylester (200 ml) extrahiert. Die wässerige Lösung wird filtriert und unter vermindertem Druck zur Trockene eingedampft. Der Rückstand wird aus Aethanol/Aether umkristallisiert. Man erhält das 2-Amino-4-(2-nitrophenyl)-buttersäure-hydrochlorid, welches bei 219-221° (Zersetzung) schmilzt.

Eine Lösung von 2-Amino-4-(2-nitrophenyl)-buttersäure-hydrochlorid (38 g) in 10 %iger äthanolischer Chlorwasserstoffsäure (1 200 ml) wird 18 Stunden unter Rückfluss gekocht. Das Reaktionsgemisch wird unter vermindertem Druck eingedampft, mit Wasser (250 ml) versetzt und die wässerige Lösung mit 2-normaler Natriumhydroxidlösung basisch gemacht.

Die Lösung wird mit Dichlormethan (2 × 500 ml) extrahiert. Die vereinigten Dichlormethan-Lösungen werden mit Wasser (2 × 150 ml) gewaschen und über wasserfreiem Natriumsulfat getrocknet. Nach Eindampfen erhält man den 2-Amino-4-(2-nitrophenyl)-buttersäure-äthylester, der ohne weitere Reinigung im nächsten Syntheseschritt verwendet wird.

Eine Lösung von 2-Amino-4-(2-nitrophenyl)-buttersäure-äthylester (27 g) in Aethanol (600 ml) wird unter Verwendung von 10 %igem Palladium-auf-Kohle-Katalysator (2,5 g), bei Zimmertemperatur und atmosphärischem Druck, bis zur beendigten Wasserstoffaufnahme hydriert. Der Katalysator wird abfiltriert und die Lösung zur Trockene eingedampft. Man erhält den 2-Amino-4-(2-aminophenyl)-buttersäure-äthyl-ester, der im nächsten Syntheseschritt ohne weitere Reinigung verwendet wird.

Eine Lösung von 2-Amino-4-(2-aminophenyl)-buttersäure-äthylester (35,0 g) in Methanol (100 ml) wird zu einer Lösung von Natrium-methoxid in Methanol [hergestellt aus Natrium (1,0 g) und Methanol (400 ml)], unter Rühren in einer Stickstoffatmosphäre, gegeben. Das Reaktionsgemisch wird 65 Stunden unter Rückfluss gekocht und unter vermindertem Druck eingedampft. Der Rückstand wird zwischen Wasser (100 ml) und Dichlormethan (400 ml) verteilt. Die wässerige Lösung wird mit Dichlormethan (400 ml) extrahiert. Die vereinigten organischen Lösungen werden mit Wasser (100 ml) gewaschen und über Magnesiumsulfat getrocknet. Die Lösung wird zur Trockene eingedampft und der Rückstand mit Aether (250 ml) trituriert. Man erhält das 3-Amino-2,3,4,5-tetrahydro-1H-[1] benzazepin-2-on, welches bei 161-162° schmilzt.

Im anderen Falle kann man wie folgt vorgehen : Eine Lösung von 2-Amino-4-(2-nitrophenyl)-buttersäure-hydrochlorid (2,5 g) in Wasser (200 ml) wird, unter Verwendung von 10 %igem Palladium-auf-Kohle-Katalysator, bei Zimmertemperatur und atmosphärischem Druck hydriert. Nach beendigter Wasserstoffaufnahme wird der Katalysator abfiltriert und das Filtrat zur Trockene eingedampft. Der

Rückstand wird in Wasser (50 ml) gelöst und der pH-Wert mit 10 %iger Natriumhydroxidlösung auf 7 eingestellt. Der Niederschlag wird abfiltriert, mit Wasser gewaschen und getrocknet. Man erhält die 2-Amino-4-(2-aminophenyl)-buttersäure. Eine Lösung der 2-Amino-4-(2-aminophenyl)-buttersäure (1,0 g), Hexamethyl-disilazan (5,4 g) und Chlortrimethylsilan (0,1 g) in Xylol (125 ml) wird 65 Stunden unter Rückfluss gekocht. Das Reaktionsgemisch wird gekühlt, in Aethanol (200 ml) gegossen und unter vermindertem Druck eingedampft. Man gibt Wasser (100 ml) dazu und extrahiert die Lösung mit Dichlormethan (2 × 125 ml). Die vereinigten Dichlormethan-Lösungen werden mit Wasser (50 ml) gewaschen, über Magnesiumsulfat getrocknet und unter vermindertem Druck eingedampft. Man erhält das 3-Amino-2,3,4,5-tetrahydro-1H-[1] benzazepin-2-on, welches mit dem obigen identisch ist.

Das 3-Amino-2,3,4,5-tetrahydro-1H-[1] benzazepin-2-on wird auch wie folgt hergestellt :

Eine Lösung von 3-Azido-2,3,4,5-tetrahydro-1H-[1] benzazepin-2-on (s. Beispiel 1) (27 g) in Aethanol (3 500 ml) wird in einer Stickstoffatmosphäre, unter Rühren bei Zimmertemperatur, mit einer Suspension von Raney-Nickel in Wasser (50 ml, gewaschen mit 10 Volumina Aethanol) versetzt. Das Gemisch wird bei Zimmertemperatur 2 Stunden gerührt und dann mit weiteren 30 ml Raney-Nickel Suspension versetzt. Das Gemisch wird weitere 30 Minuten gerührt, der Katalysator abfiltriert und das Lösungsmittel unter vermindertem Druck abgedampft. Man erhält ein Oel, welches sich nach Zugabe von Aether verfestigt. Man erhält das 3-Amino-2,3,4,5-tetrahydro-1H-[1] benzazepin-2-on, welches bei 161-162° schmilzt.

Eine Lösung von 3-Amino-2,3,4,5-tetrahydro-1H-[1] benzazepin-2-on (8,0 g) und Benzylpyruvinsäure (18,0 g) in Methanol (450 ml) wird unter Stickstoff bei Zimmertemperatur 30 Minuten gerührt. Man gibt Natrium-cyanborhydrid (4,5 g) dazu und rührt die erhaltene Lösung 48 Stunden bei Zimmertemperatur. Das Gemisch wird innerhalb 10 Minuten mit konz. Chlorwasserstoffsäure (7 ml) tropfenweise versetzt und 1 weitere Stunde gerührt. Das Reaktionsgemisch wird zur Trockene eingedampft, mit Dichlormethan (150 ml) versetzt und das Gemisch 30 Minuten gerührt. Das feste Material wird abfiltriert, mit Wasser (100 ml) 15 Minuten gerührt, dann filtriert, mit Wasser (50 ml) gewaschen und getrocknet. Man erhält das 3-(1-Carboxy-3-phenylpropylamino)-2,3,4,5-tetrahydro-1H-[1] benzazepin-2-on, als ein Gemisch von Isomeren. F 173-175°.

Eine Lösung von 3-(1-Carboxy-3-phenylpropylamino)-2,3,4,5-tetrahydro-1H-[1] benzazepin-2-on (6,0 g), Natriumhydrogencarbonat (4,0 g) und Aethyljodid (11,6 g) in Dimethylacetamid (200 ml) wird 72 Stunden unter Stickstoff bei Zimmertemperatur gerührt, filtriert und im Hochvakuum eingedampft. Der Rückstand wird in Wasser (250 ml) aufgenommen und die erhaltene Lösung mit Dichlormethan (2 × 400 ml) extrahiert. Die vereinigten Extrakte werden über Magnesiumsulfat getrocknet und das Lösungsmittel unter vermindertem Druck abgedampft. Man erhält das 3-(1-Aethoxycarbonyl-3-phenylpropylamino)-2,3,4,5-tetrahydro-1H-[1]-benzazepin-2-on als ein Isomerengemisch. NMR ($CDCl_3$) δ 9,22 (s, 1H), 4,10 (2 überlagerte Quartetts, 2H), 1,13 (2 überlagerte Tripletts, 3H).

## Beispiel 7

1-Benzyloxycarbonylmethyl-3-(1-benzyloxycarbonyl-3-phenylpropylamino)-2,3,4,5-tetrahydro-1H-[1] benzazepin-2-on

Eine Lösung von 3-(1-Benzyloxycarbonyl-3-phenylpropylamino)-2,3,4,5-tetrahydro-1H-[1] benzazepin-2-on (4,0 g) in trockenem Dimethylformamid wird unter Stickstoff zu einer gerührten Suspension von Natriumhydrid [aus 60 %iger Dispersion in Mineralöl (0,42 g), gewaschen mit Petroläther (3 × 80 ml)] in trockenem Dimethylformamid (100 ml), zu welchen Tetrabutylammonium-bromid (3,1 g) gemischt ist, bei Zimmertemperatur gegeben. Das Gemisch wird weitere 30 Minuten bei Zimmertemperatur fortgesetzt und dann mit einer Lösung von Bromessigsäure-benzyl-ester (2,2 g) in trockenem Dimethylformamid (10 ml) versetzt. Nach weiteren 30 Minuten bei Zimmertemperatur wird das Reaktionsgemisch auf 50° erhitzt und bei dieser Temperatur 18 Stunden gehalten. Das Gemisch wird auf Zimmertemperatur gekühlt und das Lösungsmittel im Hochvakuum abgedampft. Der Rückstand wird in Wasser (150 ml) aufgenommen und die Lösung mit Essigsäureäthylester (2 × 300 ml) extrahiert. Die vereinigten Essigsäureäthylester-Lösungen werden mit Wasser (100 ml) gewaschen, über Magnesiumsulfat getrocknet und unter vermindertem Druck eingedampft. Man erhält ein braunes Oel, welches auf Silicagel (250 g) chromatographiert wird. Man eluiert mit Toluol/Essigsäureäthylester (1 : 1 ; 600 ml) und erhält ein Oel, welches das Isomer A der Titelverbindung ist. NMR ($CDCl_3$) δ 5,12 (s, 4H), 4,50 (q, 2H). Die Eluierung mit weiteren 2 000 ml des Lösungsmittelgemisches ergibt ein Oel, welches das Isomer B der Titelverbindung ist. NMR ($CDCl_3$) δ 5,17 (s, 2H), 5,03 (d, 2H), 4,60 (q, 2H).

Der Ausgangsstoff wird wie folgt hergestellt : Eine Lösung von 3-(1-Carboxy-3-phenylpropylamino)-2,3,4,5-tetrahydro-1H-[1] benzazepin-2-on (wie im Beispiel 6 beschrieben ; 13,0 g), Natriumhydrogencarbonat (10,0 g) und Benzylbromid (19,0 g) in Dimethylacetamid (750 ml) wird bei Zimmertemperatur, unter Stickstoff, 7,2 Stunden gerührt. Das Reaktionsgemisch wird filtriert und im Hochvakuum eingedampft. Der Rückstand wird in Wasser (150 ml) aufgenommen und die erhaltene Lösung mit Dichlormethan (2 × 400 ml) extrahiert. Die vereinigten Extrakte werden mit Wasser (100 ml) gewaschen, über Magnesiumsulfat getrocknet und unter vermindertem Druck eingedampft. Man erhält den rohen Benzylester. Umkristallisation aus Essigsäureäthylester ergibt das 3-(1-Benzyloxycarbonyl-3-phenylpropylamino)-2,3,4,5-tetrahydro-1H-[1] benzazepin-2-on, welches bei 139-141° schmilzt.

Beispiel 8

1-Carboxymethyl-3-(1-carboxy-3-phenylpropylamino)-2,3,4,5-tetrahydro-1H-[1] benzazepin-2-on (Das niedriger schmelzende Isomer)

Eine Lösung von 1-Benzyloxycarbonylmethyl-3-(1-benzyloxycarbonyl-3-phenylpropylamino)-2,3,4,5-tetrahydro-1H-[1] benzazepin-2-on (das Isomer A des Beispiels 7 ; 2,7 g) in Aethanol (800 ml) wird mit 10 %igem Palladium-auf-Kohle-Katalysator (0,5 g) bei Zimmertemperatur und atmosphärischem Druck hydriert. Nach dem Abschluss der Wasserstoffaufnahme wird der Katalysator abfiltriert und das Lösungsmittel unter vermindertem Druck abgedampft. Man erhält die Di-säure (Titelverbindung), welche das Isomer A ist. F. 256-259°.

Die identische Verbindung wird durch Hydrolyse der Verbindung des Beispiels 5 erhalten.

Beispiel 9

1-Carboxymethyl-3-(1-carboxy-3-phenylpropylamino)-2,3,4,5-tetrahydro-1H-[1] benzazepin-2-on (Das höher schmelzende Isomer)

Eine Lösung von 1-Benzyloxycarbonylmethyl-3-(1-benzyloxycarbonyl-3-phenylpropylamino)-2,3,4,5-tetrahydro-1H-[1] benzazepin-2-on (das Isomer B des Beispiels 7 ; 5,0 g) in Aethanol (950 ml) wird mit Palladium-auf-Kohle-Katalysator (0,5 g) bei Zimmertemperatur und atmosphärischem Druck hydriert. Nach Abschluss der Wasserstoffaufnahme wird der Katalysator abfiltriert und das Lösungsmittel unter vermindertem Druck eingedampft. Man erhält die oben genannte Di-säure (Titelverbindung), welche das Isomer B ist. F. 280-282°.

Die identische Verbindung wird durch Hydrolyse der Verbindung des Beispiels 1 (Isomer B) oder der Verbindung des Beispiels 10 (Isomer B) erhalten.

Beispiel 10

1-Aethoxycarbonylmethyl-3-(1-äthoxycarbonyl-3-phenylpropylamino)-2,3,4,5-tetrahydro-1H-[1] benzazepin-2-on

Eine Lösung von 3-(1-Aethoxycarbonyl-3-phenylpropylamino)-2,3,4,5-tetrahydro-1H-[1] benzazepin-2-on (s. Beispiel 6 ; 3,0 g) in trockenem Dimethylformamid (10 ml) wird tropfenweise, innerhalb 10 Minuten, zu einer gerührten Suspension von Natriumhydrid [aus einer 60 %igen Suspension in Mineralöl (0,36 g), gewaschen mit Petroläther (3 × 75 ml)] in trockenem Dimethylformamid (100 ml), bei Zimmertemperatur in einer Stickstoffatmosphäre, gegeben. Das Gemisch wird 30 Minuten weiter gerührt, mit einer Lösung von Bromessigsäure-äthylester (1,4 g) in Dimethylformamid (15 ml) versetzt und 48 Stunden bei 60° gehalten. Das Reaktionsgemisch wird auf Zimmertemperatur gekühlt und das Lösungsmittel im Hochvakuum abgedampft. Der Rückstand wird mit Wasser (100 ml) versetzt und die Lösung mit Essigsäureäthylester (2 × 200 ml) extrahiert. Die vereinigten Extrakte werden mit Wasser (50 ml) gewaschen, über Magnesiumsulfat getrocknet und das Lösungsmittel unter vermindertem Druck abgedampft. Man erhält ein gelbes Oel (3,8 g). Dieses wird auf Silicagel (120 g) chromatographiert und mit Toluol/Essigsäureäthylester (1 : 1 ; 250 ml) eluiert. Man erhält das Isomer A des gewünschten Produkts. Eluierung mit weiteren 250 ml des Lösungsmittelgemisches ergibt ein Oel. Dieses besteht hauptsächlich aus dem Isomer B und einer geringen Menge des Isomers A des gewünschten Produkts, wie dies durch analytische h.p.l.c. (s. Beispiel 6) bestimmt wird. Eluierung mit weiteren 250 ml des Lösungsmittelgemisches ergibt ein Oel, das im wesentlichen aus dem reinen Isomer B (das langsamer wandert) besteht. Dieses Material wird in Methanol (25 ml) gelöst und durch Zugabe der äquivalenten Menge Maleinsäure in Methanol in das Maleatsalz umgewandelt. Nach Eindampfen des Lösungsmittels und Umkristallisation des Rückstandes aus Methanol/Aether erhält man das reine Isomer B des 1-Aethoxycarbonylmethyl-3-(1-äthoxycarbonyl-3-phenylpropylamino)-2,3,4,5-tetrahydro-1H-[1] benzazepin-2-ons als Maleatsalz, welches bei 114-116° schmilzt.

Beispiel 11

1-Carboxymethyl-3-carboxymethylamino-2,3,4,5-tetrahydro-1H-[1] benzazepin-2-on

Eine Lösung von 1-Benzyloxycarbonylmethyl-3-benzyloxycarbonylmethylamino-2,3,4,5-tetrahydro-1H-[1] benzazepin-2-on (4,8 g ; 0,01 mol) in Aethanol (550 ml) wird bei Zimmertemperatur und atmosphärischem Druck, mit 5 %igem Palladium-auf-Kohle-Katalysator (0,85 g) bis zum Abschluss der Wasserstoffaufnahme hydriert. Das Gemisch wird mit Wasser (300 ml) versetzt, der Katalysator abfiltriert und das Lösungsmittel unter vermindertem Druck abgedampft. Der Rückstand wird mit Aether trituriert. Man erhält die Titelverbindung (Di-säure), welche bei 232-236° schmilzt.

Der Ausgangsstoff wird wie folgt hergestellt : Eine Lösung von 3-Amino-2,3,4,5-tetrahydro-1H-[1]

benzazepin-2-on (5,0 g ; 0,028 mol) in Dimethylformamid (100 ml) wird unter Stickstoff zu einer gerührten Suspension von Natriumhydrid [hergestellt aus einer 60 %igen Suspension in Mineralöl (1,2 g) durch Waschen mit Petroläther (3 × 150 ml)] in Dimethylformamid (400 ml), zu welchem man Tetrabutylammonium-bromid (10,9 g ; 0,031 mol) gemischt hat, gegeben. Das Reaktionsgemisch wird 15 Minuten bei 50° gehalten und dann mit einer Lösung von Bromessigsäurebenzylester (7,2 g ; 0,031 mol) in Dimethylformamid (25 ml) versetzt. Das Gemisch wird weitere 18 Stunden bei 50° gerührt, dann auf Zimmertemperatur gekühlt und das Dimethylformamid im Hochvakuum abgedampft. Der Rückstand wird mit Toluol/Dichlormethan (1 : 1 ; 500 ml) gerührt, um die anorganischen Salze auszufällen. Nach Filtrierung wird die Lösung unter vermindertem Druck eingedampft. Der Rückstand wird auf Silicagel (200 ml) chromatographiert und mit 0-15 % Essigsäure-äthylester in Toluol eluiert. Man erhält das 1-Benzyloxycarbonylmethyl-3-benzyloxycarbonylmethylamino-2,3,4,5-tetrahydro-1H-[1] benzazepin-2-on als die erste Fraktion. Die weitere Eluierung ergibt das 3-Benzyloxy-carbonylmethylamino-2,3,4,5-tetrahydro-1H-[1] benzazepin-2-on, F. 124-127° und das 3-Amino-1-benzyloxycarbonylmethyl-2,3,4,5-tetrahydro-1H-[1] benzazepin-2-on (s. Beispiel 1).

## Beispiel 12

1-Carboxymethyl-3S-(1S-äthoxycarbonyl-3-phenylpropylamino)-2,3,4,5-tetrahydro-1H-[1] benzazepin-2-on

3(S)-Amino-1-carboxymethyl-2,3,4,5-tetrahydro-1H-[1] benzazepin-2-on ergibt nach Behandlung mit Benzylpyruvinsäure-äthylester in Gegenwart von Natriumcyanborhydrid (wie dies im Beispiel 1 für die racemische Verbindung beschrieben ist) und Reinigung, das 1-Carboxymethyl-3S-(1S-äthoxycarbonyl-3-phenylpropylamino)-2,3,4,5-tetrahydro-1H-[1]-benzazepin-2-on wie folgt :

Eine Lösung von Natriumhydroxid (2,1 g) in Wasser (5 ml) wird zu einer Lösung von 3(S)-Amino-1-äthoxycarbonylmethyl-2,3,4,5-tetrahydro-1H-[1] benzazepin-2-on (14,0 g) in Methanol (150 ml) bei Zimmertemperatur gegeben und die Lösung wird 2 Stunden gerührt. Die Lösungsmittel werden abgedampft, der Rückstand sorgfältig getrocknet und mit Aether aufgeschlämmt. Man erhält das 3(S)-Amino-1-carboxymethyl-2,3,4,5-tetrahydro-1H-[1] benzazepin-2-on-natriumsalz, welches ohne weitere Reinigung verwendet wird.

Eine Lösung des obigen Natriumsalzes (12,9 g) und Benzylpyruvinsäureäthylester (31 g) in Essigsäure (100 ml) und Methanol (75 ml) wird in einer trockenen Stickstoffatmosphäre bei Zimmertemperatur, eine Stunde gerührt. Eine Lösung von Natriumcyanborhydrid (3,8 g) in Methanol (30 ml) wird dann innerhalb 4 Stunden, tropfenweise zugegeben. Die vereinigten Lösungen werden über Nacht bei Zimmertemperatur gerührt und mit konz. Chlorwasserstoffsäure (10 ml) tropfenweise versetzt.

Das Gemisch wird bei Zimmertemperatur eine Stunde gerührt und eingedampft. Der Rückstand wird zwischen Wasser (400 ml) und Aether (100 ml) verteilt und der pH-Wert mit 40 %iger Natriumhydroxidlösung auf 9,3 eingestellt. Die Schichten werden getrennt und die Aetherschicht wird verworfen. Die wässerige Schicht wird mit konz. Chlorwasserstoffsäure auf den pH-Wert, 4,3 eingestellt und mit Essigsäureäthylester (3 × 100 ml) extrahiert. Die organischen Phasen werden vereinigt, über Magnesiumsulfat getrocknet und eingedampft. Das rohe Produkt wird in Methylenchlorid (150 ml) gelöst und Chlorwasserstoffgas 5 Minuten durch die Lösung geleitet. Das Lösungsmittel wird eingedampft und der erhaltene Schaum in heissem Methyläthylketon (100 ml) gelöst. Der ausgefällte Niederschlag wird abfiltriert. Man erhält ein 95 : 5 diastereomeres Gemisch, wie dies durch h.p.l.c. bestimmt wird. Das Produkt wird aus 3-Pentanon/Methanol (10 : 1) umkristallisiert. Man erhält das 1-Carboxymethyl-3(S)-(1(S)-äthoxycarbonyl-3-phenylpropylamino)-2,3,4,5-tetrahydro-1H-[1] benzazepin-2-on-hydrochlorid, F. 188-190°, $[\alpha]_D = -141,0°$ (c = 0,9 in Aethanol), der Formel IB, worin $C_nH_{2n}$ Aethylen bedeutet, $R_6$ Aethoxy ist, $R_7$ für Hydroxy steht und $R_8$ Phenyl bedeutet.

Eine Lösung des vorhergenannten Hydrochlorids (0,035 g) und Propylenoxid (0,5 ml) in Aethanol (4 ml) wird unter Stickstoff bei Zimmertemperatur über Nacht gerührt. Die Lösung wird zur Trockene eingedampft, mit Aether (2 ml) versetzt und der Niederschlag abfiltriert. Man erhält das 1-Carboxymethyl-3S-(1S-äthoxycarbonyl-3-phenylpropylamino)-2,3,4,5-tetrahydro-1H-[1] benzazepin-2-on, welches bei 148-149° schmilzt. $[\delta]_D = -159°$ (c = 1,2 in Aethanol).

Das optisch aktive Ausgangsmaterial wird wie folgt hergestellt :

a) Eine Lösung von 0,4 g 3(S)-t-Butyloxycarbonylamino-2,3,4,5-tetrahydro-1H-[1] benzazepin-2,5-dion [hergestellt aus L-Kynurenin gemäss Australian J. Chemistry, Vol. 33, 633-640 (1980)] und Bromessigsäure-äthylester (0,23 g) in trockenem Tetrahydrofuran (30 ml) wird bei 0° in einer trockenen Stickstoffatmosphäre gerührt. Das Gemisch wird mit Kalium-t-butoxid (0,254 g) in einer Portion versetzt, 1 Stunde bei 0° stehen gelassen, dann mit einer weiteren Menge Bromessigsäure-äthyl-ester (0,23 g) versetzt und bei 0° eine weitere Stunde gerührt. Das Reaktionsgemisch wird mit Wasser (100 ml) versetzt und mit Essigsäure-äthylester (2 × 50 ml) extrahiert. Die vereinigten Extrakte werden mit Wasser (100 ml) gewaschen, über Magnesiumsulfat getrocknet und unter vermindertem Druck eingedampft. Man erhält ein gelbes gummiartiges Material, welches nach Zerreibung mit Aether-Petroläther (Siedepunkt 30-60°), das 3(S)-t-Butyloxycarbonylamino-1-äthoxycarbonyl-methyl-2,3,4,5-tetrahydro-1H-[1] benzazepin-2,5-dion, F. 86-88° ergibt. $[\alpha]_D = -203°$ (c = 1 in Dimethylformamid).

Eine Lösung des 3(S)-t-Butyloxycarbonylamino-1-äthoxycarbonylmethyl-2,3,4,5-tetrahydro-1H-[1] benzoazepin-2,5-dion (0,14 g) und Natriumborhydrid (7 mg) in Aethanol (10 ml) wird 18 Stunden bei Zimmertemperatur gerührt. Das Aethanol wird unter vermindertem Druck abgedampft und der Rückstand in Dichlormethan (25 ml) gelöst. Die Lösung wird mit 2 normaler Chlorwasserstoffsäure (2 × 20 ml) und mit gesättigter wässeriger Natriumchloridlösung (20 ml) extrahiert und über Natriumsulfat getrocknet. Das Lösungsmittel wird unter vermindertem Druck abgedampft und der Rückstand mit Aether trituriert. Man erhält das 3(S)-t-Butyloxycarbonylamino-1-äthoxycarbonylmethyl-5-hydroxy-2,3,4,5-tetrahydro-1H-[1] benzazepin-2-on, welches bei 167-169,5° schmilzt. $[\alpha]_D = -193°$ (c = 0,52 in Dimethylformamid). Diese Verbindung wird auch durch Hydrierung des Benzazepin-2,5-dion-Derivates mit $H_2$/Pt in Aethanol erhalten.

Ein Gemisch von 3(S)-t-Butyloxycarbonylamino-1-äthoxycarbonylmethyl-5-hydroxy-2,3,4,5-tetrahydro-1H-[1] benzazepin-2-on (0,076 g), Dicyclohexylcarbodiimid (0,064 g) und Kupfer-I-chlorid (7 mg) wird in einer Stickstoffatmosphäre bei 60°, 32 Stunden erhitzt. Man lässt das Reaktionsgemisch sich auf Zimmertemperatur abkühlen. Der Rückstand wird in Methylenchlorid (50 ml) gelöst und zuerst mit verdünnten Ammonium-hydroxid (2 × 15 ml) und dann mit Wasser (20 ml) gewaschen. Die organische Phase wird über Natriumsulfat getrocknet und eingedampft. Man erhält ein Gemisch des gewünschten Addukts und vom überschüssigen Dicyclohexylcarbodiimid.

Das letztgenannte Gemisch (0,100 g) wird in Essigsäureäthylester (40 ml) gelöst und in ein Druckgefäss gestellt. Man gibt 10 %igen Palladium-auf-Kohle-Katalysator (0,010 g) dazu und hydriert das Gemisch 16 Stunden bei 40° und 3 Atmosphären. Der Katalysator wird abfiltriert und das Filtrat eingedampft. Der Rückstand wird mit Aether trituriert und die Aetherlösung eingedampft. Man erhält das 3(S)-t-Butyloxycarbonylamino-1-äthoxycarbonylmethyl-2,3,4,5-tetrahydro-1H-[1] benzazepin-2-on, F. 115-116,5°. $[\alpha]_D = -182°$ (c = 2,6 in Dimethylformamid).

b) Weinsäure (12,6 g) und racemisches 3-Amino-1-äthoxycarbonylmethyl-2,3,4,5-tetrahydro-1H-[1] benzazepin-2-on (22 g) werden in heissem Aethanol (200 ml) gelöst. Diese Lösung wird gekühlt und über Nacht bei Zimmertemperatur stehen gelassen. Der erhaltene Niederschlag wird abfiltriert und zweimal aus Aethanol (200 ml) umkristallisiert. Man erhält das 3(S)-Amino-1-äthoxycarbonylmethyl-2,3,4,5-tetrahydro-1H-[1]-benzazepin-2-on-tartratsalz. Dieses wird in Wasser (100 ml) gelöst, der pH-Wert mit verdünnten Ammoniumhydroxid auf 9 eingestellt und mit Methylenchlorid (2 × 50 ml) extrahiert. Die vereinigten Extrakte werden mit Wasser (75 ml) gewaschen, über Magnesiumsulfat getrocknet und eingedampft. Man erhält das 3(S)-Amino-1-äthoxycarbonylmethyl-2,3,4,5-tetrahydro-1H-[1] benzazepin-2-on. F. 104-106°. $[\alpha]_D = - 285{,}5°$ (c = 0,99 in Aethanol).

c) Durch eine Lösung von 3(S)-t-Butyloxycarbonylamino-1-äthoxycarbonyl-methyl-2,3,4,5-tetrahydro-1H-[1] benzazepin-2-on [oben unter a); 0,225 g] in Essigsäureäthylester (25 ml) wird zuerst 45 Minuten Chlorwasserstoffgas und dass 30 Minuten Stickstoff durchgeleitet. Der Essigsäureäthylester wird mit Wasser (30 ml) und 1-normaler Chlorwasserstoffsäure (30 ml) gewaschen. Die Essigsäureäthylesterschicht wird verworfen und die wässerigen Phasen werden vereinigt. Die wässerige Lösung wird mit verdünnten Ammoniumhydroxid auf den pH-Wert 9 eingestellt, und mit Essigsäureäthylester (3 × 50 ml) extrahiert. Die organischen Phasen werden vereinigt, über Natriumsulfat getrocknet und eingedampft. Man erhält das 3(S)-Amino-1-äthoxycarbonylmethyl-2,3,4,5-tetrahydro-1H-[1] benzazepin-2-on, F. 101-102°. $[\alpha]_D = - 298°$ (c = 0,46 in Aethanol).

Durch Behandlung mit Aethan-dithiol/Bortrifluorid-ätherat oder mit Trifluoressigsäure/Anisol wird die Schutzgruppe abgespalten. Man erhält das 3(S)-Amino-1-äthoxycarbonylmethyl-2,3,4,5-tetrahydro-1H-[1] benzazepin-2-on.

Das 3(S)-Amino-1-äthoxycarbonylmethyl-2,3,4,5-tetrahydro-1H-[1] benzazepin-2-on wird auch wie folgt hergestellt:

d) Eine Lösung von 3(S)-t-Butyloxycarbonylamino-1-äthoxycarbonyl-methyl-5-hydroxy-2,3,4,5-tetrahydro-1H-[1] benzazepin-2-on (vorher beschrieben; 1,0 g) in Essigsäureanhydrid (20 ml) wird 3 Stunden bei 80° gehalten. Das Reaktionsgemisch wird auf Zimmertemperatur gekühlt und unter vermindertem Druck eingedampft. Der Rückstand wird mit Aether (100 ml) versetzt, die erhaltene Lösung mit Wasser (500 ml) gewaschen und über Magnesiumsulfat getrocknet. Das Lösungsmittel wird unter vermindertem Druck abgedampft. Man erhält das 5-Acetoxy-3(S)-t-Butyloxycarbonylamino-1-äthoxycarbonylmethyl-2,3,4,5-tetrahydro-1H-[1] benzazepin-2-on als bleiches Oel, welches ohne weitere Reinigung verwendet wird.

Eine Lösung von 5-Acetoxy-3(S)-t-butyloxycarbonylamino-1-äthoxycarbonyl-methyl-2,3,4,5-tetrahydro-1H-[1] benzazepin-2-on (0,7 g) in Aethanol (50 ml) wird mit 10 %igem Palladium-auf-Kohle-Katalysator (0,5 g) 24 Stunden bei 70° und 2,9 Atmosphären hydriert. Der Katalysator wird abfiltriert und das Lösungsmittel unter vermindertem Druck abgedampft. Man erhält das 3(S)-t-butyloxycarbonylamino-1-äthoxycarbonylmethyl-2,3,4,5-tetrahydro-1H-[1] benzazepin-2-on, welches ohne weitere Reinigung, gemäss dem oben beschriebenen Verfahren in das 3(S)-Amino-1-äthoxycarbonylmethyl-2,3,4,5-tetrahydro-1H-[1] benzazepin-2-on umgewandelt wird. F. 99-101°, $[\alpha]_D = - 297$ (c = 1 in Aethanol).

e) Eine Lösung von 3(S)-t-Butyloxycarbonylamino-2,3,4,5-tetrahydro-1H-1-[1] benzazepin-2,5-dion (12,5 g), hergestellt aus L-Kynurenin wie in Australian J. Chemistry Vol. 33, 633-640 (1980) beschrieben, und Bromessigsäure-t-butylester (10,1 g) in Aceton (700 ml) wird bei Zimmertemperatur in einer trockenen Stickstoffatmosphäre gerührt. Man gibt Kaliumcarbonat (12,5 g) in einer Portion dazu und rührt die erhaltene Suspension 16 Stunden bei Zimmertemperatur. Die Kaliumsalze werden abfiltriert und das Filtrat wird zur Trockene eingedampft. Der Rückstand wird zwischen Essigsäureäthylester (250 ml) und Wasser (250 ml) verteilt. Die Schichten werden getrennt und die organische Phase über Natriumsulfat getrocknet. Der Rückstand wird mit Petroläther (350 ml ; Siedepunkt 30-60°) trituriert. Man erhält das 3(S)-t-Butyloxycarbonyl-amino-1-t-butyloxycarbonylmethyl-2,3,4,5-tetrahydro-1H-[1] benzazepin-2,5-dion. F. 75-77°. $[\alpha]_D = -172°$ (c = 0,96 in Dimethylformamid).

Eine Lösung von 3(S)-t-Butyloxycarbonylamino-1-t-butyloxycarbonylmethyl-2,3,4,5-tetrahydro-1H-[1] benzazepin-2,5-dion (8,0 g) in Aethanol (500 ml) wird mit Platinoxid (800 mg) 2 Stunden bei atmosphärischem Druck und Zimmertemperatur hydriert. Der Katalysator wird abfiltriert und das Filtrat eingedampft. Man erhält das 3(S)-t-Butyloxycarbonyl-amino-1-t-butyloxycarbonylmethyl-5-hydroxy-2,3,4,5-tetrahydro-1H-[1]-benzazepin-2-on, $[\alpha]_D = -173°$ (c = 1,8 in Dimethylformamid).

Eine Suspension von 3(S)-t-Butyloxycarbonylamino-1-t-butyloxycarbonylmethyl-5-hydroxy-2,3,4,5-tetrahydro-1H-[1] benzazepin-2-on (3,0 g), Dicyclohexylcarbodiimid (5,0 g) und Kupfer-I-chlorid (500 mg) wird mechanisch gerührt und in einer trockenen Stickstoffatmosphäre 16 Stunden auf 80° erhitzt. Das Gemisch wird gekühlt, mit Methylenchlorid (100 ml) verdünnt und filtriert. Das feste Material wird verworfen. Das Filtrat wird mit 7 %igem Ammoniumhydroxid (4 × 75 ml), dann mit 1 × 100 ml Wasser und mit gesättigter wässeriger Natriumchloridlösung (100 ml) gewaschen. Die organische Phase wird über Natriumsulfat getrocknet und eingedampft. Man erhält ein Gemisch des gewünschten Addukts und des überschüssigen Dicyclohexylcarbodiimids.

Dieses Gemisch (5,5 g) wird in Essigsäureäthylester (200 ml) gelöst, in ein Druckgefäss eingefüllt, mit 10 %igem Palladium-Kohle-Katalysator (3,0 g) versetzt und 16 Stunden bei 40° und 3 Atmosphären hydriert. Der Katalysator wird abfiltriert und das Filtrat eingedampft. Der Rückstand wird mit Aether (75 ml) trituriert. Man erhält ein weisses festes Material, welches das 3 (S)-t-Butyloxycarbonylamino-1-t-butyloxycarbonylmethyl-2,3,4,5-tetrahydro-1H-[1] benzazepin-2-on ist. F. 145-147° $[\alpha]_D = -194°$ (c = 0,46 in Dimethylformamid).

Eine Lösung von 3(S)-t-Butyloxycarbonylamino-1-t-butyloxycarbonylmethyl-5-hydroxy-2,3,4,5-tetrahydro-1H-[1] benzazepin-2-on (oben beschrieben ; 3,0 g) in Essigsäureanhydrid (50 ml) wird 2 Stunden in einer trockenen Stickstoffatmosphäre auf 80° erhitzt. Das Essigsäureanhydrid wird abgedampft, der Rückstand in Essigsäureäthylester (75 ml) gelöst und mit gesättigter wässeriger Natriumhydrogencarbonatlösung (50 ml), Wasser (50 ml) und gesättigter wässeriger Natriumchloridlösung (50 ml) gewaschen. Die organische Phase wird über Natriumsulfat getrocknet, eingedampft und der Rückstand mit Aether (50 ml) trituriert. Man erhält das 3(S)-t-Butyloxycarbonylamino-1-t-butyloxycarbonylmethyl-5-acetoxy-2,3,4,5-tetrahydro-1H-[1] benzazepin-2-on. F. 164-166,5°, $[\alpha]_D = -169°$ (c = 0,36 in Dimethylformamid).

Eine Lösung von 3(S)-t-Butyloxycarbonylamino-1-t-butyloxycarbonylmethyl-5-acetoxy-2,3,4,5-tetrahydro-1H-[1] benzazepin-2-on (2,2 g) in Aethanol (300 ml) wird mit 10 %igem Palladium-auf-Kohle-Katalysator in ein Druckgefäss eingefüllt. Das Gemisch wird 3 Tage bei 70° und 3 Atmosphären hydriert. Der Katalysator wird abfiltriert und das Filtrat eingedampft. Man erhält das 3(S)-t-Butyloxycarbonylamino-1-t-butyloxycarbonylmethyl-2,3,4,5-tetrahydro-1H-[1] benzazepin-2-on. F. 164-165°, $[\alpha]_D = -200,6°$ (c = 0,64 in Dimethylformamid).

Durch eine Lösung von 3(S)-t-Butyloxycarbonylamino-1-t-butyloxycarbonylmethyl-2,3,4,5-tetrahydro-1H-[1] benzazepin-2-on (0,85 g) in Essigsäureäthylester (40 ml) wird 2 Stunden Chlorwasserstoffgas und dann 30 Minuten Stickstoff geleitet. Der Essigsäureäthylester wird abgedampft und das als Rückstand erhaltene weisse Material in Methanol (40 ml) gleich gelöst. Propylenoxid (5 ml) wird dazu gegeben und das Gemisch 16 Stunden bei Zimmertemperatur gerührt. Das als Niederschlag erhaltene weisse Material wird abfiltriert. Man erhält das 3(S)-Amino-1-carboxymethyl-2,3,4,5-tetrahydro-1H-[1] benzazepin-2-on, F. 275-276°, $[\alpha]_D = -287°$ (c = 0,71 in 1-normaler Chlorwasserstoffsäure). Dieses Produkt wird, wie oben beschrieben, mit Benzylpyruvinsäure-äthylester in Gegenwart von Natrium-cyanborhydrid kondensiert.

## Beispiel 13

1-Carboxymethyl-3-(1-carboxy-3-phenylpropylamino)-5-hydroxy-2,3,4,5-tetrahydro-1H-[1] benzazepin-2-on

Eine Lösung von 1-Benzyloxycarbonylmethyl-3-(1-carboxy-3-phenylpropylamino)-2,5-dihydro-1H-[1] benzazepin-2,5-dion (1,00 g) in Essigsäure (50 ml) wird mit Platinoxid (0,10 g) versetzt. Das Gemisch wird in einem Druckgefäss 5 Stunden bei 2,9 Atmosphären hydriert. Der Katalysator wird abfiltriert, das Filtrat eingedampft und das erhaltene Oel mit wasserfreiem Aethanol trituriert. Das erhaltene feste Material wird abgetrennt, getrocknet und in Wasser (10 ml) suspendiert. Die Suspension wird 1,5 Stunden gerührt, das feste Material abgetrennt und getrocknet. Man erhält das unreine 1-Carboxymethyl-3-(1-carboxy-1-phenylpropylamino)-5-hydroxy-2,3,4,5-tetrahydro-1H-[1] benzazepin-2-on, welches unter Zersetzung bei 179° schmilzt.

Der Ausgangsstoff wird wie folgt hergestellt: Bromessigsäure-benzylester (9,16 g, 0,04 mol) wird tropfenweise zu einem Gemisch von 3-Methoxy-2,5-dihydro-1H-[1] benzazepin-2,5-dion [8,13 g; 0,04 mol; hergestellt wie in Canadian J. Chem., *52*, 610 (1974) beschrieben], pulverisiertem Kaliumhydroxid (2,24 g; 0,04 mol) und Tetrabutylammoniumbromid (1,29 g, 0,004 mol) in 1 000 ml Acetonitril, unter Rühren bei Zimmertemperatur, gegeben. Nach beendigter Zugabe wird die Suspension 64 Stunden bei Zimmertemperatur gerührt, filtriert und das Filtrat unter vermindertem Druck konzentriert. Man erhält ein teilweise kristallines Oel. Dieses wird mit Aether trituriert, wobei man ein festes Material erhält. Dieses wird in Essigsäureäthylester (100 ml) suspendiert und 1,5 Stunden gerührt. Das unlösliche Material wird abfiltriert und das Filtrat konzentriert. Man erhält das rohe 1-Benzyloxycarbonylmethyl-3-methoxy-2,5-dihydro-1H-[1] benzazepin-2,5-dion, welches im nächsten Schritt direkt verwendet wird.

Zu einer 1,0-molaren Lösung von Kalium-t-butoxid (0,64 g; 0,005 7 mol) in t-Butanol (5,7 ml) wird unter Rühren in einer Stickstoffatmosphäre bei Zimmertemperatur (+)-Homophenylalanin (1,02 g; 0,005 7 mol) in einer Portion gegeben. Die erhaltene Suspension wird mit t-Butanol (4,3 ml) erwärmt bis sich der Grossteil des suspendierten festen Materials auflöst. Nach Abkühlung erhält man eine Suspension. Diese Suspension wird portionenweise, mit einer Pipette, zu einer unter Rückfluss kochenden, in einer Stickstoffatmosphäre gerührten Lösung von 1-Benzyloxycarbonylmethyl-3-methoxy-2,5-dihydro-1H-[1] benzazepin-2,5-dion (2,00 g) in t-Butanol (40 ml) innerhalb 10 Minuten zugegeben. Während der Zugabe bildet sich ein gelber Niederschlag. Nach beendigter Zugabe wird die erhaltene Suspension 3 Stunden unter Rückfluss gekocht und dann filtriert. Das erhaltene gummiartige feste Material wird mit Petroläther gewaschen und in Wasser (20 ml) aufgelöst. Die Lösung wird filtriert und mit 3-normaler Chlorwasserstoffsäure auf den pH-Wert 5 eingestellt. Das erhaltene rohe 1-Benzyloxycarbonylmethyl-3-(1-carboxy-3-phenylpropylamino)-2,5-dihydro-1H-[1] benzazepin-2,5-dion wird abgetrennt und für die Herstellung der Titelverbindung direkt verwendet.

## Beispiel 14

Analog zu den beschriebenen Methoden werden die folgenden Verbindungen der Formel I, worin X = $H_2$, $R_2$ und $R_5$ = H, $R_6$ = $OC_2H_5$ und $R_7$ = OH, hergestellt:

| Nr. | $R_1$ | $R_3$ | $R_4$ |
|---|---|---|---|
| 1 | $C_6H_5CH_2$ | H | H |
| 2 | $C_6H_5CH_2CH_2$ | 7-$OCH_3$ | 8-$OCH_3$ |
| 3 | $C_6H_5CH_2CH_2$ | 7-Cl | H |
| 4 | $C_6H_5CH_2CH_2$ | 8-$CH_3$ | H |
| 5 | $C_6H_5CH_2CH_2$ | 8-$OCH_3$ | H |
| 6 | p-$ClC_6H_4CH_2CH_2$ | H | H |
| 7 | $CH_3$ | H | H |

Die für die Verbindungen 2-5 verwendeten substituierten 2,3,4,5-Tetrahydro-1H-[1] benzazepin-2-on-Ausgangsstoffe werden wie folgt hergestellt:

Das 7-Chlor-2,3,4,5-tetrahydro-1H-[1] benzazepin-2-on, F. 164-165° wird wie in der britischen Patentschrift 1,359,285 beschrieben, hergestellt.

Das 8-Methyl-2,3,4,5-tetrahydro-1H-[1] benzazepin-2-on wird gemäss der Methode von Huisgen, Liebigs Ann. Chem. *574*, 171 (1951) hergestellt. F. 153-154°.

Das 7,8-Dimethoxy-2,3,4,5-tetrahydro-1H-[1] benzazepin-2-on wird wie folgt hergestellt: Eine Lösung von 24 g 6,7-Dimethoxy-α-tetralon [Snider, T. et al., Org. Prep. Proced. Int., *5*, 291 (1973)] in Aethanol (300 ml) und Wasser (60 ml) wird zur Herstellung des Oxims, 2 Stunden mit Hydroxylamin-hydrochlorid (16 g) und Natriumhydroxid (25 g) unter Rückfluss gekocht. Das Reaktionsgemisch wird in 500 ml eines Eis/Wasser-Gemisches gegossen und mit 3 × 300 ml Dichlormethan extrahiert. Die kombinierten Extrakte werden mit 200 ml Wasser gewaschen, über wasserfreiem Magnesiumsulfat getrocknet und eingedampft. Man erhält 25 g des Oxims, welches bei 154-156° schmilzt.

Das Oxim wird wieder in Dichlormethan (170 ml) aufgelöst und mit Polyphosphat-ester (170 ml) (Fieser and Fieser: Reagents for Organic Synthesis, Wiley N.Y. 1967, p. 892) versetzt. Das Reaktionsgemisch wird 18 Stunden unter Rückfluss gekocht. Die Dichlormethan-Schicht wird abgetrennt, mit Aktivkohle behandelt und über Magnesiumsulfat getrocknet. Man erhält das 7,8-Dimethoxy-2,3,4,5-tetrahydro-1H-[1]-benzazepin-2-on. F. 153-156°.

Das 8-Methoxy-2,3,4,5-tetrahydro-1H-[1] benzazepin-2-on, F. 132-134°, wird in analoger Weise, ausgehend von 7-Methoxy-α-tetralon hergestellt.

Das 3-Amino-7-chlor-2,3,4,5-tetrahydro-1H-[1] benzazepin-2-on wird wie folgt hergestellt: Eine Lösung von 3-Amino-2,3,4,5-tetrahydro-1H-[1]-benzazepin-2-on (4,0 g), 2-t-Butyloxycarbonyloxyimino-2-phenylacetonitril (6,1 g) und Triäthylamin (5 ml) in Wasser (20 ml) und Dioxan (25 ml) wird bei Zimmertemperatur 18 Stunden gerührt. Der erhaltene Niederschlag wird abfiltriert und mit Wasser gewaschen. Nach Umkristallisation aus Essigsäureäthylester erhält man das 3-t-Butyloxycarbonylamino-2,3,4,5-tetrahydro-1H-[1] benzazepin-2-on. F. 199-201°.

Durch eine Lösung von 3-t-Butyloxycarbonylamino-2,3,4,5-tetrahydro-1H-[1] benzazepin-2-on (1,5 g) in Essigsäure (20 ml) wird 10 Minuten Chlorwasserstoffgas geleitet. Das Reaktionsgemisch wird weitere 10 Minuten gerührt. Der Niederschlag wird abgetrennt, in Wasser (30 ml) suspendiert und mit wässerigem Ammoniak basisch gemacht. Nach Filtrierung erhält man das 3-Amino-7-chlor-2,3,4,5-tetrahydro-1H-[1] benzazepin-2-on, welches bei 170-171° schmilzt.

## Beispiel 15

Herstellung von 10 000 Tabletten mit einem Gehalt von je 5 mg der aktiven Substanz des Beispiels 1 :

| Bestandteile : | |
|---|---|
| 1-Carboxymethyl-3-(1-äthoxycarbonyl-3-phenylpropylamino)-2,3,4,5-tetrahydro-1H-[1] benzazepin-2-on | 100 g |
| Milchzucker | 1 157 g |
| Maisstärke | 75 g |
| Polyäthylenglykol 6000 | 75 g |
| Talkpulver | 75 g |
| Magnesiumstearat | 18 g |
| Gereinigtes Wasser | q.s. |

Verfahren : Sämtliche pulverigen Bestandteile werden mit einem Sieb von 0,6 mm Maschenweite gesiebt. Dann wird der Wirkstoff mit Milchzucker, Talk, Magnesiumstearat und mit der Hälfte der Stärke in einem geeigneten Mischer vermischt. Die andere Hälfte der Stärke wird in 40 ml Wasser suspendiert und die Suspension zur siedenden Lösung von Polyäthylenglykol in 150 ml Wasser gegeben. Die erhaltene Paste wird zu den Pulvern gegeben und gegebenenfalls unter Zugabe einer weiteren Wassermenge granuliert. Das Granulat wird über Nacht bei 35° getrocknet, durch ein Sieb von 1,2 mm Maschenweite getrieben und zu Tabletten mit 6,4 mm Durchmesser, welche eine Bruchrille aufweisen, gepresst.

## Beispiel 16

Injektionspräparat enthaltend 25 mg des Wirkstoffs des Beispiels 1 in 5 ml Lösung :

| Bestandteile : | |
|---|---|
| 1-Carboxymethyl-3-(1-äthoxycarbonyl-3-phenylpropylamino)-2,3,4,5-tetrahydro-1H-[1] benzazepin-2-on-hydrochlorid | 25,0 g |
| Propylparaben | 1,0 g |
| Wasser für Injektionen q.s. | ad 5 000,0 ml |

Verfahren : Der Wirkstoff und das Konservierungsmittel werden in 3 500 ml Wasser für Injektionen gelöst und auf 5 000 ml verdünnt. Die Lösung wird durch ein Steril-Filter filtriert und unter sterilen Bedingungen in Ampullen, welche je 5 ml Lösung enthalten, gefüllt.

## Beispiel 17

Herstellung von 10 000 Kapseln mit einem Gehalt von je 20 mg der aktiven Substanz des Beispiels 9 :

| Bestandteile : | |
|---|---|
| 1-Carboxymethyl-3-(1-carboxy-3-phenylpropylamino)-2,3,4,5-tetrahydro-1H-[1]-benzazepin-2-on | 200 g |
| Milchzucker | 1 700 g |
| Talkpulver | 100 g |

Verfahren : Sämtliche pulverigen Bestandteile werden mit einem Sieb von 0,6 mm Maschenweite gesiebt. Dann wird der Wirkstoff zuerst mit Talk und darauffolgend mit Milchzucker in einem geeigneten Mischer homogenisiert. Kapseln Nr. 3 werden mit je 200 mg des Gemisches mit einer Füllmaschine gefüllt.

In analoger Weise werden Tabletten, injizierbare Präparate oder Kapseln von anderen erfindungsgemässen Verbindungen, z. B. solchen, die in den weiteren Beispielen hier illustriert sind, hergestellt.

Beispiel 18

1-Carboxymethyl-3S-(1R-äthoxycarbonyl-3-phenylpropylamino)-2,3,4,5-tetrahydro-1H-[1] benzazepin-2-on

Das Methyl-äthyl-keton-Filtrat der Kristallisation des 1-Carboxymethyl-3S-(1S-äthoxycarbonyl-3-phenylpropylamino)-2,3,4,5-tetrahydro-1H-[1]-benzazepin-2-on-hydrochlorids des Beispiels 12 wird eingedampft und der Rückstand mit Essigsäureäthylester (50 ml) trituriert. Das erhaltene feste Material wird zwischen Essigsäureäthylester (100 ml) und Wasser (100 ml) verteilt und der pH-Wert mit konz. Chlorwasserstoffsäure auf 4,3 eingestellt. Die Schichten werden getrennt und die wässerige Phase wird mit Essigsäureäthylester (2 × 100 ml) extrahiert. Die vereinigten Essigsäureäthylester-Lösungen werden über Natriumsulfat getrocknet und unter vermindertem Druck eingedampft. Der Rückstand wird durch Flüssigchromatographie (hplc) unter Verwendung einer $C_{18}$ Reverse Phase präparativen Säule in seine Komponenten getrennt. Als Lösungsmittel verwendet man Wasser/Methanol (3 : 7), welches 0,05 % Essigsäure enthält. So wird eine zusätzliche Menge des S,S-Isomers des Beispiels 12, und auch das S,R-Isomer erhalten. Das dem S,R-Isomer entsprechende Material wird in Dichlormethan (75 ml) gelöst und Chlorwasserstoffgas in die Lösung eingeleitet. Das Lösungsmittel wird unter vermindertem Druck eingedampft und der Rückstand aus Methyl-äthyl-keton umkristallisiert. Man erhält das 1-Carboxymethyl-3S-(1R-äthoxycarbonyl-3-phenyl-propylamino-2,3,4,5-tetrahydro-1H-[1] benzazepin-2-on-hydrochlorid. F. 181-183°, $[\alpha]_D = -188°$ (c = 0,8 in Aethanol).

Beispiel 19

1-Carboxymethyl-3S-(1S-carboxy-3-phenylpropylamino)-2,3,4,5-tetrahydro-1H-[1] benzazepin-2-on

Eine Lösung von 1-Carboxymethyl-3S-(1S-äthoxycarbonyl-3-phenylpropylamino)-2,3,4,5-tetrahydro-1H-[1] benzazepin-2-on-hydrochlorid (1 g) in Methanol (10 ml) wird mit einer Lösung von Natriumhydroxid (0,27 g) in Wasser (2 ml) versetzt. Das Reaktionsgemisch wird 18 Stunden bei Zimmertemperatur gerührt und dann unter vermindertem Druck eingedampft. Der Rückstand wird in Wasser (25 ml) gelöst und der pH-Wert mit 4-normaler Chlorwasserstoffsäure auf 3 eingestellt. Der erhaltene Niederschlag wird abfiltriert, mit Wasser gewaschen und getrocknet. Man erhält das 1-Carboxymethyl-3S-(1S-carboxy-3-phenylpropylamino)-2,3,4,5-tetrahydro-1H-[1] benzazepin-2-on, welches bei 270-272° schmilzt. $[\alpha]_D = -200,5°$ (c = 1 in 3 %igem wässerigem Ammoniak).

Beispiel 20

1-Aethoxycarbonylmethyl-3-(1-benzyloxycarbonyl-3-phenylpropylamino-2,3,4,5-tetrahydro-1H-[1] benzazepin-2-on

Eine Lösung von 3-(1-Benzyloxycarbonyl-3-phenylpropylamino)-2,3,4,5-tetrahydro-1H-[1] benzazepin-2-on (5,0 g) in trockenem Dimethylformamid wird in einer Stickstoffatmosphäre zu einer gerührten Suspension von Natriumhydrid [hergestellt aus 60 %iger Dispersion in Mineralöl (0,5 g), gewaschen mit Petroläther (3 × 80 ml)] in trockenem Dimethylformamid (100 ml) bei Zimmertemperatur gegeben. Das Gemisch wird weitere 30 Minuten bei Zimmertemperatur gerührt und dann mit einer Lösung von Bromessigsäure-äthylester (2,0 g) in trockenem Dimethylformamid (10 ml) versetzt. Das Reaktionsgemisch wird weitere 30 Minuten bei Zimmertemperatur gehalten, dann auf 50° erwärmt und 18 Stunden bei dieser Temperatur gehalten. Das Gemisch wird auf Zimmertemperatur gekühlt und das Lösungsmittel im Hochvakuum abgedampft. Der Rückstand wird in Wasser (150 ml) gelöst und die Lösung mit Essigsäureäthylester extrahiert (2 × 300 ml). Die vereinigten Extrakte werden mit Wasser (100 ml) gewaschen, über Magnesiumsulfat getrocknet und das Lösungsmittel wird unter vermindertem Druck abgedampft. Man erhält ein braunes Oel, welches auf Silicagel (250 g) chromatographiert und mit Toluol/Essigsäureäthylester (9 : 1 ; 600 ml) eluiert wird. Man erhält ein Oel, welches das Isomer A der Titelverbindung ist. Weitere Eluierung mit 1 000 ml des Lösungsmittelgemisches ergibt ein Oel, welches das Isomer B der Titelverbindung ist.

Beispiel 21

1-Aethoxycarbonylmethyl-3-(1-carboxy-3-phenylpropylamino)-2,3,4,5-tetrahydro-1H-[1] benzazepin-2-on

1-Aethoxycarbonylmethyl-3-(1-benzyloxycarbonyl-3-phenylpropylamino)-2,3,4,5-tetrahydro-1H-[1] benzazepin-2-on (das Isomer B des Beispiels 20 ; 1,1 g) in Aethanol (150 ml) wird mit Palladium-auf-Kohle-Katalysator (0,5 g) bei Zimmertemperatur und atmosphärischem Druck hydriert. Nach beendigter Wasserstoffaufnahme wird der Katalysator abfiltriert, und das Lösungsmittel unter vermindertem Druck abgedampft. Man erhält ein halbfestes Material, welches mit Aether (30 ml) trituriert wird. Man erhält das Isomer B der Titelverbindung. F. 175-177°.

Beispiel 22

1-Carboxymethyl-3-(1-äthoxycarbonyl-3-phenylpropylamino)-8-methoxy-2,3,4,5-tetrahydro-1H-[1] benzazepin-2-on (das Isomer B)

Eine Lösung von 3-Amino-1-carboxymethyl-8-methoxy-2,3,4,5-tetrahydro-1H-[1] benzazepin-2-on (4,0 g) und Benzylpyruvinsäure-äthylester (9,4 g) in einem Gemisch von Essigsäure (35 ml) und Methanol (35 ml) wird eine Stunde geruhrt. Eine Lösung von Natriumcyanborhydrid (1,1 g) in Methanol (50 ml) wird dann langsam innerhalb 5 Stunden, zugegeben. Das Gemisch wird weitere 16 Stunden gerührt, mit konzentrierter Chlorwasserstoffsaure (4 ml) versetzt und eine Stunde weiter gerührt. Die Lösungsmittel werden unter vermindertem Druck abgedampft und der Rückstand wird zwischen Wasser (75 ml) und Aether (35 ml) verteilt. Der pH-Wert wird auf 9,4 eingestellt, die Aetherschicht abgetrennt und verworfen. Die wässerige Schicht wird auf den pH-Wert 4,3 gestellt und mit Essigsäureäthylester (3 × 50 ml) extrahiert. Die vereinigten Essigsäureäthylesterlösungen werden über Magnesiumsulfat getrocknet und das Lösungsmittel wird unter vermindertem Druck abgedampft. Das Rohprodukt wird in Methylenchlorid (100 ml) gelöst und man leitet Chlorwasserstoffgas 5 Minuten in die Lösung. Die Lösung wird eingedampft und der Rückstand mit Aether (75 ml) gerührt. Das Produkt wird abfiltriert. Man erhält ein ungefähr 70 : 30-Gemisch von Diastereomeren, wie dies durch h.p.l.c. festgestellt wird.

Das Produkt wird aus 3-Pentanon umkristallisiert. Man erhält das 1-Carboxymethyl-8-methoxy-3-(1-äthoxycarbonyl-3-phenylpropylamino)-2,3,4,5-tetrahydro-1H-[1] benzazepin-2-on-hydrochlorid (das Isomer B), welches bei 240-245° (Zersetzung) schmilzt.

Der Ausgangsstoff wird wie folgt hergestellt :

Eine Lösung von 8-Methoxy-2,3,4,5-tetrahydro-1H-[1] benzazepin-2-on (7,0 g, beschrieben im Beispiel 14) und Phosphorpentachlorid (30,0 g) in Xylol (200 ml) wird in einer Stickstoffatmosphäre unter Rühren auf 90° (Oelbadtemperatur) innerhalb 30 Minuten erhitzt. Das Erhitzen wird mit Pausen bei 30° und 50° durchgeführt. Es entsteht eine ausgebige Chlorwasserstoffgas-Entwicklung. Die Temperatur wird 30 Minuten bei 90° gehalten. Das Reaktionsgemisch wird heiss filtriert, um die geringe Menge eines suspendierten festen Materials zu entfernen und das Filtrat unter vermindertem Druck zur Trockene eingedampft. Der Rückstand wird unter Rühren zu einer wässerigen gesättigten Natriumcarbonatlösung (20 ml) gegeben. Das Produkt wird nach Verfestigung abfiltriert, dann in Aethanol (30 ml) aufgeschlämmt, mit Aethanol (10 ml) und Aether (10 ml) gewaschen und getrocknet. Man erhält das 3,3-Dichlor-8-methoxy-2,3,4,5-tetrahydro-1H-[1] benzazepin-2-on, welches bei 148-150° schmilzt.

Eine Lösung von 3,3-Dichlor-8-methoxy-2,3,4,5-tetrahydro-1H-[1]-benzazepin-2-on (20 g) und wasserfreiem Natriumacetat (13,2 g) in Eisessig (250 ml) wird bei atmosphärischem Druck mit 10 %igem Palladium-auf-Kohle Katalysator (1 g) bis zur beendeten Wasserstoffaufnahme hydriert. Der Katalysator wird abfiltriert und die Essigsäure unter vermindertem Druck abgedampft. Der Rückstand wird mit Wasser (100 ml) versetzt und die Suspension 1 Stunde gerührt. Das feste Material wird abfiltriert, mit Wasser (50 ml) gewaschen und getrocknet. Man erhält das 3-Chlor-8-methoxy-2,3,4,5-tetrahydro-1H-[1] benzazepin-2-on, welches bei 162-163° schmilzt.

Eine Lösung von 3-Chlor-8-methoxy-2,3,4,5-tetrahydro-1H-[1] benzazepin-2-on (12,5 g) und Natriumazid (4,3 g) in Dimethylsulfoxid (150 ml) wird 3 Stunden unter Stickstoff bei 80° gehalten. Das Reaktionsgemisch wird in Eis/Wasser (300 ml) gegossen und die Suspension 30 Minuten gerührt. Das feste Material wird abfiltriert, mit Wasser (50 ml) gewaschen und getrocknet. Man erhält das 3-Azido-8-methoxy-2,3,4,5-tetrahydro-1H-[1] benzazepin-2-on. F. 136-138°.

3-Azido-8-methoxy-2,3,4,5-tetrahydro-1H-[1] benzazepin-2-on (5 g) wird in einer Stickstoffatmosphäre zu einer gerührten Suspension von Kaliumhydroxid (1,3 g) und Tetrabutylammoniumbromid (0,7 g) in Tetrahydrofuran, welche auf 0° gekühlt ist, in einer Portion gegeben. Das Gemisch wird 5 Minuten weiter gerührt und dann, innerhalb 5 Minuten, mit einer Lösung von Bromessigsäure-äthylester (3,6 g) in Tetrahydrofuran (15 ml) versetzt. Man lässt das Reaktionsgemisch sich auf Zimmertemperatur erwärmen, indem man es weitere 2 Stunden rührt. Das Gemisch wird filtriert und das Tetahydrofuran unter vermindertem Druck abgedampft. Der Rückstand wird zwischen Wasser (50 ml) und Aether (100 ml) verteilt. Die organische Phase wird mit 2-normaler Chlorwasserstoffsäure (10 ml) gewaschen, über Magnesiumsulfat getrocknet und das Lösungsmittel unter vermindertem Druck abgedampft. Man erhält das 3-Azido-1-äthoxycarbonyl-methyl-8-methoxy-2,3,4,5-tetrahydro-1H-[1] benzazepin-2-on. F. 90-91°.

Eine Suspension von 3-Azido-1-äthoxycarbonyläthyl-8-methoxy-2,3,4,5-tetrahydro-1H-[1] benzazepin-2-on (13,8 g) in Methanol (75 ml) wird mit einer Lösung von Natriumhydroxid (1,9 g) in Wasser (75 ml) behandelt. Das Reaktionsgemisch wird 2 Stunden bei 40-45° gerührt, mit Wasser (100 ml) versetzt, mit konz. Chlorwasserstoffsäure angesäuert (10 ml) und mit Methylenchlorid (3 × 75 ml) extrahiert. Die vereinigten Methylenchlorid-Lösungen werden über Magnesiumsulfat getrocknet und unter vermindertem Druck eingedampft. Man erhält das 3-Azido-1-carboxymethyl-8-methoxy-2,3,4,5-tetrahydro-1H-[1] benzazepin-2-on, welches bei 145-147° schmilzt.

Eine Lösung von 3-Azido-1-carboxymethyl-8-methoxy-2,3,4,5-tetrahydro-1H-[1] benzazepin-2-on (11 g) in einem Gemisch von Aethanol (250 ml) und Wasser (50 ml) wird 3 Stunden bei 3 Atmosphären, unter Verwendung eines 10 %-igen Pd-C-Katalysators, bei Zimmertemperatur hydriert. Das Gemisch wird mit 2-normaler Chlorwasserstoffsäure (50 ml) versetzt und der Katalysator abfiltriert. Das Lösungsmittel wird

unter vermindertem Druck abgedampft und der Rückstand in einem Gemisch von Wasser (50 ml) und Aethanol (50 ml) gelöst. Man gibt Propylenoxid (25 ml) dazu und rührt das Gemisch 1 Stunde. Die Lösungsmittel werden unter vermindertem Druck abgedampft. Man erhält das 3-Amino-1-carboxymethyl-8-methoxy-2,3,4,5-tetrahydro-1H-[1] benzazepin-2-on, welches über 300° schmilzt.

Beispiel 23

1-(1-Carboxyäthyl)-3-(1-äthoxycarbonyl-3-phenylpropylamino)-2,3,4,5-tetrahydro-1H-[1] benzazepin-2-on-hydrochlorid

Eine Lösung von 3-Amino-1-(1-carboxyäthyl)-2,3,4,5-tetrahydro-1H-[1] benzazepin-2-on-hydrochlorid (3 g) und Benzylpyruvinsäure-äthylester (6,5 g) in Essigsäure (30 ml) und Methanol (30 ml) wird eine Stunde bei Zimmertemperatur gerührt. Man gibt Natriumcyanborhydrid (0,8 g) in Methanol (10 ml) innerhalb 4 Stunden dazu. Das Reaktionsgemisch wird 24 Stunden bei Raumtemperatur gerührt, mit konz. Chlorwasserstoffsäure (2 ml) versetzt und eine Stunde gerührt. Die Lösungsmittel werden unter vermindertem Druck abgedampft und der Rückstand zwischen Wasser (50 ml) und Aether (30 ml) verteilt. Der pH-Wert wird auf 9,4 eingestellt, die Aetherschicht abgetrennt und verworfen. Die wässerige Lösung wird auf den pH-Wert 4,3 eingestellt und mit Essigsäureäthylester (3 × 50 ml) extrahiert. Die vereinigten Extrakte werden über Magnesiumsulfat getrocknet und das Lösungsmittel unter vermindertem Druck abgedampft. Chlorwasserstoffgas wird 2 Minuten in die Lösung des Rohproduktes in Methylenchlorid (10 ml) eingeleitet und die Lösung eingedampft. Man erhält das 1-(1-Carboxyäthyl)-3-(1-äthoxycarbonyl-3-phenylpropylamino)-2,3,4,5-tetrahydro-1H-[1] benzazepin-2-on-hydrochlorid als ein Gemisch von Diastereomeren. F. 87-94°.

Der Ausgangsstoff wird wie folgt hergestellt :

3-Azido-2,3,4,5-tetrahydro-1H-[1] benzazepin-2-on (hergestellt gemäss Beispiel 1 ; 5 g) wird in einer Portion zu einer gerührten Suspension von Kaliumhydroxid (1,8 g) und Tetrabutylammoniumbromid (1,8 g) in Tetrahydrofuran (50 ml) in einer Stickstoffatmosphäre bei 0° gegeben. Das Rühren wird 5 Minuten fortgesetzt und das Gemisch wird dann mit (R)-2-Brompropionsäure-t-butylester [J. P. Greenstein et al., J. Am. Chem. Soc. *76*, 6054 (1954), H. Niedrich und G. Koller, J. Prakt. Chem. *316*, 729 (1974)] (5,2 g) in Tetrahydrofuran (15 ml) innerhalb 5 Minuten versetzt. Man lässt das Reaktionsgemisch sich auf Raumtemperatur, unter Rühren 2 weitere Stunden, zu erwärmen. Das Reaktionsgemisch wird filtriert und unter vermindertem Druck eingedampft. Der Rückstand wird zwischen Wasser (50 ml) und Aether (100 ml) verteilt.

Die organische Phase wird mit 2-normaler Chlorwasserstoffsäure (10 ml) gewaschen, über Magnesiumsulfat getrocknet und das Lösungsmittel wird unter vermindertem Druck abgedampft. Man erhält das 3-Azido-1-(1-t-butyloxycarbonyläthyl)-2,3,4,5-tetrahydro-1H-[1] benzazepin-2-on als ein Oel, welches ohne weitere Reinigung verwendet wird.

Eine Lösung von 3-Azido-1-(1-t-butyloxycarbonyläthyl-2,3,4,5-tetrahydro-1H-[1] benzazepin-2-on (7 g) in Aethanol (70 ml) wird bei 3-Atmosphären Druck 3 Stunden, unter Verwendung von 10 %igem Pd-C-Katalysator (0,5 g) hydriert. Der Katalysator wird abfiltriert und das Aethanol unter vermindertem Druck abgedampft. Man erhält das 3-Amino-1(1-t-butyloxycarbonyläthyl)-2,3,4,5-tetrahydro-1H-[1] benzazepin-2-on als ein Oel. Die Flüssigchromatographie unter Hochdruck (h.p.l.c.) zeigt, dass das Produkt ein ungefähr 1 : 1-Gemisch von Diastereomeren ist. Dieses Material wird ohne weitere Reinigung verwendet.

Eine Lösung des obigen 3-Amino-1-(1-t-butyloxycarbonyläthyl)-2,3,4,5-tetrahydro-1H-[1] benzazepin-2-on (4,7 g) in Trifluoressigsäure (25 ml) wird eine Stunde bei Raumtemperatur gerührt. Die Trifluoressigsäure wird unter vermindertem Druck abgedampft und der Rückstand in Aether (100 ml) aufgenommen. In die Lösung wird Chlorwasserstoffgas bis zum Abschluss der Bildung eines Niederschlags eingeleitet. Das feste Material wird abfiltriert. Man erhält das 3-Amino-1-(1-carboxyäthyl)-2,3,4,5-tetrahydro-1H-[1] benzazepin-2-on-hydrochlorid. F. 165-176°. H.p.l.c. zeigt, dass das Produkt ein ungefähr 1 : 1-Gemisch der Diastereomere ist.

Beispiel 24

1-Aethoxycarbonylmethyl-3S-(1S-äthoxycarbonyl-3-phenylpropylamino)-2,3,4,5-tetrahydro-1H-[1] benzazepin-2-on

Eine Lösung von 3S-Amino-1-äthoxycarbonylmethyl-2,3,4,5-tetrahydro-1H-[1] benzazepin-2-on (1,5 g), 2-Brom-4-phenylbuttersäureäthylester (1,6 g) und Triäthylamin (0,8 ml) in Dimethylformamid (37 ml) wird unter Stickstoff 18 Stunden bei 70° gerührt. Das Dimethylformamid wird dann unter vermindertem Druck abgedampft. Der Rückstand wird in Essigsäureäthylester (70 ml) aufgenommen, mit Wasser (5 × 25 ml) gewaschen, über Magnesiumsulfat getrocknet und eingedampft. Das Produkt-Gemisch wird dann durch Silicagel-Chromatographie aufgetrennt, wobei man als Lösungsmittel Essigsäureäthylester Hexan (40 : 60) als Lösungsmittel verwendet. Man erhält ungefähr gleiche Mengen von 1-Aethoxycarbonylmethyl-3S-(1S-äthoxycarbonyl-3-phenyl-propylamino)-2,3,4,5-tetrahydro-1H-[1] benzazepin-2-on [NMR ($CDCl_3$) δ 4,52 (q, 2H)] das S,S-Enantiomer der Verbindung des Beispiels 10, und seinem

Diastereomer, 1-Aethoxycarbonylmethyl-3S-(1R-äthoxycarbonyl-3-phenylpropylamino)-2,3,4,5-tetrahydro-1H-[1] benzazepin-2-on ; NMR ($CDCl_3$) : δ 4,50 (q, 2H).

TLC (Dünnschichtchromatographie) : (Silicagel ; Essigsäureäthylester/Hexan 40 : 60) : Das (S,S)-Isomer hat einen $R_f$-Wert von 0,24 und das (S,R)-Isomer einen $R_f$-Wert von 0,33,

Beispiel 25

1-Carboxymethyl-3S-(1S-äthoxycarbonyl-3-phenylpropylamino)-2,3,4,5-1H-[1] benzazepin-2-on

Man gibt tropfenweise eine 2-normale Kaliumhydroxidlösung (0,26 ml) zu einer Lösung von 1-Aethoxycarbonylmethyl-3S-(1S-äthoxycarbonyl-3-phenylpropylamino)-2,3,4,5-tetrahydro-1H-[1] benzazepin-2-on (0,25 g) in Aethanol (5 ml), unter Rühren bei Raumtemperatur unter Stickstoff. Das Gemisch wird eine Stunde gerührt, dann das Aethanol abgedampft, der Rückstand in Wasser (5 ml) gelöst, mit 2-normaler Chlorwasserstoffsäure der pH-Wert auf 2 gestellt und mit Essigsäureäthylester (2 × 30 ml) extrahiert.

Die vereinigten Essigsäureäthylester-Lösungen werden mit gesättigter Natriumhydroxidlösung (5 ml) gewaschen, über Magnesiumsulfat getrocknet und zur Trockene eingedampft. Man erhält das 1-Carboxymethyl-3S-(1S-äthoxycarbonyl-3-phenylpropylamino)-2,3,4,5-tetrahydro-1H-[1] benzazepin-2-on, die Verbindung des Beispiels 12.

Beispiel 26

1-Carboxymethyl-7-chlor-3-(1-äthoxycarbonyl-3-phenylpropylamino)-2,3,4,5-tetrahydro-1H-[1] benzazepin-2-on (das Isomer B)

Unter Rühren bei Raumtemperatur wird in eine Lösung von 1-Carboxymethyl-3-(1-äthoxycarbonyl-3-phenylpropylamino)-2,3,4,5-tetrahydro-1H-[1] benzazepin-2-on (das Isomer B, 1,5 g) in Essigsäure (25 ml) Chlorgas eingeleitet. Es bildet sich ein weisser Niederschlag. Das Chlorgas wird bis zum Abschluss der Reaktion eingeleitet. Das feste Material wird abfiltriert und durch Flüssigchromatographie (h.p.l.c.) unter Verwendung einer $C_{18}$ reverse Phase präparativen Säule in seine Komponenten getrennt. Als Lösungsmittel wird Methanol/0,1 %ige wässerige Ammoniumcarbonatlösung (1 : 1) verwendet. Die geeignete Fraktion wird in Methanol/Essigsäureäthylester (1 : 1 ; 50 ml) gelöst und man leitet Chlorwasserstoffgas in die Lösung ein. Die Lösung wird eingedampft, der Rückstand in Aether (100 ml) suspendiert und die Suspension filtriert. Man erhält das 1-Carboxymethyl-7-chlor-3-(1-äthoxycarbonyl-3-phenylpropylamino)-2,3,4,5-tetrahydro-1H-[1] benzazepin-2-on-hydrochlorid, welches bei 149-151° schmilzt (das Isomer B).

Beispiel 27

1-Carboxymethyl-3S-(1S-äthoxycarbonyl-3-phenylpropylamino)-2,3,4,5-tetrahydro-1H-[1] benzazepin-2-on-hydrochlorid

3(S)-Amino-1-carboxymethyl-2,3,4,5-tetrahydro-1H-[1] benzazepin-2-on-natriumsalz (619 g), welches einen $[\alpha]_D^{25}$-Wert von − 304,4° (c = 1,08 in Wasser) hat, Benzylpyruvinsäureäthylester (1 960 g), wasserfreies Aethanol (5 880 ml) und Eisessig (5 880 ml) werden vermischt und bei 20-25° 1,5 Stunden gerührt. Eine Lösung von Natrium-cyanborhydrid (179 g) in wasserfreiem Aethylalkohol (2 200 ml) wird innerhalb 24 Stunden langsam und gleichmässig dazu gegeben. Nach der abgeschlossenen Zugabe wird das Reaktionsgemisch 24 Stunden gerührt. Das Reaktionsgemisch wird mit 12-normaler Chlorwasserstoffsäure (500 ml) versetzt und das Lösungsmittel bei 35-40° und mmHg abgedampft. Das zurückgebliebene Oel wird in ein Gemisch von Eis (3 000 g), Wasser (3 000 ml) und Diäthyläther (3 000 ml) gegeben und der pH-Wert des Gemisches mit 10-normaler Natriumhydroxidlösung (1 735 ml) auf 9-9,5 gestellt. Die wässerige Phase wird abgetrennt und man gibt zu der Aetherschicht weitere 8 000 ml Aether, um eine weitere Menge des Produkts als Oel zu erhalten. Die mit Aether nicht mischbare Schicht wird abgetrennt und mit der wässerigen Phase vereinigt. Dann wird der Aetherextrakt mit Wasser (2 × 1 000 ml) gewaschen, die Waschflüssigkeit wird mit dem obigen Wasser/Oel-Anteil vereinigt und der pH-Wert des Gemisches wird mit 12-normaler Chlorwasserstoffsäure (550-650 ml) auf 4,25-4,35 eingestellt. Das Gemisch wird mit Essigsäureäthylester (3 × 2 000 ml) extrahiert. Die vereinigten Essigsäureäthylester-Extrakte werden mit Wasser (2 000 ml) gewaschen und über wasserfreiem Magnesiumsulfat (500 g) getrocknet. Das Trocknungsmittel wird abfiltriert und das Lösungsmittel sorgfältig bei 40° und 3 mmHg abgedampft. Das erhaltene Oel wird in Essigsäureäthylester (4 500 ml) gelöst und mit 28 %igem ätherischen Chlorwasserstoff (309 g) unter starkem Rühren versetzt. Man gibt Diäthyläther (1 500 ml) dazu und rührt das Gemisch eine Stunde. Das feste Material wird abgetrennt und mit Essigsäureäthylester (2 × 500 ml) und Diäthyläther (3 × 1 000 ml) gewaschen. Trocknen bei 50° und 3 mmHg ergibt das rohe Produkt, welches aus ungefähr 65 % des gewünschten 1-Carboxymethyl-3S-(1S-äthoxycarbonyl-3-phenylpropylamino)-2,3,4,5-tetrahydro-1H-[1] benzazepin-2-on, welches mit dem Produkt des Beispiels 12 identisch ist, besteht. Dies wird durch h.p.l.c. unter Verwendung einer $C_{18}$ reverse Phase präparativen

Säule bestimmt. Als Lösungsmittel verwendet man ein Gemisch von Methanol, Wasser und Essigsäure (75 : 25 : 0,02).

Das obige Rohprodukt wird in Dichlormethan (26 900 ml) suspendiert und man leitet Chlorwasserstoffgas in gleichmässigem Strom ein. Nach 40 Minuten erhält man eine Lösung. Dann wird das Einleiten des Gases abgestellt. Die Lösung wird durch Filtrieren von Spuren unlöslichen Materials befreit und mit Diäthyläther (10 750 ml) versetzt.

Die Suspension wird über Nacht bei Raumtemperatur gerührt und das feste Material abfiltriert. Dieses wird mit Dichlormethan (4 × 500 ml) und Diäthyläther (3 × 1 000 ml) gewaschen. Nach dem Trocknen erhält man ein reineres Produkt als Hydrochloridsalz. F. 175-178°.

Man versetzt 1 880 g des obigen Hydrochloridsalzes mit Dichlormethan (18 000 ml). Die Suspension wird wieder mit Chlorwasserstoffgas behandelt, um eine Lösung zu erhalten. Diese wird mit Diäthyläther (7 200 ml) versetzt. Die erhaltene Suspension wird 3 Stunden gerührt und filtriert. Das feste Material wird mit Dichlormethan (2 × 1 000 ml) und Diäthyläther (2 × 1 000 ml) gewaschen und getrocknet. Man erhält das Produkt, welches bei 183-185° schmilzt. (Die h.p.l.c. zeigt, dass das Produkt 96 %-ig ist).

Man versetzt 1 280 g des obigen Salzes mit Chloroform (4 000 ml) und kocht das Gemisch 10 Minuten unter Rückfluss. Man stellt das Erhitzen ab, rührt das Gemisch 4 Stunden und filtriert es. Das feste Material wirde mit Chloroform (2 × 200 ml) und Diäthyläther (3 × 500 ml) gewaschen, getrocknet und gesiebt. Man erhält das 1-Carboxymethyl-3S-(1S-äthoxycarbonyl-3-phenylpropylamino)-2,3,4,5-tetrahydro-1H-[1] benzazepin-2-on-hydrochlorid, welches bei 184-186° schmilzt. $[\alpha]_D^{25} = -139,26°$ (c = 0,92 in absolutem Aethanol). Das Produkt ist mit dem Hydrochloridsalz des Beispiels 12 identisch.

## Beispiel 28

3-(1-Benzyloxycarbonyl-3-phenylpropylamino)-1-carboxymethyl-2,3,4,5-tetrahydro-1H-[1] benzazepin-2-on-hydrochlorid (das Isomer B).

In eine Lösung von 3-(1-Benzyloxycarbonyl-3-phenylpropylamino)-1-t-butyloxycarbonylmethyl-2,3,4,5-tetrahydro-1H-[1] benzazepin-2-on (4,0 g, siehe Beispiel 7) in Essigsäureäthylester (100 ml) wird unter Rühren bei 0° Chlorwasserstoffgas 20 Minuten eingeleitet. Das Reaktionsgemisch wird unter vermindertem Druck eingedampft und das erhaltene feste Material mit Aether (50 ml) trituriert. Das feste Material wird abfiltriert, mit Aether (15 ml) und Essigsäureäthylester (15 ml) gewaschen und dann mit Essigsäureäthylester (50 ml) gekocht. Das Produkt wird aus Methanol/Essigsäureäthylester umkristallisiert. Man erhält die Titelverbindung, welche bei 197-199° schmilzt. (Das Isomer B).

Der Ausgangsstoff wird wie folgt hergestellt : Eine Lösung von 3-(1-Benzyloxycarbonyl-3-phenylpropylamino)-2,3,4,5-tetrahydro-1H-[1] benzazepin-2-on (3,0 g) und Bromessigsäure-t-butylester (2,2 g) in Tetrahydrofuran (100 ml) wird unter trockenem Stickstoff, unter Rühren bei Raumtemperatur, mit Kalium-t-butoxid (1,2 g) versetzt. Das Reaktionsgemisch wird 20 Stunden bei Raumtemperatur gerührt, dann in Wasser (250 ml) gegossen und mit Dichlormethan (2 × 150 ml) extrahiert. Die vereinigten Extrakte werden mit Wasser (100 ml) gewaschen und über Magnesiumsulfat getrocknet. Nach Abdampfen des Lösungsmittels erhält man das 3-(1-Benzyloxycarbonyl-3-phenylpropylamino)-1-t-butyloxycarbonylmethyl-2,3,4,5-tetrahydro-1H-[1] benzazepin-2-on.

## Beispiel 29

1-Aethoxycarbonylmethyl-3-(1-äthoxycarbonyl-3-phenylpropylamino-2,3,4,5-tetrahydro-1H-[1] benzazepin-2-on

Eine Lösung von Natriummethoxid in Methanol [hergestellt aus Natrium (0,25 g) und Methanol (50 ml)] wird unter Rühren in einer Stickstoffatmosphäre mit einer Lösung von 2-(1-Aethoxycarbonyl-3-phenylpropylamino)-4-[o-(äthoxycarbonylmethylamino)-phenyl]-buttersäureäthylester (5,6 g) in Methanol (100 ml) versetzt. Das Reaktionsgemisch wird 65 Stunden unter Rückfluss gekocht und dann unter vermindertem Druck eingedampft. Der Rückstand wird zwischen Wasser (50 ml) und Dichlormethan (200 ml) verteilt. Die wässerige Lösung wird mit Dichlormethan (200 ml) extrahiert. Die vereinigten organischen Lösungen werden mit Wasser (50 ml) gewaschen und über Kaliumcarbonat getrocknet. Nach Eindampfen des Lösungsmittels erhält man ein Gemisch der Isomere A und B des 1-Aethoxycarbonylmethyl-3-(1-äthoxycarbonyl-3-phenylpropylamino)-2,3,4,5-tetrahydro-1H-[1] benzazepin-2-ons, welches durch Chromatographie auf Silicagel getrennt und gemäss Beispiel 10 in die einzelnen Maleate umgewandelt wird.

Der Ausgangsstoff wird wie folgt hergestellt :

Eine Lösung von 2-Amino-4-(o-nitrophenyl)-buttersäure-äthylester (17,4 g) in 50 %igem wässerigem Dioxan (130 ml) wird mit Triäthylamin (10,5 g) und 2-(tert.-Butyloxycarbonyloxyimino)-2-phenylacetonitril (18,7 g) versetzt. Das Reaktionsgemisch wird bei Raumtemperatur 4 Stunden gerührt und dann mit Wasser (300 ml) verdünnt. Das Gemisch wird mit Aether (2 × 150 ml) extrahiert, die wässerige Phase mit eiskalter, 2-normaler Chlorwasserstoffsäure angesäuert und mit Essigsäureäthylester (2 × 250 ml) extrahiert. Die Essigsäureäthylester-Schichten werden vereinigt, mit Wasser (150 ml) gewaschen und über Natriumsulfat getrocknet. Das Lösungsmittel wird unter vermindertem Druck abgedampft. Man erhält den 2-t-

Butyloxycarbonylamino-4-(o-nitrophenyl)-buttersäure-äthylester, welcher ohne weitere Reinigung verwendet wird.

Eine Lösung von 2-t-Butyloxycarbonylamino-4-(o-nitrophenyl)-buttersäure-äthylester (13,0 g) in Aethanol (300 ml) wird bei Raumtemperatur und atmosphärischen Druck mit 10 %igem Palladium-auf-Kohle-Katalysator (1 g) bis zum Abschluss der Wasserstoffaufnahme hydriert. Der Katalysator wird abfiltriert und das Lösungsmittel abgedampft. Man erhält den 2-t-Butyloxycarbonylamino-4-(o-aminophenyl)-buttersäure-äthylester, der ohne weitere Reinigung im folgenden Schritt verwendet wird.

Eine Lösung von 2-t-Butyloxycarbonylamino-4-(o-aminophenyl)-buttersäure-äthylester (10,0 g) und Glyoxylsäure-äthylester (4,2 g) in Aethanol (120 ml) wird bei 80° und 3-Atmosphären 72 Stunden mit 10 %igem Palladium-auf-Kohle-Katalysator hydriert. Das Reaktionsgemisch wird auf Raumtemperatur gekühlt und der Katalysator abfiltriert. Das Lösungsmittel wird unter vermindertem Druck abgedampft und der Rückstand zwischen Essigsäureäthylester (150 ml) und Wasser (75 ml) verteilt. Die organische Phase wird über Natriumsulfat getrocknet und das Lösungsmittel unter vermindertem Druck abgedampft. Man erhält den 2-t-Butyloxycarbonylamino-4-[o-(äthoxycarbonylmethylamino)-phenyl]-buttersäure-äthylester, der ohne weitere Reinigung im nächsten Schritt verwendet wird.

Man leitet Chlorwasserstoffgas 30 Minuten bei Raumtemperatur in eine Lösung von 2-t-Butyloxycarbonylamino-4-[o-(äthoxycarbonylmethylamino)-phenyl]-buttersäure-äthylester (8,5 g) in Essigsäureäthylester (150 ml) ein. Die Lösung wird unter vermindertem Druck eingedampft und der Rückstand in Essigsäureäthylester (100 ml) gelöst. Die Lösung wird mit Wasser (3 × 100 ml) gewaschen und über Natriumsulfat getrocknet. Das Lösungsmittel wird unter vermindertem Druck abgedampft. Man erhält den 2-Amino-4-[o-(äthoxycarbonylmethylamino)-phenyl]-buttersäure-äthylester, der ohne weitere Reinigung im nächsten Schritt verwendet wird.

Eine Lösung von 2-Amino-4-[o-(äthoxycarbonylmethylamino)-phenyl]-buttersäure-äthylester (4,7 g) und Benzylpyruvinsäure-äthylester (12,4 g) in Essigsäure (35 ml) und Methanol (35 ml) wird unter Stickstoff eine Stunde bei Raumtemperatur gerührt. Natriumcyanborhydrid (1,6 g) in Methanol (15 ml) wird innerhalb 4 Stunden tropfenweise dazu gegeben. Das Reaktionsgemisch wird 24 Stunden bei Raumtemperatur gerührt, tropfenweise mit konz. Chlorwasserstoffsäure (2 ml) versetzt und bei Raumtemperatur eine Stunde gerührt. Das Gemisch wird zur Trockene eingedampft, der Rückstand zwischen Wasser (75 ml) und Aether (75 ml) verteilt und der pH-Wert mit 6-normaler Chlorwasserstoffsäure auf 2 gestellt. Die Schichten werden getrennt und die wässerige Phase mit Aether (2 × 75 ml) extrahiert. Die Aetherextrakte werden verworfen und der pH-Wert der wässerigen Schicht wird mit 40 %iger Natriumhydroxidlösung auf 9 gestellt und mit Essigsäureäthylester (3 × 50 ml) extrahiert. Die Extrakte werden über Natriumsulfat getrocknet und unter vermindertem Druck eingedampft. Man erhält den 2-(1-Aethoxycarbonyl-3-phenylpropylamino)-4-[o-(äthoxycarbonylmethylamino)-phenyl]-buttersäure-äthylester, der für die Herstellung des Endproduktes direkt verwendet wird.

Beispiel 30

Man behandelt 2-Amino-4-phenylbuttersäure-äthylester unter den Bedingungen der reduktiven Alkylierung, wie in den vorhergehenden Beispielen beschrieben, mit 1-Aethoxycarbonylmethyl-2,3,4,5-tetrahydro-1H-[1] benzazepin-2,3-dion. Man erhält das 1-Aethoxycarbonylmethyl-3-(1-äthoxycarbonyl-3-phenylpropylamino)-2,3,4,5-tetrahydro-1H-[1] benzazepin-2-on des Beispiels 10.

Der Ausgangsstoff wird wie folgt hergestellt : Eine Lösung von 3,3-Dichlor-2,3,4,5-tetrahydro-1H-[1] benzazepin-2-on (1,0 g, 4,32 mMol) und Bromessigsäureäthylester (0,51 ml) in Tetrahydrofuran (30 ml) wird innerhalb 15 Minuten zu einer Lösung von Natriumhydrid (4,76 mMol) in Tetrahydrofuran (20 ml) in einer Stickstoffatmosphäre tropfenweise, unter Rühren, gegeben. Das Gemisch wird weitere 2 Stunden gerührt. Die Lösung wird zersetzt durch Zugabe von gesättigter, wässeriger Ammoniumchloridlösung und die Lösungsmittel werden unter vermindertem Druck abgedampft. Der Rückstand wird mit Aether extrahiert (3 × 20 ml), die vereinigten Aetherlösungen werden mit gesättigter wässeriger Natriumchloridlösung (20 ml) gewaschen und über Magnesiumsulfat getrocknet. Das Lösungsmittel wird unter vermindertem Druck abgedampft. Man erhält das 3,3-Dichlor-1-äthoxycarbonylmethyl-2,3,4,5-tetrahydro-1H-[1] benzazepin-2-on. NMR ($CDCl_3$) : δ 1,27 (t, 3H) ; 3,22 (m, 4H) ; 4,25 (q, 2H) ; 4,65 (s, 2H) und 7,3 (m, 4H).

Ein Gemisch von Morpholin (0,315 g ; 3,6 mMol) und 3,3-Dichlor-1-äthoxycarbonylmethyl-2,3,4,5-tetrahydro-1H-[1] benzazepin-2-on (0,5 g) wird unter Stickstoff bei 110° 18 Stunden gerührt. Die Lösung wird mit Chloroform auf 10 ml verdünnt und auf 0° gekühlt. Man gibt 20 %iger Schwefelsäure (1 ml) dazu und rührt die Lösung 2 Stunden bei 0°. Die Lösung wird mit Chloroform (2 × 20 ml) extrahiert und die Extrakte werden mit 2-normaler Chlorwasserstoffsäure (2 × 10 ml) und gesättigter wässeriger Natriumchloridlösung (5 ml) gewaschen. Die Lösung wird über Magnesiumsulfat getrocknet und unter vermindertem Druck eingedampft. Man erhält das 1-Aethoxycarbonylmethyl-2,3,4,5-tetrahydro-1H-[1] benzazepin-2,3-dion. NMR ($CDCl_3$ : δ 1,25 (t, 3H) ; 2,6 (m, 2H) ; 3,6 (m, 2H) ; 4,2 (q, 2H) und 7,3 (m, 4H).

Beispiel 31

2-Amino-4-phenylbuttersäureäthylester wird in Gegenwart von Kaliumcarbonat in Methylenchlorid

mit 3-Brom-1-äthoxycarbonylmethyl-2,3,4,5-tetrahydro-1H-[1] benzazepin-2-on behandelt. Man erhält das 1-Aethoxycarbonylmethyl-3-(1-äthoxycarbonyl-3-phenylpropylamino)-2,3,4,5-tetrahydro-1H-[1] benzazepin-2-on des Beispiels 10.

Der Ausgangsstoff wird wie folgt hergestellt : Eine Lösung von 2,3,4,5-Tetrahydro-1H-[1] benzazepin-2-on (2,5 g) in Chloroform (30 ml) wird mit Phosphorpentachlorid (3,2 g) portionenweise versetzt, wobei man die Temperatur bei 0-5° hält. Nach der beendeten Zugabe wird zuerst Jod (30 mg) und dann Brom (2,5 g) innerhalb von 5 Minuten tropfenweise dazu gegeben. Das Gemisch wird dann 4 Stunden unter Rückfluss gekocht. Die Chloroformlösung wird eingedampft und der Rückstand zwischen Eiswasser (30 ml) und Dichlormethan (75 ml) verteilt. Die organische Phase wird über Magnesiumsulfat getrocknet und unter vermindertem Druck eingedampft. Der rohe Rückstand wird durch Chromatographie auf Silicagel gereinigt, wobei man Aether/Hexan (7 : 3) eluiert. Nach Eindampfen der geeigneten Fraktionen erhält man das 3-Brom-2,3,4,5-tetrahydro-1H-[1] benzazepin-2-on, welches bei 146-148° schmilzt.

Eine gerührte Suspension von Kaliumhydroxid (90 mg) und Tetrabutylammoniumbromid (40 mg) in Tetrahydrofuran (10 ml) wird mit 3-Brom-2,3,4,5-tetrahydro-1H-[1] benzazepin-2-on (300 mg) in einer Portion, in einer Stickstoffatmosphäre bei 0° versetzt. Das Rühren wird 5 Minuten fortgesetzt. Das Gemisch wird mit Bromessigsäure-äthylester (200 mg) in einer Portion versetzt und man lässt es unter Rühren weitere 3 Stunden sich auf Raumtemperatur zu erwärmen. Das Tetrahydrofuran wird unter vermindertem Druck abgedampft und der Rückstand zwischen Wasser (5 ml) und Aether (25 ml) verteilt. Die organische Phase wird mit 2-normaler Chlorwasserstoffsäure (5 ml) gewaschen, über Magnesiumsulfat getrocknet und unter vermindertem Druck eingedampft. Man erhält das 3-Brom-1-äthoxycarbonylmethyl-2,3,4,5-tetrahydro-1H-[1] benzazepin-2-on. F. 114-116°.

Das 3-Chlor-äthoxycarbonyl-methyl-2,3,4,5-tetrahydro-1H-[1] benzazepin-2-on wird ähnlich hergestellt.

Eine Lösung von 3-Chlor-2,3,4,5-tetrahydro-1H-[1] benzazepin-2-on (1,95 g) in Dimethylformamid (10 ml) wird tropfenweise zu einer gerührten Lösung von Kalium-t-butoxid (1,11 g) in Dimethylformamid (10 ml) bei 5° gegeben. Die Lösung wird weitere 15 Minuten bei 5° gerührt und dann mit Bromessigsäure-äthylester (1,78 g) in Dimethylformamid (5 ml) tropfenweise versetzt. Das Rühren wird weitere 30 Minuten bei 5° und dann 3 Stunden bei Raumtemperatur fortgesetzt. Das Reaktionsgemisch wird auf 10° abgekühlt und mit Wasser (100 ml) versetzt. Die Lösung wird mit Chloroform (100 ml) extrahiert, die Chloroformlösung mit Wasser (2 × 10 ml) gewaschen und über Natriumsulfat getrocknet. Das Lösungsmittel wird unter vermindertem Druck abgedampft. Man erhält das 3-Chlor-1-äthoxycarbonylmethyl-2,3,4,5-tetrahydro-1H-[1] benzazepin-2-on NMR (Dimethylsulfoxid -$d_6$) : δ 1,2 (t, 3H) ; 2,65 (m, 4H) ; 4,15 (q, 2H) ; 2,6 (d, 2H) und 7,3 (m).

## Beispiel 32

1-Carboxymethyl-3S-(1S-pivaloyloxymethoxycarbonyl-3-phenylpropylamino)-2,3,4,5-tetrahydro-1H-[1] benzazepin-2-on

1-Benzyloxycarbonylmethyl-3S-(1S-pivaloyloxymethoxy-carbonyl-3-phenylpropylamino)-2,3,4,5-tetrahydro-1H-[1] benzazepin-2-on (3 g) wird in Aethanol (50 ml) aufgelöst und mit 10 %igem Palladium-auf-Kohle (0,3 g) versetzt. Die Lösung wird bei Raumtemperatur 2 Stunden bei einem Druck von 1 Atmosphäre hydriert.

Das Reaktionsgemisch wird filtriert und eingedampft. Man erhält das 1-Carboxy-methyl-3S-(1S-pivaloyloxymethoxycarbonyl-3-phenylpropylamino)-2,3,4,5-tetrahydro-1H-[1] benzazepin-2-on.

Der Ausgangsstoff wird wie folgt hergestellt : 1-Benzyloxycarbonylmethyl-3S-(1S-carboxy-3-phenoxypropylamino)-2,3,4,5-tetrahydro-1H-[1] benzazepin-2-on (5 g, Beispiel 2) wird in 2-normaler Kaliumhydroxidlösung (5,15 ml) gelöst und die Lösung zur Trockene eingedampft. Man gibt Pivalinsäurejodmethylester (2,3 g) und Dimethylformamid (50 ml) dazu und rührt das Gemisch unter Stickstoff 18 Stunden bei Raumtemperatur. Das Dimethylformamid wird abgedampft, der Rückstand in Essigsäure-äthylester (100 ml) aufgenommen, mit gesättigter Natriumcarbonatlösung (3 × 25 ml), Wasser (3 × 25 ml) und gesättigter Natriumchloridlösung (25 ml) gewaschen und über Magnesiumsulfat getrocknet. Nach Eindampfen erhält man das 1-Benzyloxycarbonylmethyl-3S-(1S-pivaloyloxymethoxycarbonyl-3-phenylpropylamino)-2,3,4,5-tetrahydro-1H-[1] benzazepin-2-on.

In analoger Weise werden hergestellt :

a) 1-Carboxymethyl-3S-(1S-l-bornyloxycarbonylmethoxycarbonyl-3-phenylpropylamino)-2,3,4,5-tetrahydro-1H-[1] benzazepin-2-on bei Verwendung von Jodessigsäure-l-bornylester als Ausgangsstoff.

b) 1-Carboxymethyl-3S-(1S-β-methoxyäthoxymethoxycarbonyl-3-phenylpropylamino)-2,3,4,5-tetrahydro-1H-[1] benzazepin-2-on bei Verwendung von β-Methoxyäthoxymethylchlorid als Ausgangsstoff.

c) 1-Carboxymethyl-3S-[1S-(3-phthalidoxycarbonyl)-3-phenylpropylamino]-2,3,4,5-tetrahydro-1H-[1] benzazepin-2-on bei Verwendung von 3-Bromphthalid als Ausgangsstoff.

d) 1-Carboxymethyl-3S-[1S-(3-pyridylmethoxycarbonyl)-3-phenylpropylamino]-2,3,4,5-tetrahydro-1H-[1] benzazepin-2-on bei Verwendung von 3-Pyridylmethylchlorid als Ausgangsstoff.

Beispiel 33

1-Carboxymethyl-3S-(1-äthoxycarbonyl-3-phenylpropylamino)-2,3,4,5-5a,6,7,8,9,9a-decahydro-1H-[1] benzazepin-2-on

Eine Lösung von 3(S)-Amino-1-carboxymethyl-2,3,4,5,5a,6,7,8,9,9a-decahydro-1H-[1] benzazepin-2-on-natriumsalz (0,6 g) und Benzylpyruvinsäure-äthylester (1,5 g) in Essigsäure (5 ml) und Methanol (3 ml) wird in einer trockenen Stickstoffatmosphäre 1 Stunde bei Raumtemperatur gerührt. Eine Lösung von Natrium-cyanborhydrid (0,2 g) in Methanol (2 ml) wird dann innerhalb 4 Stunden zugegeben. Das Reaktionsgemisch wird 18 Stunden bei Raumtemperatur gerührt. Man gibt konz. Chlorwasserstoffsäure dazu und rührt das Gemisch 1 Stunde. Die Lösungsmittel werden unter vermindertem Druck abgedampft und der Rückstand wird zwischen Wasser (20 ml) und Aether (20 ml) verteilt. Der pH-Wert wird mit 40 %iger Natriumhydroxidlösung auf 9,3 eingestellt. Die Schichten werden getrennt und die Aetherschicht wird verworfen. Die wässrige Phase wird mit konz. Chlorwasserstoffsäure auf den pH-Wert 4,3 eingestellt und mit Essigsäureäthylester (3 × 25 ml) extrahiert. Die Extrakte werden über Magnesiumsulfat getrocknet und das Lösungsmittel wird unter vermindertem Druck abgedampft. Der Rückstand wird in Dichlormethan (70 ml) gelöst und Chlorwasserstoffgas 5 Minuten in die Lösung eingeleitet. Die Lösung wird eingedampft und der Rückstand aus Aethanol/Aether umkristallisiert. Man erhält das 1-Carboxymethyl-3S-(1-äthoxycarbonyl-3-phenylpropylamino)-2,3,4,5,5a,6,7,8,9,9a-decahydro-1H-[1] benzazepin-2-on-hydrochlorid als ein Isomerengemisch.

Der Ausgangsstoff wird wie folgt hergestellt: Eine Lösung von 3(S)-t-butyloxycarbonylamino-1-äthoxycarbonylmethyl-2,3,4,5-tetrahydro-1H-[1] benzazepin 2,5-dion (3,6 g) in Essigsäure (50 ml) wird 120 Stunden bei einem Druck von 3-Atmosphären über Platinoxid (1,2 g) als Katalysator hydriert. Der Katalysator wird abfiltriert und das Filtrat unter vermindertem Druck eingedampft. Der Rückstand wird zwischen Dichlormethan (200 ml) und gesättigter wässeriger Natriumhydrogencarbonatlösung (100 ml) verteilt. Die Dichlormethanlösung wird mit Wasser (50 ml) gewaschen, über Natriumsulfat getrocknet und das Lösungsmittel unter vermindertem Druck abgedampft. Der Rückstand wird auf Silicagel chromatographiert und mit 0-50 % Essigsäureäthylester in Toluol eluiert. Die mit 50 %igem Essigsäureäthylester in Toluol eluierte Fraktion wird gesammelt. Man erhält das 3(S)-t-Butyloxycarbonylamino-1-äthoxycarbonylmethyl-2,3,4,5,5a,6,7,8,9,9a-decahydro-1H-[1] benzazepin-2,5-dion, welches ohne weitere Reinigung im nächsten Syntheseschritt verwendet wird.

Eine Lösung von 3(S)-t-Butyloxycarbonylamino-1-äthoxycarbonylmethyl-2,3,4,5,5a,6,7,8,9,9a-decahydro-1H-[1] benzazepin-2,5-dion (2,7 g) und Natriumborhydrid (0,2 g) in Aethanol (100 ml) wird 18 Stunden bei Raumtemperatur gerührt. Das Lösungsmittel wird unter vermindertem Druck abgedampft und der Rückstand in Dichlormethan (100 ml) gelöst. Die Lösung wird mit eiskalter 2-normaler Chlorwasserstoffsäure (2 × 50 ml) und mit gesättigter wässeriger Natriumchloridlösung (50 ml) extrahiert und über Natriumsulfat getrocknet. Das Lösungsmittel wird unter vermindertem Druck abgedampft und der Rückstand mit Aether trituriert. Man erhält das 3(S)-t-Butyloxycarbonylamino-1-äthoxycarbonylmethyl-5-hydroxy-2,3,4,5,5a,6,7,8,9,9a-decahydro-1H-[1] benzazepin-2-on.

Ein Gemisch von 3(S)-t-Butyloxycarbonylamino-1-äthoxycarbonylmethyl-5-hydroxy-2,3,4,5,5a,6,7,8,9,9a-decahydro-1H-[1] benzazepin-2-on (2,1 g), Dicyclohexylcarbodiimid (1,8 g) und Kupfer-(I)-chlorid (0,2 g) wird unter Stickstoff 32 Stunden bei 80° erhitzt. Das Reaktionsgemisch wird auf Raumtemperatur gekühlt, der Rückstand in Methylenchlorid (200 ml) gelöst, mit verdünnter Ammoniumhydroxidlösung (2 × 50 ml) und Wasser (50 ml) gewaschen. Die organische Phase wird über Natriumsulfat getrocknet und eingedampft. Man erhält ein Gemisch des gewünschten Addukts und des überschüssigen Dicyclohexylcarbodiimids. Dieses Gemisch wird in Essigsäureäthylester (100 ml) gelöst und in ein Druckgefäss eingefüllt. Man gibt 10 %igen Pd/C-Katalysator (0,4 g) dazu und hydriert das Gemisch bei 40° und 3 Atmosphären Druck 16 Stunden. Der Katalysator wird abfiltriert und das Filtrat eingedampft. Man erhält das 3(S)-t-Butyloxycarbonylamino-1-äthoxycarbonylmethyl-2,3,4,5,5a,6,7,8,9,9a-decahydro-1H-[1] benzazepin-2-on, welches ohne weitere Reinigung im nächsten Syntheseschritt verwendet wird.

Man löst das obige Produkt (1,1 g) in Essigsäureäthylester (50 ml) und leitet in die Lösung 45 Minuten Chlorwasserstoffgas ein. Das Reaktionsgemisch wird unter vermindertem Druck eingedampft, der Rückstand in Essigsäureäthylester (50 ml) gelöst und mit Wasser (3 × 30 ml) gewaschen. Die Essigsäureäthylester-Lösung wird über Natriumsulfat getrocknet und das Lösungsmittel unter vermindertem Druck abgedampft. Man erhält das 3(S)-Amino-1-äthoxycarbonylmethyl-2,3,4,5,5a,6,7,8,9,9a-decahydro-1H-[1] benzazepin-2-on, welches ohne weitere Reinigung im nächsten Verfahrensschritt verwendet wird.

Eine Lösung von Natriumhydroxid (0,1 g) in Wasser (0,25 ml) wird zu einer Lösung des obigen Amins (0,6 g) in Methanol (7,5 ml) bei Raumtemperatur gegeben und die Lösung 2 Stunden gerührt. Die Lösungsmittel werden abgedampft, der Rückstand sorgfältig getrocknet und in Aether aufgeschlämmt. Man erhält das Natriumsalz des 3(S)-Amino-1-carboxymethyl-2,3,4,5,5a,6,7,8,9,9a-decahydro-1H-[1] benzazepin-2-ons.

Beispiel 34

N-[1-(1-Carboxymethyl-2,3,4,5-tetrahydro-2-oxo-1H-[1] benzazepin-3S-ylamino)-3-phenylpropyl-1-carbonyl]-L-phenylalanin

L-Phenylalanin-methylester-hydrochlorid wird mit 1-Benzyloxycarbonylmethyl-3S-(1S-carboxy-3-phenylpropylamino)-2,3,4,5-tetrahydro-1H-[1] benzazepin-2-on in Methylenchlorid in Gegenwart von 1-(3-Dimethyl-aminopropyl)-3-äthyl-carbodiimid-hydrochlorid bei Raumtemperatur kondensiert. Nach Aufarbeitung erhält man das N-[1-(1-Benzyloxycarbonylmethyl-1,2,3,4,5-tetrahydro-2-oxo-1H-[1] benzazepin-3S-ylamino)-3-phenylpropyl-1-carbonyl]-L-phenylalanin-methylester.

Hydrierung mit 10 %igem Pd/C-Katalysator in Aethanol ergibt das N-[1-(1-Carboxymethyl-2,3,4,5-tetrahydro-2-oxo-1H-[1] benzazepin-3S-ylamino)-3-phenylpropyl-1-carbonyl]-L-phenylalanin-methylester.

Die Hydrolyse von 18 Stunden mit verdünnter Natriumhydroxidlösung bei Raumtemperatur ergibt das N-[1-(1-Carboxymethyl-2,3,4,5-tetrahydro-2-oxo-1H-[1] benzazepin-3S-ylamino)-3-phenylpropyl-1-carbonyl]-L-phenylalanin.

Beispiel 35

1-Aethoxycarbonylmethyl-3-(1-äthoxycarbonyl-3-phenylpropylamino)-2,3,4,5-tetrahydro-1H-[1] benzazepin-2-on

Behandlung von 3-(1-Carboxy-3-phenylpropylamino)-1-cyanmethyl-2,3,4,5-tetrahydro-1H-[1] benzazepin-2-on mit einem Gemisch von Aethanol/Aether (1 : 1), welches mit Chlorwasserstoff gesättigt ist, 48 Stunden bei Raumtemperatur, ergibt nach Aufarbeitung das 1-Aethoxycarbonylmethyl-3-(1-äthoxycarbonyl-3-phenylpropylamino)-2,3,4,5-tetrahydro-1H-[1] benzazepin-2-on, welches mit der Verbindung des Beispiels 10 identisch ist.

Der Ausgangsstoff wird wie folgt hergestellt : 3-(1-Carboxy-3-phenylpropylamino)-2,3,4,5-tetrahydro-1H-[1] benzazepin-2-on wird mit Bromacetonitril in Dimethylformamid in Gegenwart von Natriumhydrid alkyliert. Nach Aufarbeitung erhält man das 3-(1-Carboxy-3-phenylpropylamino)-1-cyanmethyl-2,3,4,5-tetrahydro-1H-[1] benzazepin-2-on, welches im nächsten Schritt direkt verwendet wird.

Beispiel 36

Herstellung von 10 000 Tabletten mit einem Gehalt von je 10 mg der aktiven Substanz des Beispiels 12 :

| | |
|---|---|
| Bestandteile : | |
| 1-Carboxymethyl-3S-(1S-äthoxycarbonyl-3-phenylpropylamino)-2,3,4,5-tetrahydro-1H-[1] benzazepin-2-on | 100 g |
| Milchzucker | 1 157 g |
| Maisstärke | 75 g |
| Polyäthylenglykol 6000 | 75 g |
| Talkpulver | 75 g |
| Magnesiumstearat | 18 g |
| Gereinigtes Wasser | q.s. |

Verfahren : Gleich wie im Beispiel 15.

Beispiel 37

Herstellung von 10 000 Kapseln mit einem Gehalt von je 20 mg des Hydrochloridsalzes der aktiven Substanz des Beispiels 12 :

| | |
|---|---|
| Bestandteile : | |
| 1-Carboxymethyl-3S-(1S-äthoxycarbonyl-3-phenylpropylamino)-2,3,4,5-tetrahydro-1H-[1] benzazepin-2-on-hydrochlorid | 200 g |
| Milchzucker | 1 700 g |
| Talkpulver | 100 g |

Verfahren : Gleich wie im Beispiel 17.

Kardiovaskulare Pharmakologie von Verbindungen der Erfindung

Die Prüfung der Wirkung von Verbindungen ist gemäss den Methoden für die Auswertung der Hemmung des Enzyms, welches das Angiotensin umwandelt [angiotensin converting enzyme = ACE] durchgeführt worden. In der biochemischen Beurteilung der in vitro ACE-Hemmung [ACE inhibition = ACEI] wird die Aktivität einer Verbindung im Kaninchen-Lungengewebe bestimmt.

In der in vivo Prüfungsmethode wird zuerst durch Verabreichung des Angiotensins I (AI) an das

Versuchstier eine Blutdrucksteigerung hervorgerufen. Es wird dann die Hemmung der einzelnen Verbindungen auf diese Blutdrucksteigerung gemessen.

I. Biochemische Testmethode

Es wurde ein Kaninchen-Lungengewebe-Präparat [Das and Saffer, J. Biol. Chem. *250* : 6762 (1975) für die Bestimmung von ACE nach der Methode von Cheung und Cushman [Cheung and Cushman, Biochim. Biophys. Acta *293*, 451 (1973)] verwendet. In dieser Methode wird die Menge des Histidyl-leucins, welche aus einem synthetischen Substrat innerhalb einer Inkubation von 30 Minuten bei 37° freigesetzt wird, spektrophotometrisch bestimmt. Die $IC_{50}$-Werte der ACE-Hemmung werden dann graphisch berechnet. Der $IC_{50}$-Wert ist die Konzentration (in Mol) der getesteten Verbindung, welche die in Abwesenheit der Testsubstanz entstehende Menge des Histidyl-leucins um 50 % herabsetzt.

II. Methode der Hemmung der durch Angiotensin I (AI) hervorgerufenen Blutdrucksteigerung, durch intravenöse Verabreichung der Testverbindung (% AI-Hemmung)

In diesen Untersuchungen wird wie oben beschrieben, je ein Katheter in eine femurale Arterie und in eine Wadenvene der Ratte eingeführt.

Der arterielle Druck wurde von dem Arterienkatheter aus registriert, während man Angiotensin I (AI) und die einzelnen Testverbindungen durch den Venenkatheter injiziert hatte. Die Hemmung der durch Angiotensin I verursachten Blutdrucksteigerung ist als prozentuelle Abnahme des vor der Behandlung erhaltenen Kontrollwertes der Blutdrucksteigerung ausgedrückt und in der Tabelle als Durchschnittswert der Hemmung innerhalb 30 Minuten nach Verabreichung der Testsubstanz angegeben.

(Siehe Tabelle Seite 41 f.)

Ergebnisse :

| Verbindung des Beispiels | In vitro ACEI $IC_{50}$ (M) | Hemmung der durch Angiotensin I (AI) verursachten Blutdrucksteigerung | |
|---|---|---|---|
| | | i.v. Dosis (mg/kg) | % AI Hemmung |
| 1 | $6 \times 10^{-7}$ | 10 | 100 |
| | | 1.0 | 100 |
| | | 0.1 | 50 |
| 5 | $2 \times 10^{-7}$ | 0.1 | 37 |
| 9 | $5 \times 10^{-9}$ | 0.3 | 93 |
| | | 0.1 | 80 |
| | | 0.03 | 40 |
| 10 Maleat Salz | $1 \times 10^{-5}$ | 1.0 | 80 |
| 12 HCl Salz | $4 \times 10^{-7}$ | 1.0 | 100 |
| | | 0.3 | 95 |
| | | 0.1 | 82 |
| | | 0.06 | 74 |
| | | 0.03 | 29 |
| 19 | $2 \times 10^{-9}$ | 0.1 | 93 |
| | | 0.06 | 84 |
| | | 0.03 | 70 |
| | | 0.02 | 69 |
| | | 0.01 | 28 |
| | | 0.007 | 14 |
| 28 Isomer B, HCl Salz | $1 \times 10^{-7}$ | 0.1 | 92 |

**Patentansprüche** (für die Vertragsstaaten : BE, CH, DE, FR, IT, LI, LU, NL, SE)

1. Verbindungen der Formel I

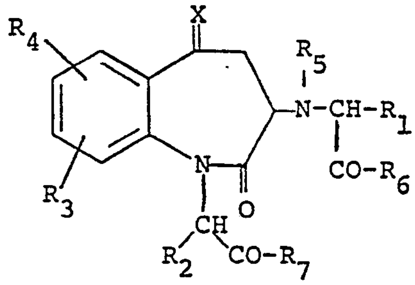

(I)

worin

$R_1$ Wasserstoff, $C_{1-7}$-Alkyl, Amino-$C_{1-7}$-alkyl, Aryl, durch $C_{1-7}$-Alkyl, $C_{1-7}$-Alkoxy, $C_{1-7}$-Alkylendioxy, $C_{1-7}$-Alkanoyloxy, Hydroxy, Halogen oder Trifluormethyl mono- oder disubstituiertes Phenyl, Aryl-$C_{1-7}$-alkyl, durch $C_{1-7}$-Alkyl, $C_{1-7}$-Alkoxy, $C_{1-7}$-Alkylendioxy, $C_{1-7}$-Alkanoyloxy, Hydroxy, Halogen oder Trifluormethyl mono- oder disubstituiertes Phenyl-$C_{1-7}$-alkyl Cycloalkyl oder Cycloalkyl-$C_{1-7}$-alkyl bedeutet ;

$R_2$ Wasserstoff oder $C_{1-7}$-Alkyl ist ;

$R_3$ und $R_4$ unabhängig Wasserstoff, $C_{1-7}$-Alkyl, $C_{1-7}$-Alkoxy, $C_{1-7}$-Alkanoyloxy, Hydroxy, Halogen oder Trifluormethyl darstellen oder $R_3$ und $R_4$ gemeinsam $C_{1-7}$-Alkylendioxy bedeuten ;

$R_5$ Wasserstoff oder $C_{1-7}$-Alkyl ist,

$R_6$ und $R_7$ unabhängig voneinander Hydroxy, $C_{1-7}$-Alkoxy ; ω-(Amino, Mono- oder Di-$C_{1-7}$-alkylamino)-substituiertes $C_{2-4}$-Alkoxy ; Carboxy-substituiertes $C_{1-7}$-Alkoxy ; $C_{1-7}$-Alkoxycarbonyl-substituiertes $C_{1-7}$-Alkoxy ; Aryl-substituiertes $C_{1-7}$-Alkoxy ; durch $C_{1-7}$-Alkyl, $C_{1-7}$-Alkoxy, $C_{1-7}$-Alkylendioxy, $C_{1-7}$-Alkanoyloxy, Hydroxy, Halogen oder Trifluormethyl mono- oder disubstituiertes Phenyl-$C_{1-7}$-alkoxy ; (Hydroxy, $C_{1-7}$-Alkanoyloxy oder $C_{1-7}$-Alkoxy)-substituiertes $C_{1-7}$-Alkoxy, (Hydroxy, $C_{1-7}$-Alkanoyloxy oder $C_{1-7}$-Alkoxy)-substituiertes $C_{1-7}$-Alkoxymethoxy ; Bicycloalkoxycarbonyl-substituiertes $C_{1-7}$-Alkoxy ; 3-Phthalidoxy ; ($C_{1-7}$-Alkyl, $C_{1-7}$-Alkoxy, Halo)-substituiertes 3-Phthalidoxy ; Amino, $C_{1-7}$-Alkylamino ; Di-$C_{1-7}$-alkylamino ; Di-$C_{1-7}$-Alkylamino, worin beide Alkylgruppen durch eine Kohlenstoff-Kohlenstoff-Bindung verbunden sind und gemeinsam mit dem Aminostickstoff einen 5-, 6- oder 7-gliedrigen heterocyclischen Ring bilden ; α-(Carboxy oder $C_{1-7}$-Alkoxycarbonyl)-substituiertes $C_{1-7}$-Alkylamino ; Aryl-substituiertes $C_{1-7}$-Alkylamino oder durch $C_{1-7}$-Alkyl, $C_{1-7}$-Alkoxy, $C_{1-7}$-Alkylendioxy, $C_{1-7}$-Alkanoyloxy, Hydroxy, Halogen oder Trifluormethyl mono- oder disubstituiertes Phenyl-$C_{1-7}$-alkylamino, welches jeweils an dem α-Kohlenstoffatom durch Carboxy oder $C_{1-7}$-Alkoxycarbonyl substituiert sein kann, bedeuten ;

und X Oxo, zwei Wasserstoffatome oder ein Wasserstoffatom und eine Hydroxygruppe bedeutet ; und worin der carbocyclische Ring auch Hexahydro oder 6,7,8,9-Tetrahydro sein kann ; deren Stereoisomere und Salze.

2. Verbindungen der Formel I gemäss Anspruch 1, worin $R_2$, $R_3$, $R_4$, $R_5$ und X die in Anspruch 1 gegebenen Bedeutungen haben,

$R_1$ Wasserstoff, $C_{1-7}$-Alkyl, Amino-$C_{1-7}$-alkyl, Aryl, Aryl-$C_{1-7}$-alkyl, Cycloalkyl-$C_{1-7}$-alkyl oder durch $C_{1-7}$-Alkyl, $C_{1-7}$-Alkoxy, $C_{1-7}$-Alkylendioxy, $C_{1-7}$-Alkanoyloxy, Hydroxy, Halogen oder Trifluormethyl mono- oder disubstituiertes Phenyl oder Phenyl-$C_{1-7}$-alkyl bedeutet, und

$R_6$ und $R_7$ unabhängig voneinander Hydroxy, Amino, Mono- oder Di-$C_{1-7}$-alkylamino, $C_{1-7}$-Alkoxy, Aryl-$C_{1-7}$-alkoxy, $C_{1-7}$-Alkanoyloxymethoxy, ω-(Amino, Mono- oder Di-$C_{1-7}$-alkylamino)-$C_{2-4}$-alkoxy, (Carboxy oder $C_{1-7}$-Alkoxy-carbonyl)-$C_{1-7}$-alkoxy bedeuten, oder deren Salze.

3. Verbindungen der Formel I gemäss Anspruch 1, worin $R_2$, $R_5$ und X die in Anspruch 1 gegebenen Bedeutungen aufweisen,

$R_1$ Wasserstoff, $C_{1-7}$-Alkyl, Amino-$C_{1-7}$-alkyl, Phenyl-$C_{1-7}$-alkyl, oder im Phenylteil durch $C_{1-7}$-Alkyl, Hydroxy, $C_{1-7}$-Alkoxy, $C_{1-7}$-Alkylendioxy, $C_{1-7}$-Alkanoyloxy, Halogen oder Trifluormethyl mono- oder disubstituiertes Phenyl-$C_{1-7}$-alkyl bedeutet, ist,

$R_3$ und $R_4$ Wasserstoff, $C_{1-7}$-Alkoxy, $C_{1-7}$-Alkyl, Halogen oder Trifluormethyl bedeuten, oder $R_3$ und $R_4$ gemeinsam $C_1$-$C_7$-Alkylendioxy bedeuten,

$R_6$ und $R_7$ unabhängig voneinander Hydroxy, Amino, $C_{1-7}$-Alkoxy, Phenyl-$C_{1-7}$-alkoxy, $C_{1-7}$-Alkoxycarbonyl-$C_{1-7}$-alkoxy bedeuten, oder deren Salze.

4. Verbindungen der Formel IA

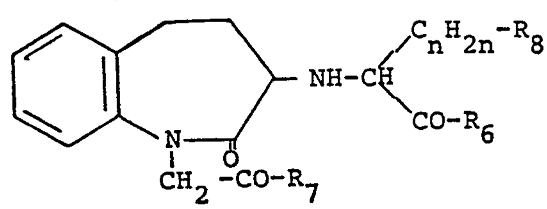

(IA)

worin n eine ganze Zahl von 1 bis 4 bedeutet, $R_8$ Wasserstoff, unsubstituiertes Phenyl oder durch $C_{1-7}$-Alkyl, $C_{1-7}$-Alkoxy, $C_{1-7}$-Alkanoyloxy, Halogen, Hydroxy oder Trifluormethyl monosubstituiertes Phenyl, bedeutet,
sowie $R_6$ und $R_7$ unabhängig voneinander Hydroxy, $C_{1-4}$-Alkoxy, Benzyloxy oder Amino bedeuten, oder deren pharmazeutisch verträgliche Salze.

5. Verbindungen der Formel IB

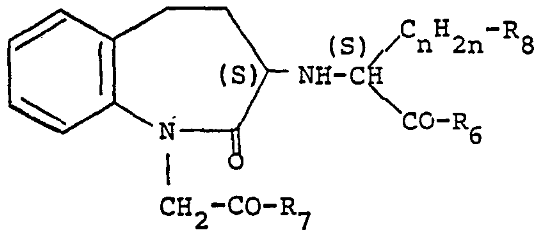

(IB)

worin (S) die Chiralität bedeutet, sowie n, $R_6$, $R_7$ und $R_8$ die in Anspruch 4 angebene Bedeutung haben, oder deren pharmazeutisch verträgliche Salze.

6. 1-Carboxymethyl-3-(1-äthoxycarbonyl-3-phenylpropylamino)-2,3,4,5-tetrahydro-1H-[1] benzazepin-2-on, seine Stereoisomere, und Salze.

7. Die bei 138-140 °C schmelzende racemische Verbindung der im Anspruch 6 genannten Verbindung, ihre Enantiomere, und Salze.

8. Die bei 126-129 °C schmelzende racemische Verbindung der im Anspruch 6 genannten Verbindung, ihre Enantiomere, und Salze.

9. 1-Carboxymethyl-3S-(1S-äthoxycarbonyl-3-phenylpropylamino)-2,3,4,5-tetrahydro-1H-[1] benzazepin-2-on und seine Salze.

10. 1-Carboxymethyl-3-(1-carboxy-3-phenylpropylamino)-2,3,4,5-tetrahydro-1H-[1] benzazepin-2-on, seine Stereoisomere, und Salze.

11. 1-Carboxymethyl-3S-(1S-carboxy-3-phenylpropylamino)-2,3,4,5-tetrahydro-1H-[1] benzazepin-2-on und seine Salze.

12. 1-Aethoxycarbonylmethyl-3-(1-carboxy-3-phenylpropylamino)-2,3,4,5-tetrahydro-1H-[1] benzazepin-2-on, seine Stereoisomere, und Salze.

13. Das Racemat der im Anspruch 12 genannten Verbindung, das bei 175-177 °C schmilzt, und dessen Salze.

14. Das Racemat von 1-Carboxymethyl-7-chlor-3-(1-äthoxycarbonyl-3-phenylpropylamino)-2,3,4,5-tetrahydro-1H-[1] benzazepin-2-on, dessen Hydrochlorid bei 149-151 °C schmilzt, und dessen Salze.

15. 3-(1-Benzyloxycarbonyl-3-phenylpropylamino)-1-carboxymethyl-2,3,4,5-tetrahydro-1H-[1] benzazepin-2-on, seine Stereoisomere, und Salze.

16. Pharmazeutische Präparate enthaltend Verbindungen der Ansprüche 2 und 4-10 zusammen mit einem pharmazeutischen Trägermaterial.

17. Pharmazeutische Präparate enthaltend Verbindungen der Ansprüche 3 und 11-13 zusammen mit einem pharmazeutischen Trägermaterial.

18. Pharmazeutische Präparate enthaltend Verbindungen der Ansprüche 1, 14 und 15 zusammen mit einem pharmazeutischen Trägermaterial.

19. Die in den Ansprüchen 2 und 4-10 genannten Verbindungen zur Anwendung in einem Verfahren zur therapeutischen Behandlung des menschlischen oder tierischen Körpers.

20. Die in den Ansprüchen 3 und 11-13 genannten Verbindungen zur Anwendung in einem Verfahren zur therapeutischen Behandlung des menschlichen oder tierischen Körpers.

21. Die in den Ansprüchen 1, 14 und 15 genannten Verbindungen zur Anwendung in einem Verfahren zur therapeutischen Behandlung des menschlischen oder tierischen Körpers.

22. Die in den Ansprüchen 2 und 4-10 genannten Verbindungen zur Verwendung als Inhibitoren des Angiotensinumwandelnden Enzyms.

23. Die in den Ansprüchen 3 und 11-13 genannten Verbindungen zur Verwendung als Inhibitoren des Angiotensinumwandelnden Enzyms.

24. Die in den Ansprüchen 1, 14 und 15 genannten Verbindungen zur Verwendung als Inhibitoren des Angiotensinumwandelnden Enzyms.

25. Verwendung der in den Ansprüchen 2 und 4-10 genannten Verbindungen zur Herstellung von pharmazeutischen Präparaten.

26. Verwendung der in den Ansprüchen 3 und 11-13 genannten Verbindungen zur Herstellung von pharmazeutischen Präparaten.

27. Verwendung der in den Ansprüchen 1, 14 und 15 genannten Verbindungen zur Herstellung von pharmazeutischen Präparaten.

28. Verfahren zur Herstellung von den im Anspruch 1 genannten Verbindungen der Formel I, ihren Stereoisomeren und Salzen, dadurch gekennzeichnet, dass man

a) in eine Verbindung der Formel II

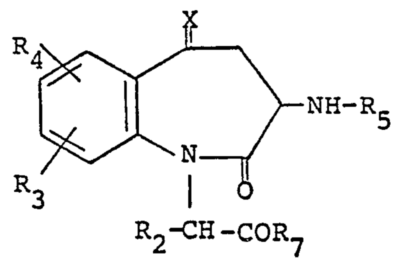

(II)

worin der carbocyclische Ring auch Hexahydro oder 6,7,8,9-Tetrahydro bedeuten kann und worin X, $R_2$, $R_3$, $R_4$, $R_5$ und $R_7$ die vorstehend angegebenen Bedeutungen besitzen, den Rest —$CH(R_1)COR_6$ durch Alkylierung mit einer Verbindung der Formel $R_1$—CH(Z)—$COR_6$ (IIIA), worin Z eine reaktive veresterte Hydroxygruppe bedeutet und $R_1$ und $R_6$ die vorstehend angegebenen Bedeutungen besitzen, oder mit einer Verbindung der Formel $R_1$—CO—$COR_6$ (IV), worin $R_1$ und $R_6$ die oben angegebenen Bedeutungen haben, in Gegenwart eines Reduktionsmittels, unter vorübergehendem Schutz etwaiger primärer und sekundärer Aminogruppen und/oder gegebenenfalls Hydroxy- und/oder Oxogruppen, die in irgendeinem der Reste X, $R_1$, $R_3$, $R_4$, $R_6$ und $R_7$ anwesend sein können, einführt oder

b) eine Verbindung der Formel V

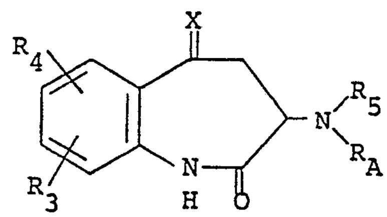

(V)

worin der carbocyclische Ring auch Hexahydro oder 6,7,8,9-Tetrahydro sein kann und worin X, $R_3$, $R_4$ und $R_5$ die vorstehend angegebenen Bedeutungen besitzen und $R_A$ Wasserstoff oder —$CH(R_1)COR_6$ wie vorstehend definiert ist, mit einer Verbindung der Formel $R_2$—CH(Z)—$COR_7$ (IIIB), worin Z eine reaktive veresterte Hydroxygruppe bedeutet und $R_2$ und $R_7$ die vorstehend angegebenen Bedeutungen besitzen, alkyliert, wobei man vorübergehend etwaige primäre und sekundäre Aminogruppen und/oder gegebenenfalls Hydroxy- und/oder Oxogruppen, die in irgendeinem der Reste X, $R_1$, $R_3$, $R_4$, $R_6$ und $R_7$ anwesend sein können, schützt oder

c) eine Verbindung der Formel VI

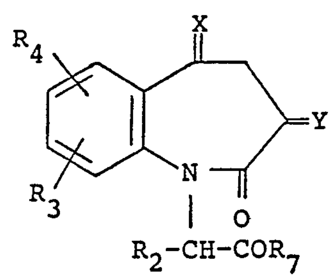

(VI)

worin der carbocyclische Ring auch Hexahydro oder 6,7,8,9-Tetrahydro sein kann und worin Y Oxo oder eine reaktive veresterte Hydroxygruppe Z und Wasserstoff bedeutet und X, $R_2$, $R_3$, $R_4$ und $R_7$ die vorstehend angegebenen Bedeutungen besitzen, mit einem Amin der Formel VII

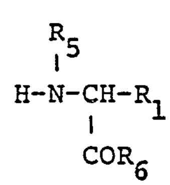

(VII)

worin $R_1$, $R_5$ und $R_6$ die vorstehend angegebenen Bedeutungen besitzen, kondensiert, wobei, wenn Y Oxo bedeutet, die Kondensation in Gegenwart eines Reduktionsmittels und unter vorübergehendem Schutz der Oxogruppe, die als Substituent X anwesend sein kann, durchgeführt wird, oder

d) in einer Verbindung der Formel VIII

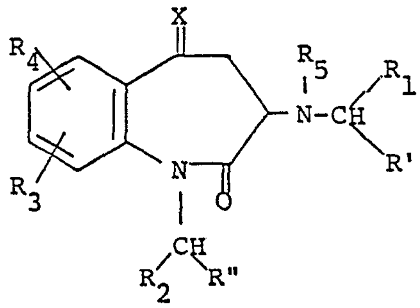

(VIII)

worin der carbocyclische Ring auch Hexahydro oder 6,7,8,9-Tetrahydro sein kann und worin X und $R_1$ bis $R_5$ die vorstehend angegebenen Bedeutungen besitzen, eines der Symbole R' und R" Cyan ist und das andere ebenfalls Cyan oder $COR_6$ bzw. $COR_7$, wie vorstehend definiert, ist, die Cyangruppe(n) einer Solvolyse unterzieht oder

e) eine Verbindung der Formel IX

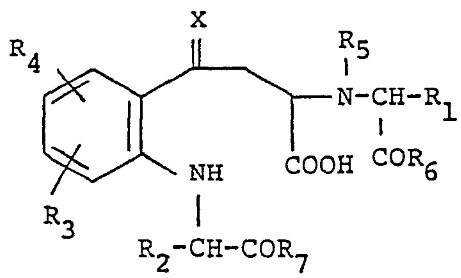

(IX)

worin der carbocyclische Ring auch Hexahydro oder 3,4,5,6-Tetrahydro sein kann und worin X und $R_1$ bis $R_7$ die vorstehend angegebenen Bedeutungen besitzen, oder einen Ester hiervon cyclisiert oder

f) eine Verbindung, die strukturell mit einer Verbindung der vorstehend angegebenen Formel I identisch ist, mit Ausnahme dessen, dass sie eine zusätzliche Doppelbindung in C-3-Stellung oder zwischen dem exocyclischen Stickstoffatom und dem benachbarten Kohlenstoffatom in der Seitenkette aufweist, mit einem Reduktionsmittel behandelt, um diese Doppelbindung zu sättigen,

und gewünschtenfalls eine erhaltene Verbindung der Formel I, wie vorstehend definiert, in eine andere Verbindung der Formel I innerhalb des vorstehend angegebenen Bereichs umwandelt und/oder gewünschtenfalls eine erhaltene Verbindung der Formel I wie vorstehend definiert, die salzbildende Eigenschaften aufweist, in ihr Salz überführt oder eine freie Verbindung aus einem derartigen Salz freisetzt und/oder gewünschtenfalls ein optisches Isomeres, das eine spezifische Konfiguration im Hinblick auf zumindest ein Chiralitätszentrum besitzt, aus einer Mischung der Stereoisomeren Formen einer erhaltenen Verbindung der Formel I anreichert.

29. Verfahren zur Herstellung von den in Anspruch 1 genannten Verbindungen der Formel I, worin X Oxo bedeutet und $R_1$ bis $R_7$ die in Anspruch 1 angegebenen Bedeutungen aufweisen, dadurch gekennzeichnet, dass man eine Verbindung der Formel X

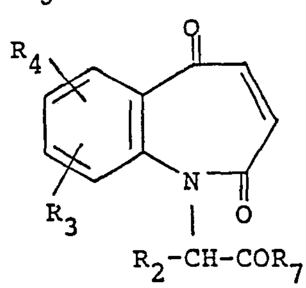

(X)

worin der carbocyclische Ring auch Hexahydro oder 6,7,8,9-Tetrahydro sein kann und worin $R_2$ bis $R_4$ und $R_7$ die vorstehend angegebenen Bedeutungen besitzen, mit einem Amin der Formel VII

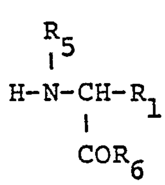

(VII)

worin $R_1$, $R_5$ und $R_6$ die vorstehend angegebenen Bedeutungen besitzen, kondensiert und gewünschtenfalls eine erhaltene Verbindung der Formel I, wie vorstehend definiert, in eine andere Verbindung der Formel I innerhalb des vorstehend angegebenen Bereichs umwandelt und/oder gewünschtenfalls eine erhaltene Verbindung der Formel I wie vorstehend definiert, die salzbildende Eigenschaften aufweist, in ihr Salz überführt oder eine freie Verbindung aus einem derartigen Salz freisetzt und/oder gewünschtenfalls ein optisches Isomeres, das eine spezifische Konfiguration im Hinblick auf zumindest ein Chiralitätszentrum besitzt, aus einer Mischung der stereoisomeren Formen einer erhaltenen Verbindung der Formel I anreichert.

30. Die nach dem Verfahren des Anspruchs 28 erhältlichen Verbindungen.

31. Die nach dem Verfahren des Anspruchs 29 erhältlichen Verbindungen.

**Patentansprüche** (für den Vertragsstaat AT)

1. Verfahren zur Herstellung von neuen 3-Amino-[1] benzazepin-2-on-1-alkansäuren und deren Derivaten der allgemeinen Formel I

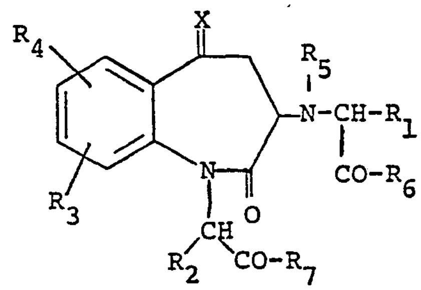

(I)

worin

$R_1$ Wasserstoff, $C_{1-7}$-Alkyl, Amino-$C_{1-7}$-alkyl, Aryl, durch $C_{1-7}$-Alkyl, $C_{1-7}$-Alkoxy, $C_{1-7}$-Alkylendioxy, $C_{1-7}$-Alkanoyloxy, Hydroxy, Halogen oder Trifluormethyl mono- oder disubstituiertes Phenyl, Aryl-$C_{1-7}$-alkyl, durch $C_{1-7}$-Alkyl, $C_{1-7}$-Alkoxy, $C_{1-7}$-Alkylendioxy, $C_{1-7}$-Alkanoyloxy, Hydroxy, Halogen oder Trifluormethyl mono- oder disubstituiertes Phenyl-$C_{1-7}$-alkyl Cycloalkyl oder Cycloalkyl-$C_{1-7}$-alkyl bedeutet ;

$R_2$ Wasserstoff oder $C_{1-7}$-Alkyl ist ;

$R_3$ und $R_4$ unabhängig Wasserstoff, $C_{1-7}$-Alkyl, $C_{1-7}$-Alkoxy, $C_{1-7}$-Alkanoyloxy, Hydroxy, Halogen oder Trifluormethyl darstellen oder $R_3$ und $R_4$ gemeinsam $C_{1-7}$-Alkylendioxy bedeuten ;

$R_5$ Wasserstoff oder $C_{1-7}$-Alkyl ist,

$R_6$ und $R_7$ unabhängig voneinander Hydroxy, $C_{1-7}$-Alkoxy ; ω-(Amino, Mono- oder Di-$C_{1-7}$-alkylamino)-substituiertes $C_{2-4}$-Alkoxy ; Carboxy-substituiertes $C_{1-7}$-Alkoxy ; $C_{1-7}$-Alkoxycarbonyl-substituiertes $C_{1-7}$-Alkoxy ; Aryl-substituiertes $C_{1-7}$-Alkoxy ; durch $C_{1-7}$-Alkyl, $C_{1-7}$-Alkoxy, $C_{1-7}$-Alkylendioxy, $C_{1-7}$-Alkanoyloxy, Hydroxy, Halogen oder Trifluormethyl mono- oder disubstituiertes Phenyl-$C_{1-7}$-Alkoxy ; (Hydroxy, $C_{1-7}$-Alkanoyloxy oder $C_{1-7}$-Alkoxy)-substituiertes $C_{1-7}$-Alkoxy, (Hydroxy, $C_{1-7}$-Alkanoyloxy oder $C_{1-7}$-Alkoxy)-substituiertes $C_{1-7}$-Alkoxymethoxy ; Bicycloalkoxycarbonyl-substituiertes $C_{1-7}$-Alkoxy ; 3-Phthalidoxy ; ($C_{1-7}$-Alkyl, $C_{1-7}$-Alkoxy, Halo)-substituiertes 3-Phthalidoxy ; Amino, $C_{1-7}$-Alkylamino ; Di-$C_{1-7}$-alkylamino ; Di-$C_{1-7}$-Alkylamino, worin beide Alkylgruppen durch eine Kohlenstoff-Kohlenstoff-Bindung verbunden sind und gemeinsam mit dem Aminostickstoff einen 5-, 6- oder 7-gliedrigen heterocyclischen Ring bilden ; α-(Carboxy oder $C_{1-7}$-Alkoxycarbonyl)-substituiertes $C_{1-7}$-Alkylamino ; Aryl-substituiertes $C_{1-7}$-Alkylamino oder durch $C_{1-7}$-Alkyl, $C_{1-7}$-Alkoxy, $C_{1-7}$-Alkylendioxy, $C_{1-7}$-Alkanoyloxy, Hydroxy, Halogen oder Trifluormethyl mono- oder disubstituiertes Phenyl-$C_{1-7}$-alkylamino, welches jeweils an dem α-Kohlenstoffatom durch Carboxy oder $C_{1-7}$-Alkoxycarbonyl substituiert sein kann, bedeuten ;

und X Oxo, zwei Wasserstoffatome oder ein Wasserstoffatom und eine Hydroxygruppe bedeutet ;

und worin der carbocyclische Ring auch Hexahydro oder 6,7,8,9-Tetrahydro sein kann ; deren Stereoisomeren und Salzen, dadurch gekennzeichnet, dass man

a) in eine Verbindung der Formel II

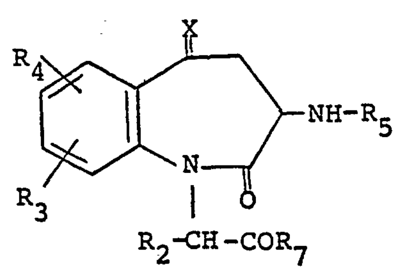

(II)

worin der carbocyclische Ring auch Hexahydro oder 6,7,8,9-Tetrahydro bedeuten kann und worin X, $R_2$, $R_3$, $R_4$, $R_5$ und $R_7$ die vorstehend angegebenen Bedeutungen besitzen, den Rest $—CH(R_1)COR_6$ durch Alkylierung mit einer Verbindung der Formel $R_1—CH(Z)—COR_6$ (IIIA), worin Z eine reaktive veresterte Hydroxygruppe bedeutet und $R_1$ und $R_6$ die vorstehend angegebenen Bedeutungen besitzen, oder mit einer Verbindung der Formel $R_1—CO—COR_6$ (IV), worin $R_1$ und $R_6$ die oben angegebenen Bedeutungen haben, in Gegenwart eines Reduktionsmittels, unter vorübergehendem Schutz etwaiger primärer und sekundärer Aminogruppen und/oder gegebenenfalls Hydroxy- und/oder Oxogruppen, die in irgendeinem der Reste X, $R_1$, $R_3$, $R_4$, $R_6$ und $R_7$ anwesend sein können, einführt oder

b) eine Verbindung der Formel V

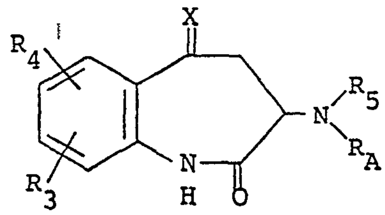
(V)

worin der carbocyclische Ring auch Hexahydro oder 6,7,8,9-Tetrahydro sein kann und worin X, $R_3$, $R_4$ und $R_5$ die vorstehend angegebenen Bedeutungen besitzen und $R_A$ Wasserstoff oder $—CH(R_1)COR_6$ wie vorstehend definiert ist, mit einer Verbindung der Formel $R_2—CH(Z)—COR_7$ (IIIB), worin Z eine reaktive veresterte Hydroxygruppe bedeutet und $R_2$ und $R_7$ die vorstehend angegebenen Bedeutungen besitzen, alkyliert, wobei man vorübergehend etwaige primäre und sekundäre Aminogruppen und/oder gegebenenfalls Hydroxy- und/oder Oxogruppen, die in irgendeinem der Reste X, $R_1$, $R_3$, $R_4$, $R_6$ und $R_7$ anwesend sein können, schützt oder

c) eine Verbindung der Formel VI

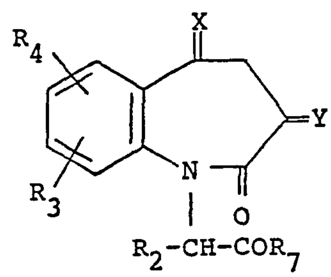
(VI)

worin der carbocyclische Ring auch Hexahydro oder 6,7,8,9-Tetrahydro sein kann und worin Y Oxo oder eine reaktive veresterte Hydroxygruppe Z gemeinsam mit Wasserstoff bedeutet und X, $R_2$, $R_3$, $R_4$ und $R_7$ die vorstehend angegebenen Bedeutungen besitzen, mit einem Amin der Formel VII

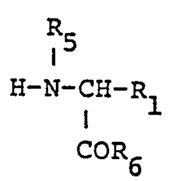
(VII)

worin $R_1$, $R_5$ und $R_6$ die vorstehend angegebenen Bedeutungen besitzen, kondensiert, wobei, wenn Y Oxo bedeutet, die Kondensation in Gegenwart eines Reduktionsmittels und unter vorübergehendem Schutz der Oxogruppe, die als Substituent X anwesend sein kann, durchgeführt wird, oder

d) in einer Verbindung der Formel VIII

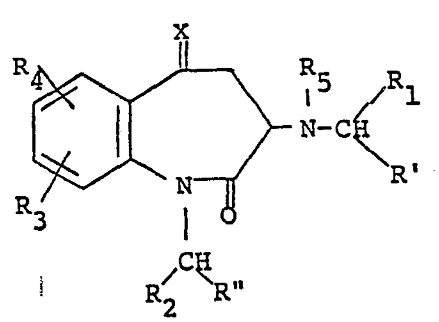
(VIII)

worin der carbocyclische Ring auch Hexahydro oder 6,7,8,9-Tetrahydro sein kann und worin X und $R_1$ bis $R_5$ die vorstehend angegebenen Bedeutungen besitzen, eines der Symbole R′ und R″ Cyan ist und das andere ebenfalls Cyan oder $COR_6$ bzw. $COR_7$, wie vorstehend definiert, ist, die Cyangruppe(n) einer Solvolyse unterzieht oder

e) eine Verbindung der Formel IX

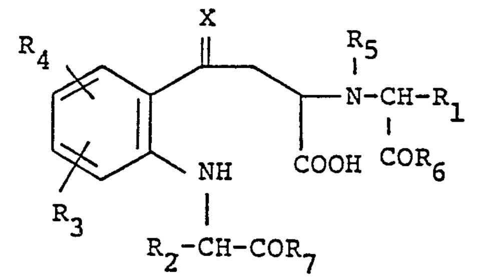

(IX)

worin der carbocyclische Ring auch Hexahydro oder 3,4,5,6-Tetrahydro sein kann und worin X und $R_1$ bis $R_7$ die vorstehend angegebenen Bedeutungen besitzen, oder einen Ester hiervon cyclisiert oder

f) eine Verbindung, die strukturell mit einer Verbindung der vorstehend angegebenen Formel I identisch ist, mit Ausnahme dessen, dass sie eine zusätzliche Doppelbindung in C-3-Stellung oder zwischen dem exocyclischen Stickstoffatom und dem benachbarten Kohlenstoffatom in der Seitenkette aufweist, mit einem Reduktionsmittel behandelt, um diese Doppelbindung zu sättigen,

und gewünschtenfalls eine erhaltene Verbindung der Formel I, wie vorstehend definiert, in eine andere Verbindung der Formel I innerhalb des vorstehend angegebenen Bereichs umwandelt und/oder gewünschtenfalls eine erhaltene Verbindung der Formel I wie vorstehend definiert, die salzbildende Eigenschaften aufweist, in ihr Salz überführt oder eine freie Verbindung aus einem derartigen Salz freisetzt und/oder gewünschtenfalls ein optisches Isomeres, das eine spezifische Konfiguration im Hinblick auf zumindest ein Chiralitätszentrum besitzt, aus einer Mischung der stereoisomeren Formen einer erhaltenen Verbindung der Formel I anreichert.

2. Verfahren nach Anspruch 1, dadurch gekennzeichnet, dass man Verbindungen der Formel I, worin $R_2$, $R_3$, $R_4$, $R_5$ und X die in Anspruch 1 gegebenen Bedeutungen haben,

$R_1$ Wasserstoff, $C_{1-7}$-Alkyl, Amino-$C_{1-7}$-alkyl, Aryl, Aryl-$C_{1-7}$-alkyl, Cycloalkyl-$C_{1-7}$-alkyl oder durch $C_{1-7}$-Alkyl, $C_{1-7}$-Alkoxy, $C_{1-7}$-Alkylendioxy, $C_{1-7}$-Alkanoyloxy, Hydroxy, Halogen oder Trifluormethyl mono- oder disubstituiertes Phenyl oder Phenyl-$C_{1-7}$-alkyl bedeutet, und

$R_6$ und $R_7$ unabhängig voneinander Hydroxy, Amino, Mono- oder Di-$C_{1-7}$-alkylamino, $C_{1-7}$-Alkoxy, Aryl-$C_{1-7}$-alkoxy, durch $C_{1-7}$-Alkyl, $C_{1-7}$-Alkoxy, $C_{1-7}$-Alkylendioxy, $C_{1-7}$-Alkanoyloxy, Hydroxy, Halogen oder Trifluormethyl mono- oder disubstituiertes Phenyl-$C_{1-7}$-alkoxy, $C_{1-7}$-Alkanoyloxymethoxy, ω-(Amino, Mono- oder Di-$C_{1-7}$-alkylamino)-$C_{2-4}$-alkoxy, (Carboxy oder $C_{1-7}$-Alkoxycarbonyl)-$C_{1-7}$-alkoxy bedeuten, deren Salze herstellt.

3. Verfahren nach Anspruch 1, dadurch gekennzeichnet, dass man Verbindungen der Formel I, worin $R_2$, $R_5$ und X die in Anspruch 1 gegebenen Bedeutungen aufweisen,

$R_1$ Wasserstoff, $C_{1-7}$-Alkyl, Amino-$C_{1-7}$-alkyl, Phenyl-$C_{1-7}$-alkyl, oder im Phenylteil durch $C_{1-7}$-Alkyl, Hydroxy, $C_{1-7}$-Alkoxy, $C_{1-7}$-Alkylendioxy, $C_{1-7}$-Alkanoyloxy, Halogen oder Trifluormethyl mono- oder disubstituiertes Phenyl-$C_{1-7}$-alkyl bedeutet, ist,

$R_3$ und $R_4$ Wasserstoff, $C_{1-7}$-Alkoxy, $C_{1-7}$-Alkyl, Halogen oder Trifluormethyl bedeuten, oder $R_3$ und $R_4$ gemeinsam $C_{1-7}$-Alkylendioxy bedeuten,

$R_6$ und $R_7$ unabhängig voneinander Hydroxy, Amino, $C_{1-7}$-Alkoxy, Phenyl-$C_{1-7}$-alkoxy, $C_{1-7}$-Alkoxycarbonyl-$C_{1-7}$-alkoxy bedeuten, oder deren Salze herstellt.

4. Verfahren nach Anspruch 1, dadurch gekennzeichnet, dass man Verbindungen der Formel IA

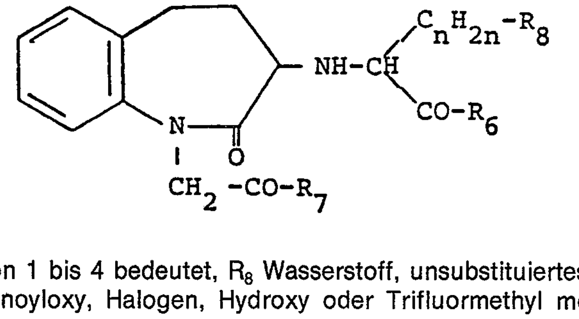

(IA)

worin n eine ganze Zahl von 1 bis 4 bedeutet, $R_8$ Wasserstoff, unsubstituiertes Phenyl oder durch $C_{1-7}$-Alkyl, $C_{1-7}$-Alkoxy, $C_{1-7}$-Alkanoyloxy, Halogen, Hydroxy oder Trifluormethyl monosubstituiertes Phenyl, bedeutet,

sowie $R_6$ und $R_7$ unabhängig voneinander Hydroxy, $C_{1-4}$-Alkoxy, Benzyloxy oder Amino bedeuten, oder deren pharmazeutisch verträgliche Salze herstellt.

5. Verfahren nach Anspruch 1, dadurch gekennzeichnet, dass man 1-Carboxymethyl-3-(1-äthoxycarbonyl-3-phenylpropylamino)-2,3,4,5-tetrahydro-1H-[1] benzazepin-2-on, seine Stereoisomere und Salze herstellt.

6. Verfahren nach Anspruch 1, dadurch gekennzeichnet, dass man die bei 138-140 °C schmelzende racemische Verbindung der im Anspruch 5 genannten Verbindung, ihre Enantiomere und Salze herstellt.

7. Verfahren nach Anspruch 1, dadurch gekennzeichnet, dass man die bei 126-129 °C schmelzende racemische Verbindung der im Anspruch 5 genannten Verbindung, ihre Enantiomere und Salze herstellt.

8. Verfahren nach Anspruch 1, dadurch gekennzeichnet, dass man 1-Carboxymethyl-3S-(1S-äthoxycarbonyl-3-phenylpropylamino)-2,3,4,5-tetrahydro-1H-[1] benzazepin-2-on und seine Salze herstellt.

9. Verfahren zur Herstellung von den in Anspruch 1 genannten Verbindungen der Formel I, worin X Oxo bedeutet und $R_1$ bis $R_7$ die in Anspruch 1 angegebenen Bedeutungen aufweisen, dadurch gekennzeichnet, dass man eine Verbindung der Formel X

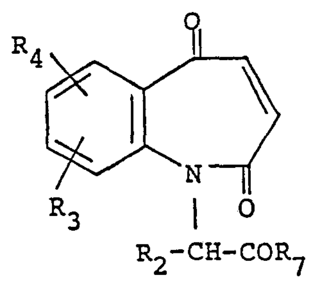

(X)

worin der carbocyclische Ring auch Hexahydro oder 6,7,8,9-Tetrahydro sein kann und worin $R_2$ bis $R_4$ und $R_7$ die vorstehend angegebenen Bedeutungen besitzen, mit einem Amin der Formel VII

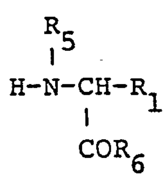

(VII)

worin $R_1$, $R_5$ und $R_6$ die vorstehend angegebenen Bedeutungen besitzen, kondensiert und gewünschtenfalls eine erhaltene Verbindung der Formel I, wie vorstehend definiert, in eine andere Verbindung der Formel I innerhalb des vorstehend angegebenen Bereichs umwandelt und/oder gewünschtenfalls eine erhaltene Verbindung der Formel I wie vorstehend definiert, die salzbildende Eigenschaften aufweist, in ihr Salz überführt oder eine freie Verbindung aus einem derartigen Salz freisetzt und/oder gewünschtenfalls ein optisches Isomeres, das eine spezifische Konfiguration im Hinblick auf zumindest ein Chiralitätszentrum besitzt, aus einer Mischung der stereoisomeren Formen einer erhaltenen Verbindung der Formel I anreichert.

10. Verfahren zur Herstellung eines pharmazeutischen Präparates, gekennzeichnet durch die Verarbeitung eines erfindungsgemässen Wirkstoffes nach einem der Ansprüche 1-9 mit einem pharmazeutischen Trägermaterial.

**Claims** (for the Contracting States : DE, FR, CH, LI, IT, NL, BE, SE, LU)

1. A coumpound of formula I

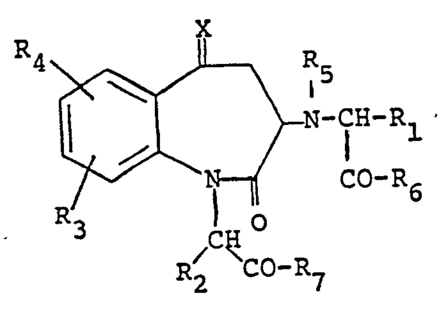

(I)

wherein

$R_1$ is hydrogen, $C_{1-7}$-alkyl, amino-$C_{1-7}$-alkyl, aryl, phenyl which is mono- or disubstituted by $C_{1-7}$-

alkyl, $C_{1-7}$-alkoxy, $C_{1-7}$-alkylenedioxy, $C_{1-7}$-alkanoyloxy, hydroxy, halogen or trifluoromethyl, or is aryl-$C_{1-7}$-alkyl, phenyl-$C_{1-7}$-alkyl which is mono- or disubstituted by $C_{1-7}$-alkyl, $C_{1-7}$-alkoxy, $C_{1-7}$-alkylenedioxy, $C_{1-7}$-alkanoyloxy, hydroxy, halogen or trifluoromethyl, or is cycloalkyl-$C_{1-7}$-alkyl ;

$R_2$ is hydrogen or $C_{1-7}$-alkyl ;

$R_3$ and $R_4$ are each independently hydrogen, $C_{1-7}$-alkyl, $C_{1-7}$-alkoxy, $C_{1-7}$-alkanoyloxy, hydroxy, halogen or trifluoromethyl or $R_3$ and $R_4$ together are $C_{1-7}$-alkylenedioxy ;

$R_5$ is hydrogen or $C_{1-7}$-alkyl ;

$R_6$ and $R_7$ are each independently hydroxy ; $C_{1-7}$-alkoxy ; ω-(amino, mono- or di-$C_{1-7}$-alkylamino)-substituted $C_{2-4}$-alkoxy ; carboxy-substituted $C_{1-7}$-alkoxy ; $C_{1-7}$-alkoxy-carbonyl-substituted $C_{1-7}$-alkoxy ; aryl-substituted $C_{1-7}$-alkoxy ; phenyl-$C_{1-7}$-alkoxy which is mono- or disubstituted by $C_{1-7}$-alkyl, $C_{1-7}$-alkoxy, $C_{1-7}$-alkylenedioxy, $C_{1-7}$-alkanoyloxy, hydroxy, halogen or trifluoromethyl ; or (hydroxy, $C_{1-7}$-alkanoyloxy or $C_{1-7}$-alkoxy)-substituted $C_{1-7}$-alkoxy ; (hydroxy, $C_{1-7}$-alkanoyloxy or $C_{1-7}$-alkoxy)-substituted $C_{1-7}$-alkoxymethoxy ; bicycloalkylcarbonylsubstituted $C_{1-7}$-alkoxy ; 3-phthalidoxy ; ($C_{1-7}$-alkyl, $C_{1-7}$-alkoxy, halo)-substituted 3-phthalidoxy ; amino ; $C_{1-7}$-alkylamino ; di-$C_{1-7}$-alkylamino ; di-$C_{1-7}$-alkylamino in which the two alkyl moities are linked by a carbon carbon bond and, together with the amino nitrogen, form a 5-, 6-, or 7-membered heterocyclic ring ; α-(carboxy or $C_{1-7}$-alkoxycarbonyl)-substituted $C_{1-7}$-alkylamino ; aryl-substituted $C_{1-7}$-alkylamino which may be substituted at the α-carbon atom by carboxy or $C_{1-7}$-alkoxycarbonyl ; or phenyl-$C_{1-7}$-alkylamino which is mono- or disubstituted by $C_{1-7}$-alkyl, $C_{1-7}$-alkoxy, $C_{1-7}$-alkylenedioxy, $C_{1-7}$-alkanoyloxy, hydroxy, halogen or trifluoromethyl and which may be substituted at the α-carbon atom by carboxy or $C_{1-7}$-alkoxycarbonyl ; and

X is oxo, two hydrogen atoms or a hydrogen atom and a hydroxyl group ;

and wherein the carbocyclic ring may also be hexahydro or 6,7,8,9-tetrahydro ; or a stereoisomer or a salt thereof.

2. A compound of formula I according to claim 1, wherein

$R_2$, $R_3$, $R_4$, $R_5$ and X are as defined in claim 1,

$R_1$ is hydrogen, $C_{1-7}$-alkyl, amino-$C_{1-7}$-alkyl, aryl, aryl-$C_{1-7}$-alkyl, cycloalkyl-$C_{1-7}$-alkyl, or is phenyl or phenyl-$C_{1-7}$-alkyl, each mono- or disubstituted by $C_{1-7}$-alkyl, $C_{1-7}$-alkoxy, $C_{1-7}$-alkylenedioxy, $C_{1-7}$-alkanoyloxy, hydroxy, halogen or trifluoromethyl, and

$R_6$ and $R_7$ are each independently hydroxy, amino, mono- or di-$C_{1-7}$-alkylamino, $C_{1-7}$-alkoxy, aryl-$C_{1-7}$-alkoxy, $C_{1-7}$-alkanoyloxymethoxy, ω-(amino, mono- or di-$C_{1-7}$-alkylamino)-$C_{2-4}$-alkoxy, (carboxy or $C_{1-7}$-alkoxycarbonyl)-$C_{1-7}$-alkoxy.

or a salt thereof.

3. A compound of formula I according to claim 1, wherein

$R_2$, $R_5$ and X are as defined in claim 1,

$R_1$ is hydrogen, $C_{1-7}$-alkyl, amino-$C_{1-7}$-alkyl, phenyl-$C_{1-7}$-alkyl, or is phenyl-$C_{1-7}$-alkyl which is mono- or disubstituted in the phenyl moiety by $C_{1-7}$-alkyl, hydroxy, $C_{1-7}$-alkoxy, $C_{1-7}$-alkylenedioxy, $C_{1-7}$-alkanoyloxy, halogen or trifluoromethyl,

$R_3$ and $R_4$ are hydrogen, $C_{1-7}$-alkyl, halogen or trifluoromethyl, or $R_3$ and $R_4$ together are $C_{1-7}$-alkylenedioxy,

$R_6$ and $R_7$ are each independently hydroxy, amino, $C_{1-7}$-alkoxy, phenyl-$C_{1-7}$-alkoxy or $C_{1-7}$-alkoxycarbonyl-$C_{1-7}$-alkoxy,

or a salt thereof.

4. A compound of formula IA

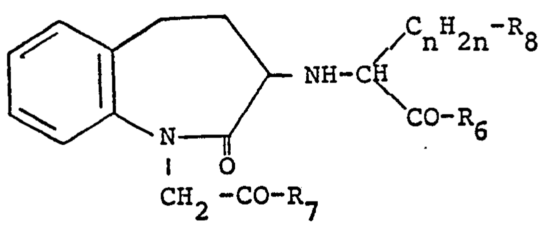

(IA)

wherein n is an integer from 1 to 4, $R_8$ is hydrogen, unsubstituted phenyl or phenyl which is monosubstituted by $C_{1-7}$-alkyl, $C_{1-7}$-alkoxy, $C_{1-7}$-alkanoyloxy, halogen, hydroxy or trifluoromethyl, and $R_6$ and $R_7$ are each independently hydroxy, $C_{1-4}$-alkoxy, benzyloxy or amino, or a pharmaceutically acceptable salt thereof.

5. A compound of formula IB

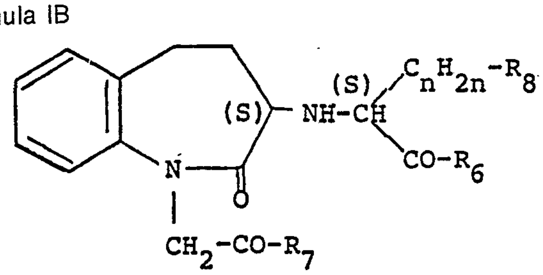

(IB)

wherein (S) is the chirality and n, $R_6$, $R_7$ and $R_8$ are as defined in claim 4, or a pharmaceutically acceptable salt thereof.

6. 1-Carboxymethyl-3-(1-ethoxycarbonyl-3-phenylpropylamino)-2,3,4,5-tetrahydro-1H-[1] benzazepin-2-one, or a stereoisomer or a salt thereof.

7. The racemic compound which melts at 138 to 140 °C of the compound claimed in claim 6, or an enantiomer or a salt thereof.

8. The racemic compound which melts at 126 to 129 °C of the compound claimed in claim 6, or an enantiomer or a salt thereof.

9. 1-Carboxymethyl-3S-(1S-ethoxycarbonyl-3-phenylpropylamino)-2,3,4,5-tetrahydro-1H-[1] benzazepin-2-one, or a salt thereof.

10. 1-Carboxymethyl-3-(1-carboxy-3-phenylpropylamino)-2,3,4,5-tetrahydro-1H-[1] benzazepin-2-one, or a stereoisomer or a salt thereof.

11. 1-Carboxymethyl-3S-(1S-carboxy-3-phenylpropylamino)-2,3,4,5-tetrahydro-1H-[1] benzazepin-2-one, or a salt thereof.

12. 1-Ethoxycarbonylmethyl-3-(1-carboxy-3-phenylpropylamino)-2,3,4,5-tetrahydro-1H-[1] benzazepin-2-one, or a stereoisomer or a salt thereof.

13. The racemate which melts at 175-177 °C of the compound as claimed in claim 12, or a salt thereof.

14. The racemate of 1-carboxymethyl-7-chloro-3-(1-ethoxycarbonyl-3-phenylpropylamino)-2,3,4,5-tetrahydro-1H-[1] benzazepin-2-one, the hydrochloride of which melts at 149 to 151 °C, or a salt thereof.

15. 3-(1-Benzyloxycarbonyl-3-phenylpropylamino)-1-carboxymethyl-2,3,4,5-tetrahydro-1H-[1] benzazepin-2-one, or a stereoisomer or a salt thereof.

16. A pharmaceutical composition containing a compound as claimed in any one of clams 2 and 4 to 10, together with a pharmaceuticaly acceptable carrier.

17. A pharmaceutical composition containing a compound as claimed in any one of claims 3 and 11 to 13, together with a pharmaceutically acceptable carrier.

18. A pharmaceutical composition containing a compound as claimed in any one of claims 1, 14 and 15, together with a pharmaceutically acceptable carrier.

19. Use of a compound as claimed in any one of claims 2 and 4 to 10 in a therapeutic method of treating the human or animal body.

20. Use of a compound as claimed in any one of claims 3 and 11 to 13 in a therapeutic method of treating the human or animal body.

21. Use of a compound as claimed in any one of claims 1, 14 and 15 in a therapeutic method of treating the human or animal body.

22. Use of a compound as claimed in any one of claims 2 and 4 to 10 as angiotensin-converting enzyme inhibitor.

23. Use of a compound as claimed in any one of claims 3 and 11 to 13 as angiotensin-converting enzyme inhibitor.

24. Use of a compound as claimed in any one of claims 1, 14 and 15 as angiotensin-converting enzyme inhibitor.

25. Use of a compound as claimed in any one of claims 2 and 4 to 10 for the preparation of a pharmaceutical composition.

26. Use of a compound as claimed in any one of claims 3 and 11 to 13 for the preparation of a pharmaceutical composition.

27. Use of a compound as claimed in any one of claims 1, 14 and 15 for the preparation of a pharmaceutical composition.

28. A process for the preparation of a compound of formula I as claimed in claim 1, or of a stereoisomer or a salt thereof, which process comprises

a) introducing the radical $—CH(R_1)COR_6$ into a compound of formula II

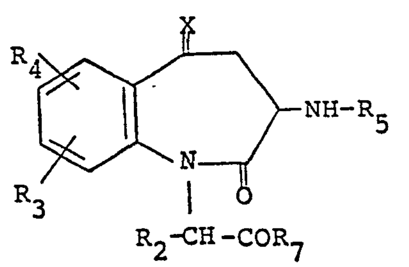

(II)

wherein the carbocyclic ring may also be hexahydro or 6,7,8,9-tetrahydro and wherein X, $R_2$, $R_3$, $R_4$, $R_5$ and $R_6$ are as defined above, by alkylation with a compound of the formula $R_1—CH(Z)—COR_6$ (IIIA), wherein Z is a reactive esterified hydroxyl group and $R_1$ and $R_6$ are as defined above, or with a compound of the formula $R_1—CO—COR_6$ (IV), wherein $R_1$ and $R_6$ are as defined above, in the presence of a

reducing agent, with temporary protection of any primary and secondary amino groups and/or, optionally, hydroxyl and/or oxo groups, which may be present in any one of the radicals X, $R_1$, $R_3$, $R_4$, $R_6$ and $R_7$, or

b) alkylating a compound of formula V

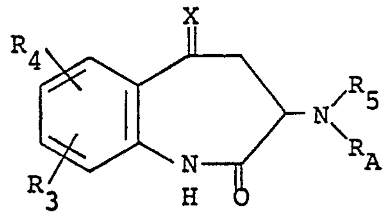

(V)

wherein the carbocyclic ring may also be hexahydro or 6,7,8,9-tetrahydro and wherein X, $R_3$, $R_4$ and $R_5$ are as defined above and $R_A$ is hydrogen or —CH($R_1$)—$COR_6$ as defined above, with a compound of the formula $R_2$—CH(Z)—$COR_7$ (IIIB), wherein Z is a reactive esterified hydroxyl group and $R_2$ and $R_7$ are as defined above, while protecting temporarily any primary and secondary amino groups and/or, optionally, hydroxyl and/or oxo groups which may be present in any one of the radicals X, $R_1$, $R_3$, $R_4$, $R_6$ and $R_7$, or

c) condensing a compound of formula VI

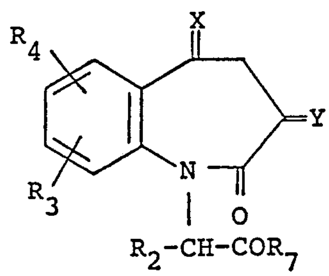

(VI)

wherein the carbocyclic group may also be hexahydro or 6,7,8,9-tetrahydro and wherein Y is oxo or a reactive esterified hydroxyl group Z and hydrogen, and X, $R_2$, $R_3$, $R_4$ and $R_7$ are as defined above, with an amine of formula VII

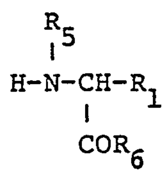

(VII)

wherein $R_1$, $R_5$ and $R_6$ are as defined above, with the proviso that, if Y is oxo, the condensation is carried out in the presence of a reducing agent and with temporary protection of the oxo group which may be present as the substituent X, or

d) in a compound of formula VIII

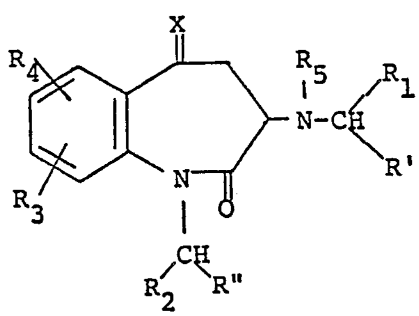

(VIII)

wherein the carbocyclic ring may also be hexahydro or 6,7,8,9-tetrahydro and wherein X and $R_1$ to $R_5$ are as defined above, one of the symbols R' and R" is cyano and the other is also cyano or $COR_6$ or $COR_7$ as defined above, subjecting the cyano group or groups to solvolysis, or

e) cyclising a compound of formula IX

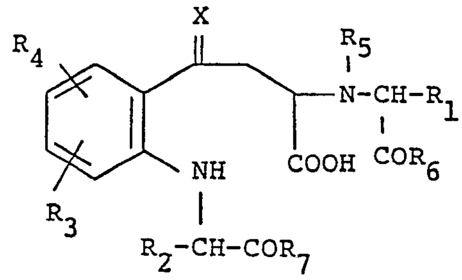

(IX)

wherein the carbocyclic ring may also be hexahydro or 3,4,5,6-tetrahydro and wherein X and $R_1$ to $R_7$ are as defined above, or cyclising an ester thereof, or

f) treating a compound which is structurally identical with a compound of formula I above, except for having an additional double bond in the C-3-position or between the exocyclic nitrogen atom and the adjacent carbon atom in the side chain, with a reducing agent in order to saturate this double bond, and, if desired, converting a resultant compound of formula I as defined above into another compound of formula I within its above-specified scope, and/or if desired, converting a resultant compound of formula I as defined above and having salt-forming properties into a salt thereof or liberating a free compound from such a salt, and/or, if desired, enriching an optical isomer which has a specific configuration with respect to at least one center of chirality from a mixture of stereoisomeric forms of a resultant compound of formula I.

29. A process for the preparation of a compound of formula I as claimed in claim 1, wherein X is oxo and $R_1$ to $R_7$ are as defined above, which process comprises condensing a compound of formula X

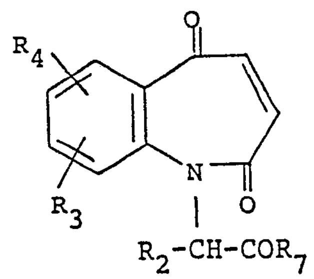

(X)

wherein the carbocyclic ring may also be hexahydro or 6,7,8,9-tetrahydro and wherein $R_2$ to $R_4$ and $R_7$ are as defined above, with an amine of formula VII

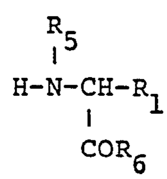

(VII)

wherein $R_1$, $R_5$ and $R_6$ are as defined above, and, if desired, converting a resultant compound of formula I as defined above into another compound of formula I within its above-specified scope, and/or, if desired, converting a resultant compound of formula I as defined above and having salt-forming properties into a salt thereof of liberating a free compound from such a salt, and/or, if desired, enriching an optical isomer which has a specific configuration with respect to at least one center of chirality from a mixture of stereoisomeric forms of a resultant compound of formula I.

30. A compound obtainable by the process according to claim 28.

31. A compound obtainable by the process according to claim 29.

**Claims** (for the Contracting State AT)

1. A process for the preparation of a novel 3-amino-[1] benzazepin-2-one-1-alkane acid, or of a derivative thereof, of the general formula I

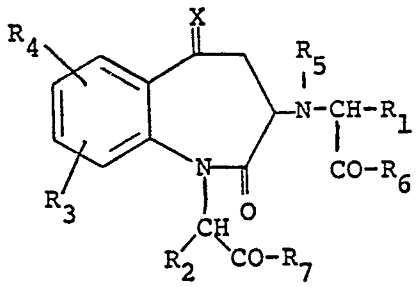

(I)

wherein

$R_1$ is hydrogen, $C_{1-7}$-alkyl, amino-$C_{1-7}$-alkyl, aryl, phenyl which is mono- or disubstituted by $C_{1-7}$-alkyl, $C_{1-7}$-alkoxy, $C_{1-7}$-alkylenedioxy, $C_{1-7}$-alkanoyloxy, hydroxy, halogen or trifluoromethyl, or is aryl-$C_{1-7}$-alkyl, phenyl-$C_{1-7}$-alkyl which is mono- or disubstituted by $C_{1-7}$-alkyl, $C_{1-7}$-alkoxy, $C_{1-7}$-alkylenedioxy, $C_{1-7}$-alkanoyloxy, hydroxy, halogen or trifluoromethyl, or is cycloalkyl-$C_{1-7}$-alkyl ;

$R_2$ is hydrogen or $C_{1-7}$-alkyl ;

$R_3$ and $R_4$ are each independently hydrogen, $C_{1-7}$-alkyl, $C_{1-7}$-alkoxy, $C_{1-7}$-alkanoyloxy, hydroxy, halogen or trifluoromethyl or $R_3$ and $R_4$ together are $C_{1-7}$-alkylenedioxy ;

$R_5$ is hydrogen or $C_{1-7}$-alkyl ;

$R_6$ and $R_7$ are each independently hydroxy ; $C_{1-7}$-alkoxy ; ω-(amino, mono- or di-$C_{1-7}$-alkylamino)-substituted $C_{2-4}$-alkoxy ; carboxy-substituted $C_{1-7}$-alkoxy ; $C_{1-7}$-alkoxycarbonyl-substituted $C_{1-7}$-alkoxy ; aryl-substituted $C_{1-7}$-alkoxy ; phenyl-$C_{1-7}$-alkoxy which is mono- or disubstituted by $C_{1-7}$-alkyl, $C_{1-7}$-alkoxy, $C_{1-7}$-alkylenedioxy, $C_{1-7}$-alkanoyloxy, hydroxy, halogen or trifluoromethyl ; or (hydroxy, $C_{1-7}$-alkanoyloxy or $C_{1-7}$-alkoxy)-substituted $C_{1-7}$-alkoxy ; (hydroxy, $C_{1-7}$-alkanoyloxy or $C_{1-7}$-alkoxy)-substituted $C_{1-7}$-alkoxymethoxy ; bicycloalkylcarbonyl-substituted $C_{1-7}$-alkoxy ; 3-phthalidoxy ; ($C_{1-7}$-alkyl, $C_{1-7}$-alkoxy, halo)-substituted 3-phthalidoxy ; amino ; $C_{1-7}$-alkylamino ; di-$C_{1-7}$-alkylamino ; di-$C_{1-7}$-alkylamino in which the two alkyl moities are linked by a carbon carbon bond and, together with the amino nitrogen, form a 5-, 6- or 7-membered heterocyclic ring ; α-(carboxy or $C_{1-7}$-alkoxycarbonyl)-substituted $C_{1-7}$-alkylamino ; aryl-substituted $C_{1-7}$-alkylamino which may be substituted at the α-carbon atom by carboxy or $C_{1-7}$-alkoxycarbonyl ; or phenyl-$C_{1-7}$-alkylamino which is mono- or disubstituted by $C_{1-7}$-alkyl, $C_{1-7}$-alkoxy, $C_{1-7}$-alkylenedioxy, $C_{1-7}$-alkanoyloxy, hydroxy, halogen or trifluoromethyl and which may be substituted at the α-carbon atom by carboxy or $C_{1-7}$-alkoxycarbonyl ; and

X is oxo, two hydrogen atoms or a hydrogen atom and a hydroxyl group ;

and wherein the carbocyclic ring may also be hexahydro or 6,7,8,9-tetrahydro ; or of a stereoisomer or a salt thereof, which process comprises

a) introducing the radical —$CH(R_1)COR_6$ into a compound of formula II

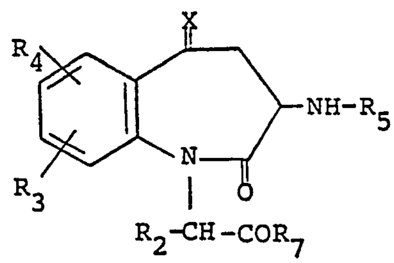

(II)

wherein the carbocyclic ring may also be hexahydro or 6,7,8,9-tetrahydro and wherein X, $R_2$, $R_3$, $R_4$, $R_5$ and $R_6$ are as defined above, by alkylation with a compound of the formula $R_1$—CH(Z)—$COR_6$ (IIIA), wherein Z is a reactive esterified hydroxyl group and $R_1$ and $R_6$ are as defined above, or with a compound of the formula $R_1$—CO—$COR_6$ (IV), wherein $R_1$ and $R_6$ are as defined above, in the presence of a reducing agent, with temporary protection of any primary and secondary amino groups and/or, optionally, hydroxyl and/or oxo groups, which may be present in any one of the radicals X, $R_1$, $R_3$, $R_4$, $R_6$ and $R_7$, or

b) alkylating a compound of formula V

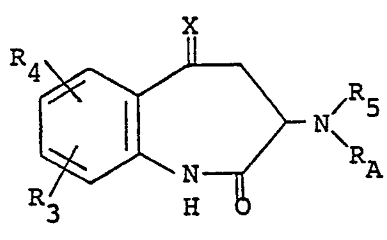

(V)

wherein the carbocyclic ring may also be hexahydro or 6,7,8,9-tetrahydro and wherein X, $R_3$, $R_4$ and $R_5$ are as defined above and $R_A$ is hydrogen or $-CH(R_1)COR_6$ as defined above, with a compound of the formula $R_2-CH(Z)-COR_7$ (IIIB), wherein Z is a reactive esterified hydroxyl group and $R_2$ and $R_7$ are as defined above, while protecting temporarily any primary and secondary amino groups and/or, optionally, hydroxyl and/or oxo groups which may be present in any one of the radicals X, $R_1$, $R_3$, $R_4$, $R_6$ and $R_7$, or

c) condensing a compound of formula VI

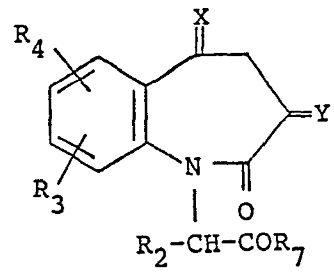

(VI)

wherein the carbocyclic group may also be hexahydro or 6,7,8,9-tetrahydro and wherein Y is oxo or a reactive esterified hydroxyl group Z and hydrogen, and X, $R_2$, $R_3$, $R_4$ and $R_7$ are as defined above, with an amine of formula VII

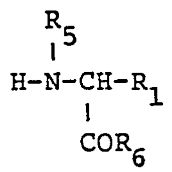

(VII)

wherein $R_1$, $R_5$ and $R_6$ are as defined above, with the proviso that, if Y is oxo, the condensation is carried out in the presence of a reducing agent and with temporary protection of the oxo group which may be present as the substituent X, or

d) in a compound of formula VIII

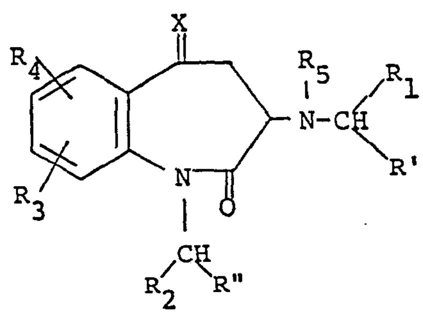

(VIII)

wherein the carbocyclic ring may also be hexahydro or 6,7,8,9-tetrahydro and wherein X and $R_1$ to $R_5$ are as defined above, one of the symbols R' and R" is cyano and the other is also cyano or $COR_6$ or $COR_7$ as defined above, subjecting the cyano group or groups to solvolysis, or

e) cyclising a compound of formula IX

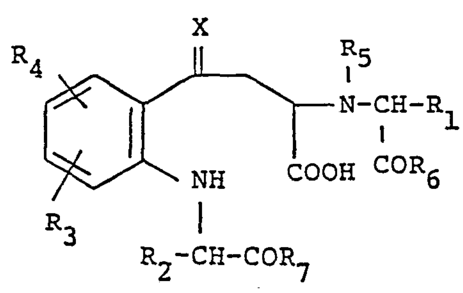

(IX)

wherein the carbocyclic ring may also be hexahydro or 3,4,5,6-tetrahydro and wherein X and $R_1$ to $R_7$ are as defined above, or cyclising an ester thereof, or

f) treating a compound which is structurally identical with a compound of formula I, above, except for having an additional double bond in the C-3-position or between the exocyclic nitrogen atom and the adjacent carbon atom in the side chain, with a reducing agent in order to saturate this double bond, and, if desired, converting a resultant compound of formula I as defined above into another compound of formula I within its above-specified scope, and/or, if desired, converting a resultant compound of formula I as defined above and having salt-forming properties into a salt thereof or liberating a free compound from such a salt, and/or, if desired, enriching an optical isomer which has a specific configuration with respect to at least one center of chirality from a mixture of stereoisomeric forms of a resultant compound of formula I.

2. A process according to claim 1, which comprises preparing a compound of formula I, wherein $R_2$, $R_3$, $R_4$, $R_5$ and X are as defined in claim 1,

$R_1$ is hydrogen, $C_{1-7}$-alkyl, amino-$C_{1-7}$-alkyl, aryl, aryl-$C_{1-7}$-alkyl, cycloalkyl-$C_{1-7}$-alkyl, or is phenyl or phenyl-$C_{1-7}$-alkyl, each mono- or disubstituted by $C_{1-7}$-alkyl, $C_{1-7}$-alkoxy, $C_{1-7}$-alkylenedioxy, $C_{1-7}$-alkanoyloxy, hydroxy, halogen or trifluoromethyl, and

$R_6$ and $R_7$ are each independently hydroxy, amino, mono- or di-$C_{1-7}$-alkylamino, $C_{1-7}$-alkoxy, aryl-$C_{1-7}$-alkoxy, $C_{1-7}$-alkanoyloxymethoxy, ω-(amino- mono- or di-$C_{1-7}$-alkylamino)-$C_{2-4}$-alkoxy, (carboxy or $C_{1-7}$-alkoxycarbonyl)-$C_{1-7}$-alkoxy,

or a salt thereof,

3. A process according to claim 1, which comprises preparing a compound of formula I, wherein $R_2$, $R_5$ and X are as defined in claim 1,

$R_1$ is hydrogen, $C_{1-7}$-alkyl, amino-$C_{1-7}$-alkyl, phenyl-$C_{1-7}$-alkyl, or is phenyl-$C_{1-7}$-alkyl which is mono- or disubstituted in the phenyl moiety by $C_{1-7}$-alkyl, hydroxy, $C_{1-7}$-alkoxy, $C_{1-7}$-alkylenedioxy, $C_{1-7}$-alkanoyloxy, halogen or trifluoromethyl,

$R_3$ and $R_4$ are hydrogen, $C_{1-7}$-alkoxy, $C_{1-7}$-alkyl, halogen or trifluoromethyl, or $R_3$ and $R_4$ together are $C_{1-7}$-alkylenedioxy,

$R_6$ and $R_7$ are each independently hydroxy, amino, $C_{1-7}$-alkoxy, phenyl-$C_{1-7}$-alkoxy or $C_{1-7}$-alkoxycarbonyl-$C_{1-7}$-alkoxy,

or a salt thereof.

4. A process according to claim 1, which comprises preparing a compound of formula IA

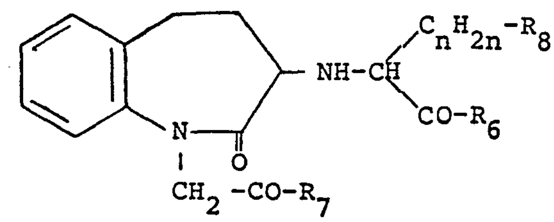

(IA)

wherein n is an integer from 1 to 4, $R_8$ is hydrogen, unsubstituted phenyl or phenyl which is monosubstituted by $C_{1-7}$-alkyl, $C_{1-7}$-alkoxy, $C_{1-7}$-alkanoyloxy, halogen, hydroxy or trifluoromethyl, and $R_6$ and $R_7$ are each independently hydroxy, $C_{1-4}$-alkoxy, benzyloxy or amino, or a pharmaceutically acceptable salt thereof.

5. A process according to claim 1, which comprises preparing 1-carboxymethyl-3-(1-ethoxycarbonyl-3-phenylpropylamino)-2,3,4,5-tetrahydro-1H-[1] benzazepin-2-one, or a stereoisomer or a salt thereof.

6. A process according to claim 1, which comprises preparing the racemic compound which melts at 138 to 140 °C of the compound claimed in claim 5, or an enantiomer or a salt thereof.

7. A process according to claim 1, which comprises preparing the racemic compound which melts at 126 to 129 °C of the compound claimed in claim 5, or an enantiomer or a salt thereof.

8. A process according to claim 1, which comprises preparing 1-carboxymethyl-3S-(1S-ethoxycarbonyl-3-phenylpropylamino)-2,3,4,5-tetrahydro-1H-[1] benzazepin-2-one, or a salt thereof.

9. A process for the preparation of a compound for formula I as claimed in claim 1, wherein X is oxo and $R_1$ to $R_7$ are as defined above, which process comprises condensing a compound of formula X

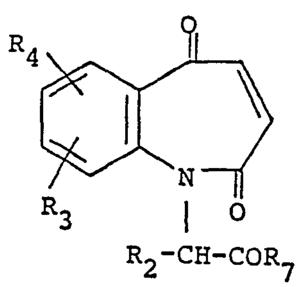

(X)

wherein the carbocyclic ring may also be hexahydro or 6,7,8,9-tetrahydro and wherein $R_2$ to $R_4$ and $R_7$ are as defined above, with an amine of formula VII

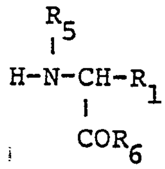

(VII)

wherein $R_1$, $R_5$ and $R_6$ are as defined above, and, if desired, converting a resultant compound of formula I as defined above into another compound of formula I within its above-specified scope, and/or, if desired, converting a resultant compound of formula I as defined above and having salt-forming properties into a salt thereof or liberating a free compound from such a salt, and/or, if desired, enriching an optical isomer which has a specific configuration with respect to at least one center of chirality from a mixture of stereoisomeric forms of a resultant compound of formula I.

10. A process for the preparation of a pharmaceutical composition, which process comprises combining a compound of the invention as claimed in any one of claims 1 to 9 with a pharmaceutical carrier.

**Revendications** (pour les Etats Contractants : DE, FR, CH, LI, IT, NL, BE, SE, LU)

1. Composés de formule I

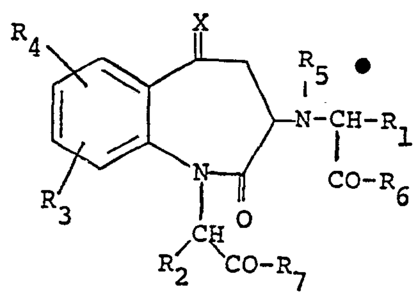

(I)

dans laquelle

$R_1$ représente l'hydrogène, un groupe alkyle en C1-C7, amino-alkyle en C1-C7, aryle, phényle mono- ou di-substitué par des groupes alkyle en C1-C7, alcoxy en C1-C7, alkylène-dioxy en C1-C7, alcanoyloxy en C1-C7, hydroxy, des halogènes ou des groupes trifluorométhyle, un groupe aryl-alkyle en C1-C7, phényl-alkyle en C1-C7 mono- ou di-substitué par des groupes alkyle en C1-C7, alcoxy en C1-C7, alkylène-dioxy en C1-C7, alcanoyloxy en C1-C7, hydroxy, des halogènes ou des groupes trifluorométhyle, cycloalkyle ou cycloalkyl-(alkyle en C1-C7) ;

$R_2$ représente l'hydrogène ou un groupe alkyle en C1-C7 ;

$R_3$ et $R_4$ représentent chacun, indépendamment l'un de l'autre, l'hydrogène, un groupe alkyle en C1-C7, alcoxy en C1-C7, alcanoyloxy en C1-C7, hydroxy, un halogène ou un groupe trifluorométhyle ou bien $R_3$ et $R_4$ forment ensemble un groupe alkylène-dioxy en C1-C7 ;

$R_5$ représente l'hydrogène ou un groupe alkyle en C1-C7 ;

$R_6$ et $R_7$ représentent chacun, indépendamment l'un de l'autre, un groupe hydroxy, alcoxy en C1-C7, alcoxy en C2-C4 substitué en oméga par un groupe amino, mono- ou di-(alkyle en C1-C7)-amino ; alcoxy en C1-C7 substitué par un groupe carboxy ; alcoxy en C1-C7 substitué par un groupe (alcoxy en C1-C7)-carbonyle ; alcoxy en C1-C7 substitué par un groupe aryle ; phényl-alcoxy en C1-C7 mono- ou di-substitué par des groupes alkyle en C1-C7, alcoxy en C1-C7, alkylène-dioxy en C1-C7, alcanoyloxy en C1-C7, hydroxy, des halogènes ou des groupes trifluorométhyle ; alcoxy en C1-C7 substitué par des groupes hydroxy, alcanoyloxy en C1-C7 ou alcoxy en C1-C7, (alcoxy en C1-C7)-méthoxy substitué par des groupes hydroxy, alcanoyloxy en C1-C7 ou alcoxy en C1-C7 ; alcoxy en C1-C7 substitué par un groupe bicycloalcoxycarbonyle ; 3-phtalidoxy ; 3-phtalidoxy substitué par des groupes alkyle en C1-C7, alcoxy en C1-C7 ou des halogènes ; amino, alkylamino en C1-C7 ; di-(alkyle en C1-C7)-amino ; di-(alkyle en C1-C7)-amino dans lequel les deux groupes alkyle sont reliés par une liaison carbone-carbone et forment ensemble et avec l'atome d'azote aminé un noyau hétérocyclique à 5, 6 ou 7 chaînons ; alkylamino en C1-C7 substitué en alpha par un groupe carboxy ou (alcoxy en C1-C7)-carbonyle ; alkylamino en C1-C7 substitué par un groupe aryle ou phényl-alkylamino en C1-C7 mono- ou di-substitué par des groupes alkyle en C1-C7, alcoxy en C1-C7, alkylène-dioxy en C1-C7, alcanoyloxy en C1-C7, hydroxy, des halogènes ou des groupes trifluorométhyle, et éventuellement substitué sur l'atome de carbone alpha par un groupe carboxy ou (alcoxy en C1-C7)-carbonyle ;

et X représente un groupe oxo, deux atomes d'hydrogène ou un atome d'hydrogène et un groupe hydroxy ;

le noyau carbocyclique pouvant également être hexahydrogéné ou 6,7,8,9-tétrahydrogéné ; leurs stéréoisomères et leurs sels.

2. Composés de formule I selon la revendication 1, dans laquelle

$R_2$, $R_3$, $R_4$, $R_5$ et X ont les significations indiquées dans la revendication 1,

$R_1$ représente l'hydrogène, un groupe alkyle en C1-C7, aminoalkyle en C1-C7, aryle, aryl-alkyle en C1-C7, cycloalkyl-alkyle en C1-C7 ou phényle ou phényl-alkyle en C1-C7 mono- ou di-substitué par des groupes alkyle en C1-C7, alcoxy en C1-C7, alkylène-dioxy en C1-C7, alcanoyloxy en C1-C7, hydroxy, des halogènes ou des groupes trifluorométhyle, et

$R_6$ et $R_7$ représentent chacun, indépendamment l'un de l'autre, un groupe hydroxy, amino, mono- ou di-(alkyle en C1-C7)-amino, alcoxy en C1-C7, aryl-alcoxy en C1-C7, (alcanoyloxy en C1-C7)-méthoxy, alcoxy en C2-C4 substitué en oméga par un groupe amino, mono- ou di-(alkyle en C1-C7)-amino, alcoxy en C1-C7 substitué par un groupe carboxy ou (alcoxy en C1-C7)-carbonyle, ou leurs sels.

3. Composés de formule I selon la revendication 1, dans laquelle

$R_2$, $R_5$ et X ont les significations indiquées dans la revendication 1,

$R_1$ représente l'hydrogène, un groupe alkyle en C1-C7, aminoalkyle en C1-C7, phényle-alkyle en C1-C7 ou phényl-alkyle en C1-C7 mono- ou di-substitué dans la partie phényle par des groupes alkyle en C1-C7, hydroxy, alcoxy en C1-C7, alkylène-dioxy en C1-C7, alcanoyloxy en C1-C7, des halogènes ou des groupes trifluorométhyle,

$R_3$ et $R_4$ représentent l'hydrogène, des groupes alcoxy en C1-C7, alkyle en C1-C7, des halogènes ou des groupes trifluorométhyle ou bien $R_3$ et $R_4$ forment ensemble un groupe alkylène-dioxy en C1-C7,

$R_6$ et $R_7$ représentent chacun, indépendamment l'un de l'autre, un groupe hydroxy, amino, alcoxy en C1-C7, phényl-alcoxy en C1-C7, (alcoxy en C1-C7)-carbonyl-(alcoxy en C1-C7), ou leurs sels.

4. Composés de formule IA

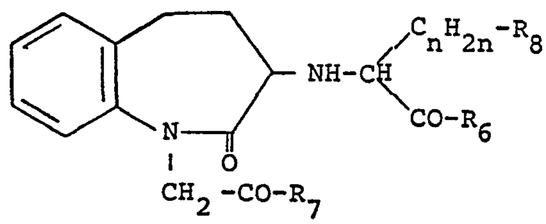

(IA)

dans laquelle n est un nombre entier de 1 à 4, $R_8$ représente l'hydrogène, phényle non substitué ou phényle monosubstitué par des groupes alkyle en C1-C7, alcoxy en C1-C7, alcanoyloxy en C1-C7, un halogène, un groupe hydroxy ou trifluorométhyle, $R_6$ et $R_7$ représentent chacun, indépendamment l'un de l'autre, un groupe hydroxy, alcoxy en C1-C4, benzyloxy ou amino, ou leurs sels acceptables pour l'usage pharmaceutique.

5. Composés de formule IB

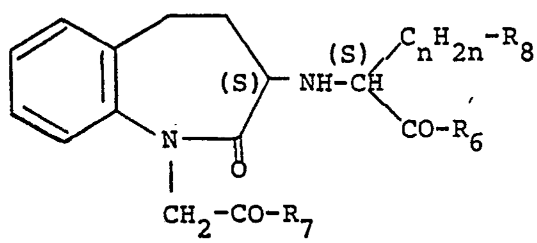

(IB)

dans laquelle (S) indique la chiralité, et n, $R_6$, $R_7$ et $R_8$ ont les significations indiquées dans la revendication 4, ou leurs sels acceptables pour l'usage pharmaceutique.

6. La 1-carboxyméthyl-3-(1-éthoxycarbonyl-3-phényl-propylamino)-2,3,4,5-tétrahydro-1H-[1] benzazépine-2-one, ses stéréoisomères et ses sels.

7. Le composé racémique fondant à 138-140 °C du composé mentionné dans la revendication 6, ses énantiomères et ses sels.

8. Le composé racémique fondant à 126-129 °C du composé mentionné dans la revendication 6, ses énantiomères et ses sels.

9. La 1-carboxyméthyl-3S-(1S-éthoxycarbonyl-3-phénylpropylamino)-2,3,4,5-tétrahydro-1H-[1] benzazépine-2-one et ses sels.

10. La 1-carboxyméthyl-3-(1-carboxy-3-phénylpropylamino)-2,3,4,5-tétrahydro-1H-[1] benzazépine-2-one, ses stéréoisomères et ses sels.

11. La 1-carboxyméthyl-3S-(1S-carboxy-3-phénylpropylamino)-2,3,4,5-tétrahydro-1H-[1] benzazépine-2-one et ses sels.

12. La 1-éthoxycarbonylméthyl-3-(1-carboxy-3-phényl-propylamino)-2,3,4,5-tétrahydro-1H-[1] benzazépine-2-one, ses stéréoisomères et ses sels.

13. Le racémate du composé mentionné dans la revendication 12, fondant à 175-177 °C, et ses sels.

14. Le racémate de la 1-carboxyméthyl-7-chloro-3-(1-éthoxycarbonyl-3-phénylpropylamino)-2,3,4,5-tétrahydro-1H-[1] benzazépine-2-one dont le chlorhydrate fond à 149-151 °C, et ses sels.

15. La 3-(1-benzyloxycarbonyl-3-phénylpropylamino)-1-carboxyméthyl-2,3,4,5-tétrahydro-1H-[1] benzazépine-2-one, ses stéréoisomères et ses sels.

16. Compositions pharmaceutiques contenant des composés des revendications 2 et 4 à 10, avec un véhicule pharmaceutique.

17. Compositions pharmaceutiques contenant des composés des revendications 3 et 11 à 13, avec un véhicule pharmaceutique.

18. Compositions pharmaceutiques contenant des composés des revendications 1, 14 et 15, avec un véhicule pharmaceutique.

19. Les composés mentionnés dans les revendications 2 et 4 à 10 pour l'utilisation dans un procédé pour le traitement thérapeutique de l'organisme humain ou animal.

20. Les composés mentionnés dans les revendications 3 et 11 à 13 pour l'utilisation dans un procédé pour le traitement thérapeutique de l'organisme humain ou animal.

21. Les composés mentionnés dans les revendications 1, 14 et 15 pour l'utilisation dans un procédé pour le traitement thérapeutique de l'organisme humain ou animal.

22. Les composés mentionnés dans les revendications 2 et 4 à 10 pour l'utilisation en tant qu'inhibiteurs de l'enzyme convertissant l'angiotensine.

23. Les composés mentionnés dans les revendications 3 et 11 à 13 pour l'utilisation en tant qu'inhibiteurs de l'enzyme convertissant l'angiotensine.

24. Les composés mentionnés dans les revendications 1, 14 et 15 pour l'utilisation en tant qu'inhibiteurs de l'enzyme convertissant l'angiotensine.

25. Utilisation des composés mentionnés dans les revendications 2 et 4 à 10 pour la préparation de compositions pharmaceutiques.

26. Utilisation des composés mentionnés dans les revendications 3 et 11 à 13 pour la préparation de compositions pharmaceutiques.

27. Utilisation des composés mentionnés dans les revendications 1, 14 et 15 pour la préparation de compositions pharmaceutiques.

28. Procédé de préparation des composés de formule I mentionnés dans la revendication 1, de leurs stéréoisomères et de leurs sels, caractérisé en ce que

a) dans un composé de formule II

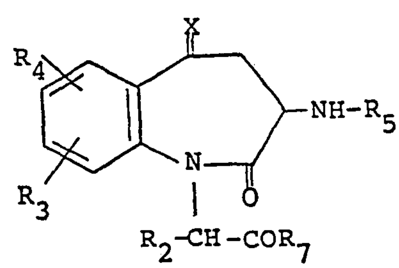

(II)

dans laquelle le noyau carbocyclique peut également être hexahydrogéné ou 6,7,8,9-tétrahydrogéné et X, $R_2$, $R_3$, $R_4$, $R_5$ et $R_7$ ont les significations indiquées ci-dessus, on introduit le reste —$CH(R_1)COR_6$ par alkylation à l'aide d'un composé de formule $R_1$—CH(Z)—$COR_6$ (IIIA), dans laquelle Z représente un groupe hydroxy estérifié réactif et $R_1$ et $R_6$ ont les significations indiquées ci-dessus, ou à l'aide d'un composé de formule $R_1$—CO—$COR_6$ (IV) dans laquelle $R_1$ et $R_6$ ont les significations indiquées ci-dessus, en présence d'un agent réducteur, avec protection transitoire des groupes amino primaires et secondaires éventuels et/ou, le cas échéant, des groupes hydroxy et/ou oxo éventuellement présents dans l'un quelconque des restes X, $R_1$, $R_3$, $R_4$, $R_6$ et $R_7$, ou bien

b) on alkyle un composé de formule V

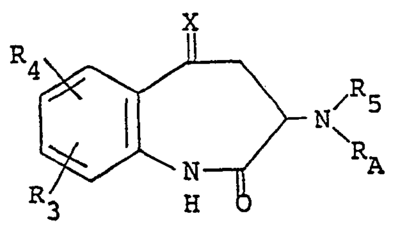

(V)

dans laquelle le noyau carbocyclique peut également être hexahydrogéné ou 6,7,8,9-tétrahydrogéné et X, $R_3$, $R_4$ et $R_5$ ont les significations indiquées ci-dessus, $R_A$ représentant l'hydrogène ou le groupe

—CH($R_1$)COR$_6$ tel que défini ci-dessus, à l'aide d'un composé de formule $R_2$—CH(Z)—COR$_7$ (IIIB), dans laquelle Z représente un groupe hydroxy estérifié réactif et $R_2$ et $R_7$ ont les significations indiquées ci-dessus, en protégeant transitoirement des groupes amino primaires et secondaires éventuels et/ou, le cas échéant, les groupes hydroxy et/ou oxo éventuellement présents dans l'un quelconque des restes X, $R_1$, $R_3$, $R_4$, $R_6$ et $R_7$ ou bien

c) on condense un composé de formule VI

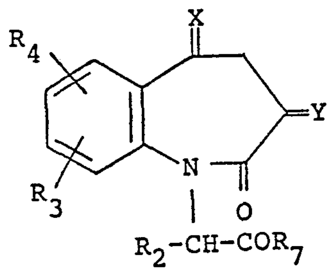

(VI)

dans laquelle le noyau carbocyclique peut également être hexahydrogéné ou 6,7,8,9-tétrahydrogéné et Y représente un groupe oxo ou un groupe hydroxy estérifié réactif Z et l'hydrogène, X, $R_2$, $R_3$, $R_4$ et $R_7$ ayant les significations indiquées ci-dessus, avec une amine de formule VII

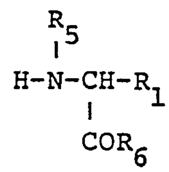

(VII)

dans laquelle $R_1$, $R_5$ et $R_6$ ont les significations indiquées ci-dessus, la condensation étant effectuée en présence d'un agent réducteur et avec protection transitoire du groupe oxo éventuellement présent en tant que substituant X lorsque Y représente un groupe oxo, ou bien

d) dans un composé de formule VIII

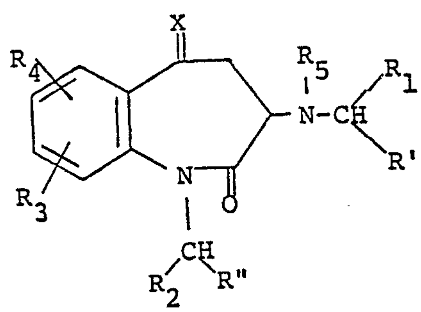

(VIII)

dans laquelle le noyau carbocyclique peut également être hexahydrogéné ou 6,7,8,9-tétrahydrogéné et X et $R_1$ à $R_5$ ont les significations indiquées ci-dessus, l'un des symboles R' et R" représente un groupe cyano et l'autre également un groupe cyano ou un groupe COR$_6$ ou COR$_7$ tel que défini ci-dessus, on soumet le ou les groupes cyano à une solvolyse, ou bien

e) on cyclise un composé de formule IX

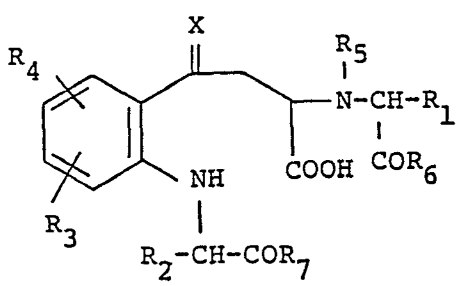

(IX)

dans laquelle le noyau carbocyclique peut également être hexahydrogéné ou 3,4,5,6-tétrahydrogéné et X et $R_1$ à $R_7$ ont les significations indiquées ci-dessus, ou un ester d'un tel composé, ou bien

f) on traite un composé dont la structure est identique à celle d'un composé de formule I ci-dessus sauf qu'il contient une double liaison supplémentaire en position C-3 ou entre l'atome d'azote exocyclique et l'atome de carbone voisin dans la chaîne latérale, par un agent réducteur afin de saturer cette double liaison, et, si on le désire, on convertit un composé obtenu répondant à la formule I telle que définie ci-dessus en un autre composé de formule I dans le cadre des limites indiquées ci-dessus, et/ou, si on le désire, on convertit un composé obtenu répondant à la formule I telle que définie ci-dessus et qui est salifiable, en un sel, ou bien on libère un composé à partir d'un tel sel et/ou, si on le désire, on enrichit un isomère optique, possédant une configuration spécifique par rapport à au moins un centre de chiralité, à partir d'un mélange des formes stéréoisomères d'un composé obtenu répondant à la formule I.

29. Procédé de préparation des composés de formule I mentionnés dans la revendication 1 dans lesquels X représente un groupe oxo et $R_1$ à $R_7$ ont les significations indiquées dans la revendication 1, caractérisé en ce que l'on condense un composé de formule X

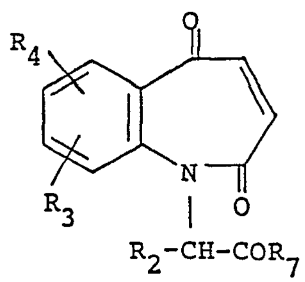

(X)

dans laquelle le noyau carbocyclique peut également être hexahydrogéné ou 6,7,8,9-tétrahydrogéné et $R_2$ à $R_4$ et $R_7$ ont les significations indiquées ci-dessus, avec une amine de formule VII

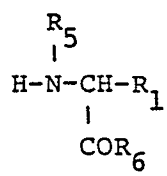

(VII)

dans laquelle $R_1$, $R_5$ et $R_6$ ont les significations indiquées ci-dessus, et, si on le désire, on convertit un composé obtenu répondant à la formule I telle que définie ci-dessus en un autre composé de formule I à l'intérieur des limites spécifiées ci-dessus, et/ou, si on le désire, on convertit un composé obtenu répondant à la formule I telle que définie ci-dessus et qui est salifiable, en un sel, ou bien on libère un composé à partir d'un tel sel et/ou, si on le désire, on enrichit un isomère optique présentant une configuration spécifique par rapport à au moins un centre de chiralité, à partir d'un mélange des formes stéréoisomères d'un composé obtenu répondant à la formule I.

30. Les composés qu'on peut obtenir par le procédé de la revendication 28.

31. Les composés qu'on peut obtenir par le procédé de la revendication 29.

**Revendications** (pour l'Etat Contractant AT)

1. Procédé de préparation de nouveaux acides 3-amino-[1] benzazépine-2-one-1-alacanoïques et de leurs dérivés, de formule générale I

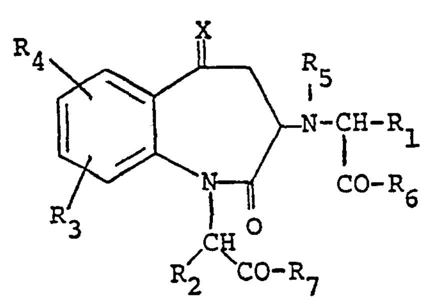

(I)

dans laquelle

$R_1$ représente l'hydrogène, un groupe alkyle en C1-C7, aminoalkyle en C1-C7, aryle, phényle mono-ou di-substitué par des groupes alkyle en C1-C7, alcoxy en C1-C7, alkylène-dioxy en C1-C7, alcanoyloxy en C1-C7, hydroxy, des halogènes ou des groupes trifluorométhyle, aryl-alkyle en C1-C7, phényl-alkyle en C1-C7 mono- ou disubstitué par des groupes alkyle en C1-C7, alcoxy en C1-C7, alkylène-dioxy en C1-C7, alcanoyloxy en C1-C7, hydroxy, des halogènes ou des groupes trifluorométhyle, cycloalkyle ou cycloalkyl-alkyle en C1-C7 ;

$R_2$ représente l'hydrogène ou un groupe alkyle en C1-C7 ;

$R_3$ et $R_4$ représentent chacun, indépendamment l'un de l'autre, l'hydrogène, un groupe alkyle en C1-C7, alcoxy en C1-C7, alcanoyloxy en C1-C7, hydroxy, un halogène ou un groupe trifluorométhyle ou bien $R_3$ et $R_4$ forment ensemble un groupe alkylène-dioxy en C1-C7 ;

$R_5$ représente l'hydrogène ou un groupe alkyle en C1-C7 ;

$R_6$ et $R_7$ représentent chacun, indépendamment l'un de l'autre, un groupe hydroxy, alcoxy en C1-C7 ; alcoxy en C2-C4 substitué en oméga par un groupe amino, mono- ou di-(alkyle en C1-C7)-amino ; alcoxy en C1-C7 substitué par un groupe carboxy ; alcoxy en C1-C7 substitué par un groupe (alcoxy en C1-C7)-carbonyle ; alcoxy en C1-C7 substitué par un groupe aryle ; phényl-alcoxy en C1-C7, mono- ou di-substitué par des groupes alkyle en C1-C7 alcoxy en C1-C7, alkylène-dioxy en C1-C7, alcanoyloxy en C1-C7, hydroxy, des halogènes ou des groupes trifluorométhyle, alcoxy en C1-C7 substitué par des groupes hydroxy, alcanoyloxy en C1-C7 ou alcoxy en C1-C7, (alcoxy en C1-C7)-méthoxy substitué par un groupe hydroxy, alcanoyloxy en C1-C7 ou alcoxy en C1-C7 ; alcoxy en C1-C7 substitué par un groupe bicycloalcoxycarbonyle ; 3-phtalidoxy ; 3-phtalidoxy substitué par un groupe alkyle en C1-C7, alcoxy en C1-C7 ou des halogènes ; amino, alkylamino en C1-C7 ; di-(alkyle en C1-C7)-amino ; di-(alkyle en C1-C7)-amino dans lequel les deux groupes alkyle sont reliés par une liaison carbone-carbone et forment ensemble et avec l'azote aminé un noyau hétérocyclique à 5, 6 ou 7 chaînons ; alkylamino en C1-C7 substitué en alpha par un groupe carboxy ou (alcoxy en C1-C7)-carbonyle ; alkylamino en C1-C7 substitué par un groupe aryle ou phényl-alkylamino en C1-C7 mono- ou di-substitué par des groupes alkyle en C1-C7, alcoxy en C1-C7, alkylène-dioxy en C1-C7, alcanoyloxy en C1-C7, hydroxy, des halogènes ou des groupes trifluorométhyle, et qui peut dans chaque cas être substitué sur l'atome de carbone alpha par un groupe carboxy ou (alcoxy en C1-C7)-carbonyle ;

et X représente un groupe oxo, deux atomes d'hydrogène ou bien un atome d'hydrogène et un groupe hydrody ;

le noyau carbocyclique pouvant également être hexahydrogéné ou 6,7,8,9-tétrahydrogéné ; de leurs stéréoisomères et de leurs sels, caractérisé en ce que

a) dans un composé de formule II

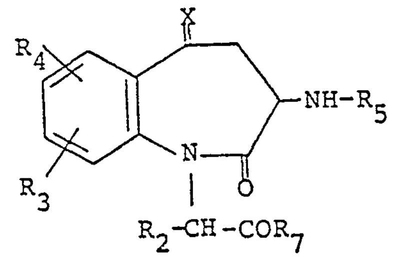

(II)

dans laquelle le noyau carbocyclique peut également être hexahydrogéné ou 6,7,8,9-tétrahydrogéné et X, $R_2$, $R_3$, $R_4$, $R_5$ et $R_7$ ont les significations indiquées ci-dessus, on introduit le reste $-CH(R_1)COR_6$ par alkylation à l'aide d'un composé de formule $R_1-CH(Z)-COR_6$ (IIIA) dans laquelle Z représente un groupe hydroxy estérifié réactif et $R_1$ et $R_6$ ont les significations indiquées ci-dessus, ou à l'aide d'un composé de formule $R_1-CO-COR_6$ (IV) dans laquelle $R_1$ et $R_6$ ont les significations indiquées ci-dessus, en présence d'un agent réducteur, en protégeant transitoirement des groupes amino primaires et secondaires éventuels et/ou, le cas échéant, des groupes hydroxy et/ou oxo éventuellement présents dans l'un quelconque des restes X, $R_1$, $R_3$, $R_4$, $R_6$ et $R_7$, ou bien

b) on alkyle un composé de formule V

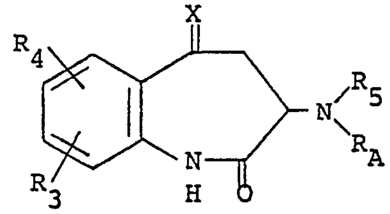

(V)

dans laquelle le noyau carbocyclique peut également être hexahydrogéné ou 6,7,8,9-tétrahydrogéné et X, $R_3$, $R_4$ et $R_5$ ont les significations indiquées ci-dessus, $R_A$ représentant l'hydrogène ou un groupe $-CH(R_1)COR_6$ tel que défini ci-dessus, à l'aide d'un composé de formule $R_2-CH(Z)COR_7$ (IIIB) dans

laquelle Z représente un groupe hydroxy estérifié réactif et $R_2$ et $R_7$ ont les significations indiquées ci-dessus, en protégeant transitoirement des groupes amino primaires et secondaires éventuels et/ou, le cas échéant, des groupes hydroxy et/ou oxo éventuellement présents dans l'un quelconque des restes X, $R_1$, $R_3$, $R_4$, $R_6$ et $R_7$, ou bien

c) on condense un composé de formule VI

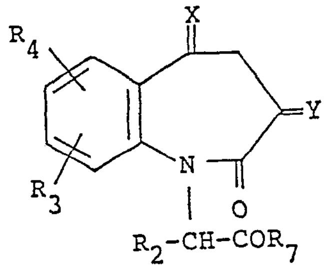

(VI)

dans laquelle le noyau carbocyclique peut également être hexahydrogéné ou 6,7,8,9-tétrahydrogéné et Y représente un groupe oxo ou un groupe hydroxy estérifié réactif Z et un atome d'hydrogène, X, $R_2$, $R_3$, $R_4$ et $R_7$ ayant les significations indiquées ci-dessus, avec une amine de formule VII

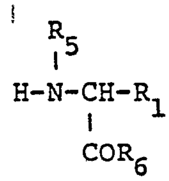

(VII)

dans laquelle $R_1$, $R_5$ et $R_6$ ont les significations indiquées ci-dessus, la condensation étant effectuée en présence d'un agent réducteur et avec protection transitoire du groupe oxo éventuellement présent en tant que substituant X lorsque Y représente un groupe oxo, ou bien

d) dans un composé de formule VIII

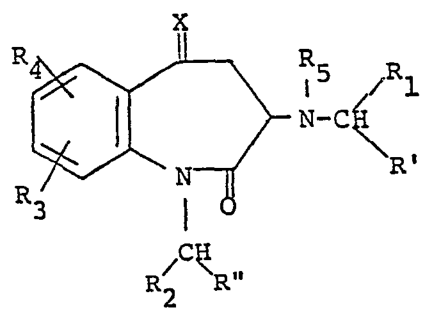

(VIII)

dans laquelle le noyau carbocyclique peut également être hexahydrogéné ou 6,7,8,9-tétrahydrogéné et X et $R_1$ à $R_5$ ont les significations indiquées ci-dessus, l'un des symboles R' et R" représente un groupe cyano et l'autre également un groupe cyano ou un groupe $COR_6$ ou $COR_7$ tel que défini ci-dessus, on soumet le ou les groupes cyano à une solvolyse, ou bien

e) on cyclise un composé de formule IX

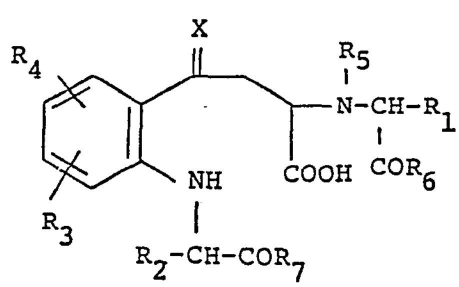

(IX)

dans laquelle le noyau carbocyclique peut également être hexahydrogéné ou 3,4,5,6-tétrahydrogéné et X et $R_1$ à $R_7$ ont les significations indiquées ci-dessus, ou un ester d'un tel composé, ou bien

f) on traite un composé dont la structure est identique à celle d'un composé de formule I ci-dessus à l'exception de la présence d'une double liaison supplémentaire dans la position C-3 ou entre l'atome

d'azote exocyclique et l'atome de carbone voisin dans la chaîne latérale, par un agent réducteur afin de saturer cette double liaison, et, si on le désire, on convertit un composé obtenu répondant à la formule I telle que définie ci-dessus en un autre composé de formule I à l'intérieur des limites spécifiées, et/ou si on le désire, on convertit un composé obtenu répondant à la formule I telle que définie ci-dessus et qui est salifiable en un sel, ou bien on libère un composé à partir d'un tel sel et/ou si on le désire, on enrichit un isomère optique possédant une configuration spécifique par rapport à au moins un centre de chiralité, à partir d'un mélange des formes stéréoisomères d'un composé obtenu répondant à la formule I.

2. Procédé selon la revendication 1, caractérisé en ce que l'on prépare des composés de formule I dans laquelle

$R_2$, $R_3$, $R_4$, $R_5$ et X ont les significations indiquées ci-dessus,

$R_1$ représente l'hydrogène, un groupe alkyle en C1-C7, aminoalkyle en C1-C7, aryle, aryl-alkyle en C1-C7, cyclo-alkyl-alkyle en C1-C7 ou phényle ou phényl-alkyle en C1-C7 mono- ou disubstitué par des groupes alkyle en C1-C7, alcoxy en C1-C7, alkylène-dioxy en C1-C7, alcanoyloxy en C1-C7, hydroxy, des halogènes ou des groupes trifluoro-méthyle et

$R_6$ et $R_7$ représentent chacun, indépendamment l'un de l'autre, un groupe hydroxy, amino, mono- ou di-(alkyle en C1-C7)-amino, alcoxy en C1-C7, aryl-alcoxy en C1-C7, (alcanoyloxy en C1-C7)-méthoxy, alcoxy en C2-C4 substitué en oméga par un groupe amino, mono- ou di-(alkyle en C1-C7)-amino, alcoxy en C1-C7 substitué par un groupe carboxy ou (alcoxy en C1-C7)-carbonyle, et leurs sels.

3. Procédé selon la revendication 1, caractérisé en ce que l'on prépare des composés de formule I dans laquelle

$R_2$, $R_5$ et X ont les significations indiquées dans la revendication 1,

$R_1$ représente l'hydrogène, un groupe alkyle en C1-C7, aminoalkyle en C1-C7, phényl-alkyle en C1-C7 ou phényl-alkyle en C1-C7 mono- ou di-substitué dans la partie phényle par des groupes alkyle en C1-C7, hydroxy, alcoxy en C1-C7, alkylène-dioxy en C1-C7, alcanoyloxy en C1-C7, des halogènes ou des groupes trifluorométhyle,

$R_3$ et $R_4$ représentent l'hydrogène, des groupes alcoxy en C1-C7, alkyle en C1-C7, des halogènes ou des groupes trifluorométhyle, ou bien $R_3$ et $R_4$ forment ensemble un groupe alkylène-dioxy en C1-C7,

$R_6$ et $R_7$ représentent chacun, indépendamment l'un de l'autre, un groupe hydroxy, amino, alcoxy en C1-C7, phényl-alcoxy en C1-C7, (alcoxy en C1-C7)-carbonyl-(alcoxy en C1-C7) ou leurs sels.

4. Procédé selon la revendication 1, caractérisé en ce que l'on prépare des composés de formule IA

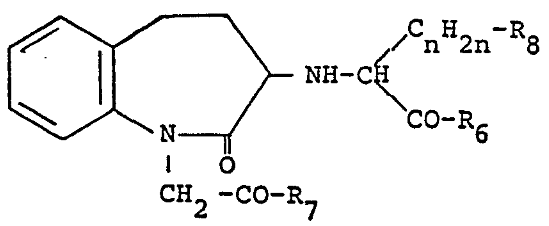

(IA)

dans laquelle n est un nombre entier de 1 à 4, $R_8$ représente l'hydrogène, un groupe phényle non substitué ou monosubstitué par un groupe alkyle en C1-C7, alcoxy en C1-C7, alcanoyloxy en C1-C7, un halogène, un groupe hydroxy ou trifluorométhyle, et $R_6$ et $R_7$ représentent chacun, indépendamment l'un de l'autre, un groupe hydroxy, alcoxy en C1-C4, benzyloxy ou amino, ou leurs sels acceptables pour l'usage pharmaceutique.

5. Procédé selon la revendication 1, caractérisé en ce que l'on prépare la 1-carboxyméthyl-3-(1-éthoxy-carbonyl-3-phényl-propylamino)-2,3,4,5-tétrahydro-1H-[1] benzazépine-2-one, ses stéréoisomères et ses sels.

6. Procédé selon la revendication 1, caractérisé en ce que l'on prépare le composé racémique, fondant à 138-140 °C, du composé mentionné dans la revendication 5, ses énantiomères et ses sels.

7. Procédé selon la revendication 1, caractérisé en ce que l'on prépare le composé racémique, fondant à 126-129 °C, du composé mentionné dans la revendication 5, ses énantiomères et ses sels.

8. Procédé selon la revendication 1, caractérisé en ce que l'on prépare la 1-carboxyméthyl-3S-(1S-éthoxy-carbonyl-3-phénylpropylamino)-2,3,4,5-tétrahydro-1H-[1] benzazépine-2-one et ses sels.

9. Procédé de préparation des composés de formule I mentionnés dans la revendication 1, dans lesquels X représente un groupe oxo et $R_1$ à $R_7$ ont les significations indiquées dans la revendication 1, caractérisé en ce que l'on condense un composé de formule X

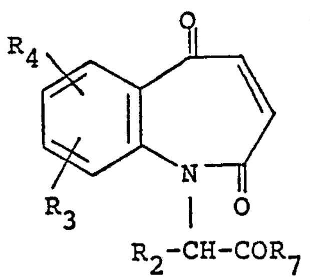

(X)

dans laquelle le noyau carbocyclique peut également être hexahydrogéné ou 6,7,8,9-tétrahydrogéné et $R_2$ à $R_4$ et $R_7$ ont les significations indiquées ci-dessus, avec une amine de formule VII

$$\begin{array}{c} R_5 \\ | \\ H-N-CH-R_1 \\ \quad\;\; | \\ \quad\;\; COR_6 \end{array} \qquad (VII)$$

dans laquelle $R_1$, $R_5$ et $R_6$ ont les significations indiquées ci-dessus, et si on le désire, on convertit un composé obtenu répondant à la formule I telle que définie ci-dessus en un autre composé de formule I à l'intérieur des limites spécifiées, et/ou si on le désire, on convertit un composé obtenu répondant à la formule I telle que définie ci-dessus et qui est salifiable en un sel, ou bien on libère un composé à partir d'un tel sel et/ou, si on le désire, on enrichit un isomère optique possédant une configuration spécifique par rapport à au moins un centre de chiralité à partir d'un mélange des formes stéréoisomères d'un composé obtenu répondant à la formule I.

10. Procédé de préparation d'une composition pharmaceutique, caractérisé en ce que l'on combine une substance active selon l'invention selon l'une des revendications 1 à 9 avec un véhicule pharmaceutique.

s